(19) Europäisches Patentamt
European Patent Office
Office européen des brevets

(11) **EP 4 657 445 A2**

(12) **EUROPEAN PATENT APPLICATION**

(43) Date of publication:
**03.12.2025 Bulletin 2025/49**

(21) Application number: **25208712.7**

(22) Date of filing: **17.09.2021**

(51) International Patent Classification (IPC):
*G16B 40/20* (2019.01)

(52) Cooperative Patent Classification (CPC):
**G16B 30/00; G16B 40/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.09.2020 US 202063080670 P**

(62) Document number(s) of the earlier application(s) in
accordance with Art. 76 EPC:
**21790728.6 / 4 193 362**

(71) Applicant: **Grail, Inc.**
**Menlo Park, California 94025 (US)**

(72) Inventors:
• **SAKARYA, Onur**
**Menlo Park, CA 94025 (US)**
• **YAKYM, Christopher-James, A.V.**
**Menlo Park, CA 94025 (US)**
• **SINGH, Pranav Parmjit**
**Menlo Park, CA 94025 (US)**
• **CALEF, Robert Abe Paine**
**Menlo Park, CA 94025 (US)**
• **HUANG, Richard**
**Menlo Park, CA 94025 (US)**

(74) Representative: **Murgitroyd & Company**
**165-169 Scotland Street**
**Glasgow G5 8PL (GB)**

Remarks:
This application was filed on 14.10.2025 as a
divisional application to the application mentioned
under INID code 62.

(54) **DETECTING CROSS-CONTAMINATION IN SEQUENCING DATA**

(57) Detecting cross-contamination between test samples used for determining cancer in a subject is beneficial. To detect cross-contamination, test sequences including at least one single nucleotide polymorphism are prepared using genome sequencing techniques. Some of the test sequences can be filtered to improve accuracy and precision. A prior contamination probability for each test sequence is determined based on a minor allele frequency. A contamination model including a likelihood test is applied to a test sequence. The likelihood test obtains a current contamination probability representing the likelihood that the test sample is contaminated. The contamination model can also determine a likelihood that the sample includes loss of heterozygosity representing the likelihood that the test sequence is contaminated. Test samples that are contaminated are removed. A source for the contaminated test sample can be found by comparing contaminated test sequences to other test sequences.

EP 4 657 445 A2

**Description**

CROSS-REFERENCE TO RELATED APPLICATIONS

This claims benefit to and priority to U.S. Provisional Application No. 63/080,670 filed September 18, 2020, which is hereby incorporated by reference herein in its entirety.

BACKGROUND

1. FIELD OF ART

This application relates generally to detecting contamination in a sample, and more specifically to detecting contamination in a sample including targeted sequencing used for early detection of cancer.

2. DESCRIPTION OF THE RELATED ART

Next generation sequencing-based assays of circulating tumor DNA must achieve high sensitivity and specificity in order to detect cancer early. Early cancer detection and liquid biopsy both require highly sensitive methods to detect low tumor burden as well as specific methods to reduce false positive calls. Contaminating DNA from adjacent samples can compromise specificity which can result in false positive calls. In various instances, compromised specificity can be because rare SNPs from the contaminant may look like low level mutations. Methods currently exist for detecting and estimating contamination in whole genome sequencing data, typically from relatively low-depth sequencing studies. However, existing methods are not designed for detection of contamination in sequencing data from cancer detection samples, which typically require high-depth sequencing studies and include tumor-derived mutations (e.g., single base mutations and/or copy number variations (CNVs)) that may be present at varying frequencies (e.g., clonal and/or sub-clonal tumor-derived mutations). There is a need for new methods of detecting cross-sample contamination in sequencing data from a test sample used for cancer detection.

SUMMARY

The systems and methods described herein can be used to determine cross-contamination between test samples used for determining cancer in a subject. The test samples are prepared using genome sequencing techniques. Each test sample includes a number of sequence read pairs. Each of the sequence read pairs includes forward strand sequence reads and reverse strand sequence reads. Typically, the sequence read pairs are obtained via a methylation aware sequence process, and each of the sequence read pairs comprise at least one single nucleotide polymorphism.

The system and methods can filter the sequence read pairs to generate a filtered population in a variety of manners. In one example, the filtering includes filtering forward strand sequence reads according to a first ruleset and reverse strand sequence reads according to a second ruleset. The first ruleset describes forward strand sequence reads that may indicate contamination, and the second ruleset describes reverse strand sequence reads that may indicate contamination.

The system and methods can determine a prior contamination probability for each SNP of the population of sequence read pairs based on a minor allele frequency for each of the SNPS. To do so, the system and methods can apply a contamination model (e.g., a negative binomial distribution applied to the population). The contamination model includes at least one likelihood test that tests a sequence read pair of the population using the contamination probabilities for the SNPs in that sequence read pair. Each of the at least one likelihood test may be configured to produce a test contamination probability representing the likelihood that the sequence read pair is a contamination. The system and methods can identify the contamination in the test sample when the contamination in the test sample when the contamination probability is above a likelihood threshold.

The ruleset-based filtering process may be based on which nucleotide base is at SNP site. More specifically, filtering may be based on which nucleotide base is at the SNP site on the forward strand sequence reads, and any determined nucleotide base at the corresponding SNP site on the corresponding reverse strand sequence read. To explain, a sequence read pair $x$ the forward sequence read $y$ and the reverse strand sequence read $z$ are corresponding sequence reads. Each of the forward strand sequence ready $y$ and reverse strand sequence read $z$ include an SNP at site $iy$ and $iz,$ respectively. The SNP sites iy and iz are corresponding SNP sites. The nucleotide base at SNP sites $iy$ and $iz$ of the sequence read pair $x$ may indicate cancer.

Given this context, the system and methods may apply various rules from the ruleset in the filtering process.

In an example, the systems and methods may identify a sequence read pair in the population where the nucleotide base is a cytosine base at an SNP site in the forward strand sequence read, and the corresponding nucleotide base is a guanine base at the corresponding SNP site in the corresponding reverse strand sequence read. Once identified, the systems and methods may remove the identified sequence read pair from the population.

In an example, the systems and methods may identify a sequence read pair in the population where the nucleotide base is a guanine base at an SNP site in the forward strand sequence read, and the corresponding nucleotide base is a cytosine base at the corresponding SNP site in the corresponding reverse strand sequence read. Once identified, the systems and methods may remove the identified sequence read pair from the population.

In an example, the systems and methods may identify a sequence read pair in the population where the nucleotide base is an adenine base at an SNP site in the forward strand sequence read, and the corresponding nucleotide base is a thymine base at the corresponding SNP site in the corresponding reverse strand sequence read. Once identified, the systems and methods may maintain the identified sequence read pair in the population.

In an example, the systems and methods may identify a sequence read pair in the population where the nucleotide base is a guanine base at an SNP site in the forward strand sequence read, and the corresponding nucleotide base is a cytosine base at the corresponding SNP site in the corresponding reverse strand sequence read. Once identified, the systems and methods may maintain the identified sequence read pair in the population.

In an example, the systems and methods may identify a forward strand sequence read in the population where the nucleotide base is an adenine base at an SNP site in the forward strand sequence read, and the corresponding nucleotide base is a guanine base at the corresponding SNP site in the corresponding reverse strand sequence read. Once identified, the systems and methods may maintain the identified forward strand sequence read in the population.

In an example, the systems and methods may identify a forward strand sequence read in the population where the nucleotide base is a thymine base at an SNP site in the forward strand sequence read, and the corresponding nucleotide base is a guanine base at the corresponding SNP site in the corresponding reverse strand sequence read. Once identified, the systems and methods may maintain the identified forward strand sequence read in the population.

In an example, the systems and methods may identify a forward strand sequence read in the population where the nucleotide base is a guanine base at an SNP site in the forward strand sequence read, and the corresponding nucleotide base is a adenine base at the corresponding SNP site in the corresponding reverse strand sequence read. Once identified, the systems and methods may maintain the identified forward strand sequence read in the population.

In an example, the systems and methods may identify a forward strand sequence read in the population where the nucleotide base is a guanine base at an SNP site in the forward strand sequence read, and the corresponding nucleotide base is a thymine base at the corresponding SNP site in the corresponding reverse strand sequence read. Once identified, the systems and methods may maintain the identified forward strand sequence read in the population.

In an example, the systems and methods may identify a forward strand sequence read in the population where the nucleotide base is a cytosine base at an SNP site in the forward strand sequence read, and the corresponding nucleotide base is a guanine base at the corresponding SNP site in the corresponding reverse strand sequence read. Once identified, the systems and methods may remove the identified forward strand sequence read in the population.

In an example, the systems and methods may identify a forward strand sequence read in the population where the nucleotide base is a cytosine base at an SNP site in the forward strand sequence read, and the corresponding nucleotide base is an adenine base at the corresponding SNP site in the corresponding reverse strand sequence read. Once identified, the systems and methods may remove the identified forward strand sequence read in the population.

In an example, the systems and methods may identify a forward strand sequence read in the population where the nucleotide base is an adenine base at an SNP site in the forward strand sequence read, and the corresponding nucleotide base is a cytosine base at the corresponding SNP site in the corresponding reverse strand sequence read. Once identified, the systems and methods may remove the identified forward strand sequence read in the population.

In an example, the systems and methods may identify a forward strand sequence read in the population where the nucleotide base is a thymine base at an SNP site in the forward strand sequence read, and the corresponding nucleotide base is a cytosine base at the corresponding SNP site in the corresponding reverse strand sequence read. Once identified, the systems and methods may remove the identified forward strand sequence read in the population.

In an example, the systems and methods may identify a reverse strand sequence read in the population where the nucleotide base is an adenine base at an SNP site in the forward strand sequence read, and the corresponding nucleotide base is a cytosine base at the corresponding SNP site in the corresponding reverse strand sequence read. Once identified, the systems and methods may maintain the identified reverse strand sequence read in the population.

In an example, the systems and methods may identify a reverse strand sequence read in the population where the nucleotide base is an thymine base at an SNP site in the forward strand sequence read, and the corresponding nucleotide base is a cytosine base at the corresponding SNP site in the corresponding reverse strand sequence read. Once identified, the systems and methods may maintain the identified reverse strand sequence read in the population.

In an example, the systems and methods may identify a reverse strand sequence read in the population where the nucleotide base is a cytosine base at an SNP site in the forward strand sequence read, and the corresponding nucleotide base is an adenine base at the corresponding SNP site in the corresponding reverse strand sequence read. Once identified, the systems and methods may maintain the identified reverse strand sequence read in the population.

In an example, the systems and methods may identify a reverse strand sequence read in the population where the nucleotide base is a cytosine base at an SNP site in the forward strand sequence read, and the corresponding nucleotide base is a thymine base at the corresponding SNP site in the corresponding reverse strand sequence read. Once identified, the systems and methods may maintain the identified reverse strand sequence read in the population.

In an example, the systems and methods may identify a reverse strand sequence read in the population where the nucleotide base is a guanine base at an SNP site in the forward strand sequence read, and the corresponding nucleotide base is an adenine base at the corresponding SNP site in the corresponding reverse strand sequence read. Once identified, the systems and methods may remove the identified reverse strand sequence read in the population.

In an example, the systems and methods may identify a reverse strand sequence read in the population where the nucleotide base is a guanine base at an SNP site in the forward strand sequence read, and the corresponding nucleotide base is a thymine base at the corresponding SNP site in the corresponding reverse strand sequence read. Once identified, the systems and methods may remove the identified reverse strand sequence read in the population.

In an example, the systems and methods may identify a reverse strand sequence read in the population where the nucleotide base is an adenine base at an SNP site in the forward strand sequence read, and the corresponding nucleotide base is a guanine base at the corresponding SNP site in the corresponding reverse strand sequence read. Once identified, the systems and methods may remove the identified reverse strand sequence read in the population.

In an example, the systems and methods may identify a reverse strand sequence read in the population where the nucleotide base is a thymine base at an SNP site in the forward strand sequence read, and the corresponding nucleotide base is a guanine base at the corresponding SNP site in the corresponding reverse strand sequence read. Once identified, the systems and methods may remove the identified reverse strand sequence read in the population.

The systems and methods may filter the population using additional methods. For example, the systems and methods may filter the plurality of sequence ready pairs by removing sequence read pairs comprising one or more nucleotide bases at one or more SNP sites included in an SNP removal table. The SNP sites in the SNP removal table indicate SNPs that inaccurately indicate the contamination. Similarly, the systems or methods may remove sequence read pairs comprising one or more corresponding nucleotide bases at one or more corresponding SNP sites included in an SNP removal table, the SNP removal table indicating SNPs that inaccurately indicate the contamination.

Test samples may be from a variety of locations and be one or more of a variety of sample types. For example, the test sample may be a plasma sample, or comprise a plurality of cell-free DNA molecules.

Moreover, the sequence read pairs me be variable. For instance, the plurality of sequence read pairs may be obtained from methylation-aware sequencing. In this case, the sequence read pairs may comprise a plurality of cell-free DNA (cfDNA) molecules treated such that unmethylated cytosine bases in the cfDNA molecules are converted to uracil bases. The sequence read pairs may be treated cfDNA molecules. The sequence read pairs may be treated with, for example, sodium bisulfite. In another example, the sequence read pairs may be treated with cytidine deaminase. The treated sequences may be obtained via genome-wide bisulfite sequencing and/or paired-end massively parallel sequencing. In some examples, the systems and method may enrich a test sample for a plurality of targeted cfDNA molecules before performing the methylation-aware sequencing.

BRIEF DESCRIPTION OF DRAWINGS

Figure (FIG.) 1 is a flowchart of a method for preparing a nucleic acid sample for sequencing, according to one example embodiment.

FIG. 2 is a block diagram of a processing system for processing sequence reads, according to one example embodiment.

FIG. 3 is a flowchart of a method for determining variants of sequence reads, according to one example embodiment.

FIG. 4 illustrates a block diagram of a contamination detection application and workflow for detecting and calling contamination in a test sample, according to one example embodiment.

FIG. 5A-5F are probability distribution plots showing the probability of observing data at a given contamination level as a function of minor allele depth for different contamination levels and probabilities of observing specific mutation, according to some example embodiments.

FIG. 5G is probability distribution plot for a test sample with a contamination level $\alpha$ and a probability of observing a specific mutation $\varepsilon$ of Y% with a depth of 50, according to one example embodiment.

FIG. 5H is probability distribution plot for a test sample with a contamination level $\alpha$ and a probability of observing a specific mutation $\varepsilon$ of Y% with a depth of 1000, according to one example embodiment.

FIG. 6A illustrates a flow diagram of a workflow for detecting contamination of sequencing data, according to one example embodiment.

FIG. 6B a flow diagram of a contamination detection workflow, according to one example embodiment.

FIG. 7A is a plot showing the number of informative SNPs for a given sample pair for a contamination event, according to one example embodiment.

FIG. 7B is an informative SNP spider plot for a contamination event, according to one example embodiment.

FIG. 8 is an SNP frequency plot 800 showing the number of SNPs for each frequency bin for a first SNP set comprising 2718 SNPs and for a second SNP set comprising 12174 SNPs, according to one example embodiment.

FIG. 9 is a plot showing the expected power of informative SNPs based on population minor allele frequency (MAF), according to one example embodiment.

FIG. 10 is a plot showing the limit of contamination detection obtained using the contamination detection application, according to one example embodiment.

FIG. 11 illustrates a workflow of a method of validating the contamination detection application, according to one embodiment, according to one example embodiment.

FIG. 12 is a plot showing an example of a ROC curve generated during cross-validation for threshold evaluation, according to one example embodiment.

FIG. 13 is a plot showing the probability distributions for the three hypotheses of the loss of heterozygosity likelihood test, according to one example embodiment.

FIG. 14A is a plot showing the probability distributions for the three hypotheses of the loss of heterozygosity likelihood test for a sample with a high contamination probability, according to one example embodiment.

FIG. 14B is a plot showing the probability distributions for the three hypotheses of the loss of heterozygosity likelihood test for a sample with a low contamination probability, according to one example embodiment.

FIG. 14C illustrates a probability comparison plot comparing loss of heterozygosity models employing a negative binomial distribution to those employing a binomial distribution, according to one example embodiment.

FIG. 15 illustrates a flow diagram of a method for removing sequencing data including loss of heterozygosity and detecting contamination in the remaining sequencing data, according to one example embodiment.

FIG. 16 is a plot showing the validation of the contamination detection including loss of heterozygosity removal via in-vitro titration experiments, according to one example embodiment.

FIGs. 17A-17C are plots comparing the performance of methods for determining contamination detection including loss of heterozygosity removal to alternative detection methods known in the art for detecting contamination in samples contaminated via in-vitro titration, according to one example embodiment.

FIGs. 18A-18B are plots comparing the performance of contamination detection including loss of heterozygosity removal to alternative detection methods known in the art for detecting contamination in samples known to be cancer free, according to one example embodiment.

FIGs. 19A-19C are plots comparing the performance of contamination detection including loss of heterozygosity removal and alternative detection methods known in the art for detecting contamination in samples obtained from tumors, according to one example embodiment.

FIG. 20 illustrates a flow diagram of an example of a method for generating a contamination noise baseline, according to one example embodiment.

FIG. 21 is a plot showing an example of the noise rate of SNPs, according to one example embodiment.

FIG. 22A is a plot showing the MAF distribution of informative SNPs compared to all SNPs, according to one example embodiment.

FIG. 22B is a plot showing the noise rate distribution of informative SNPs compared to all SNPs, according to one example embodiment.

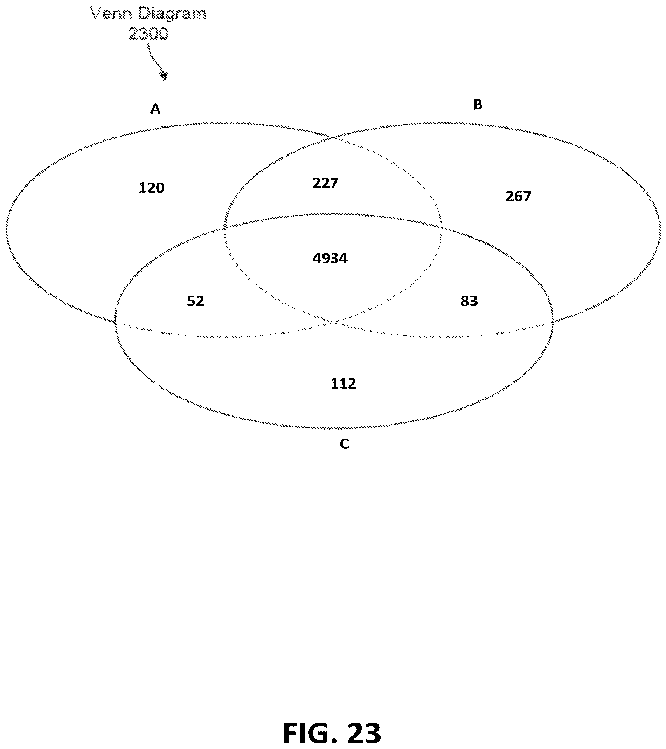

FIG. 23 is a Venn diagram showing a comparison of the contamination (noise) baselines generated for three separate studies (designated A, B, and C), according to one example embodiment.

FIG. 24 is a panel of plots showing the variant allele frequencies for 25 SNPs, according to one example embodiment.

FIG. 25A illustrates a tri-nucleotide context error plots, according to some example embodiments.

FIG. 25B illustrates a tri-nucleotide context error comparison plot, according to one example embodiment.

FIG. 25C illustrates a trio of contamination detection plots, according to one example embodiment.

FIG. 26A is a screenshot of an example of an output file opened in MS Excel that includes information for each baseline/normal sample tested, according to one example embodiment.

FIG. 26B is a screenshot of a portion of the output file of FIG. 14 that shows the analysis data for two contamination events in the baseline/normal sample dataset, according to one example embodiment.

FIG.s 27A and 27B are plots showing the log-likelihood of different hypotheses of contamination levels for baseline/normal samples B1_6_WO44216569493 and B6_14_WO44216552592, according to one example embodiment.

FIG. 28 shows a flow diagram illustrating a dual-strand filtering workflow, according to one example embodiment.

FIG. 29 is a sample distribution plot illustrating the average number of SNPs in a chromosome after the sample has been filtered based on bisulfite conversion, according to one example embodiment.

FIGS. 30A and 30Bare validation plots showing the improvement in the limit of detection of contamination detection when filtering SNPs associated with bisulfite conversions, according to one example embodiment.

FIG. 31 shows a flow diagram illustrating a dual-strand filtering workflow, according to one example embodiment.

FIG. 32 is a filter verification plot comparing a single-strand workflow to a dual-strand filtering workflow, according to one example embodiment.

FIG. 33A is a SNP density plot for a test sample filtered according to a dual-strand workflow, according to one example embodiment.

FIG. 33B is a SNP density plot for a test sample filtered according to a single-strand workflow, according to one example embodiment.

FIG. 34A is a filter density plot comparing SNP density resulting from a dual-strand workflow and a PRS workflow, according to one example embodiment

FIG. 34B is a filter depth plot showing comparing SNP depth resulting from a dual-strand workflow and a PRS workflow, according to one example embodiment.

FIG. 35 show a flow diagram illustrating a blacklist filtering workflow, according to one example embodiment.

FIG. 36A illustrates a contamination event comparison plot where the test samples are not filtered according to a blacklist filtering workflow, according to one example embodiment.

FIG. 36B illustrates a contamination comparison plot where the test samples are filtered according to a blacklist filtering workflow, according to one example embodiment.

FIG. 37 shows a flow diagram illustrating a blacklist generation workflow, according to one example embodiment.

FIG. 38 is a cohort characteristic plot showing the observed minor allele frequency for SNPs in a cohort of test samples, according to one example embodiment.

FIG. 39 shows a threshold variance plot illustrating how changing the variant threshold affects incorrectly calling an uncontaminated sample, according to some example embodiments.

FIG. 40 shows a size variance plot illustrating how changing the size of the SNP blacklist affects incorrectly calling an uncontaminated sample, according to some example embodiments.

FIG. 41 shows a size and threshold variance plot illustrating how changing both the size of the SNP blacklist and the variant threshold of an outlier indicator affects incorrectly calling an uncontaminated sample, according to some example embodiments.

FIG. 42 illustrates a flow diagram illustrating a contamination threshold determination workflow, according to one example embodiment.

FIG. 43 illustrates an average LLR heuristic plot, according to one example embodiment.

FIG. 44 illustrates a ROC heuristic plot 4400, according to one example embodiment.

The figures depict embodiments of the present invention for purposes of illustration only. One skilled in the art will readily recognize from the following discussion that alternative embodiments of the structures and methods illustrated herein may be employed without departing from the principles of the invention described herein.

DETAILED DESCRIPTION

I. DEFINITIONS

The term "individual" refers to a human individual. The term "healthy individual" refers to an individual presumed to not have cancer or disease. The term "subject" refers to an individual who is known to have, or potentially has, cancer or disease.

The term "sequence reads" refers to nucleotide sequences read from a sample obtained from an individual. Sequence reads can be obtained through various methods known in the art.

The term "read segment" or "read" refers to any nucleotide sequences including sequence reads obtained from an individual and/or nucleotide sequences derived from the initial sequence read from a sample obtained from an individual. For example, a read segment can refer to an aligned sequence read, a collapsed sequence read, or a stitched read. Furthermore, a read segment can refer to an individual nucleotide base, such as a single nucleotide variant.

The term "single nucleotide variant" or "SNV" refers to a substitution of one nucleotide to a different nucleotide at a position (e.g., site) of a nucleotide sequence, e.g., a sequence read from an individual. A substitution from a first nucleobase X to a second nucleobase Y may be denoted as "X>Y." For example, a cytosine to thymine SNV may be denoted as "C>T."

The term "single nucleotide polymorphism" or "SNP" refers to a position on the genome where significant fraction of the population has a substitution of one nucleotide to a different nucleotide at a position (e.g., site) of a nucleotide sequence, e.g., a sequence read from an individual. For example, at a specific base site, the nucleobase C may appear in most individuals, but in a minority of individuals, the position is occupied by base A. There is a SNP at this specific site.

The term "indel" refers to any insertion or deletion of one or more base pairs having a length and a position (which may also be referred to as an anchor position) in a sequence read. An insertion corresponds to a positive length, while a deletion corresponds to a negative length.

The term "mutation" refers to one or more SNVs or indels.

The term "true positive" refers to a mutation that indicates real biology, for example, the presence of potential cancer, disease, or germline mutation in an individual. True positives are not caused by mutations naturally occurring in healthy individuals (e.g., recurrent mutations) or other sources of artifacts such as process errors during assay preparation of nucleic acid samples.

The term "false positive" refers to a mutation incorrectly determined to be a true positive. Generally, false positives may be more likely to occur when processing sequence reads associated with greater mean noise rates or greater uncertainty in noise rates.

The term "cell-free nucleic acid," "cell-free DNA," or "cfDNA" refers to nucleic acid fragments that circulate in an individual's body (e.g., bloodstream) and originate from one or more healthy cells and/or from one or more cancer cells.

The term "circulating tumor DNA" or "ctDNA" refers to nucleic acid fragments that originate from tumor cells or other types of cancer cells, which may be released into an individual's bloodstream as result of biological processes such as apoptosis or necrosis of dying cells or actively released by viable tumor cells.

The term "genomic nucleic acid," "genomic DNA," or "gDNA" refers to nucleic acid including chromosomal DNA that originates from one or more healthy cells.

The term "alternative allele" or "ALT" refers to an allele having one or more mutations relative to a reference allele, e.g., corresponding to a known gene.

The term "minor allele" or "MIN" refers to the second most common allele in a given population.

The term "sequencing depth" or "depth" refers to a total number of read segments from a sample obtained from an individual at a particular position of the genome.

The term "allele depth" or "AD" or "DP" refers to a number of read segments in a sample that supports an allele in a population. The terms "AAD", "MAD" refer to the "alternate allele depth" (i.e., the number of read segments that support an ALT) and "minor allele depth" (i.e., the number of read segments that support a MIN), respectively.

The term "contaminated" refers to a test sample that is contaminated with at least some portion of a second test sample. That is, a contaminated test sample unintentionally includes DNA sequences from an individual that did not generate the test sample. Similarly, the term "uncontaminated" refers to a test sample that does not include at least some portion of a second test sample.

The term "contamination level" refers to the degree of contamination in a test sample. That is, the contamination level the number of reads in a first test sample from a second test sample. For example, if a first test sample of 1000 reads includes 30 reads from a second test sample, the contamination level is 3.0%.

The term "contamination event" refers to a test sample being called contaminated. Generally, a test sample is called contaminated if the determined contamination level is above a threshold contamination level and the determined contamination level is statistically significant.

The term "allele frequency" or "AF" refers to the frequency of a given allele in a sample. The terms "AAF", "MAF" refer to the "alternate allele frequency" and "minor allele frequency", respectively. The AF may be determined by dividing the corresponding AD of a sample by the depth of the sample for the given allele.

II. EXAMPLE ASSAY PROTOCOL

The methods and systems described herein are related to U.S. Application No. 16/019,315, filed on February 15, 2018, which is incorporated herein by reference in their entirety for all purposes.

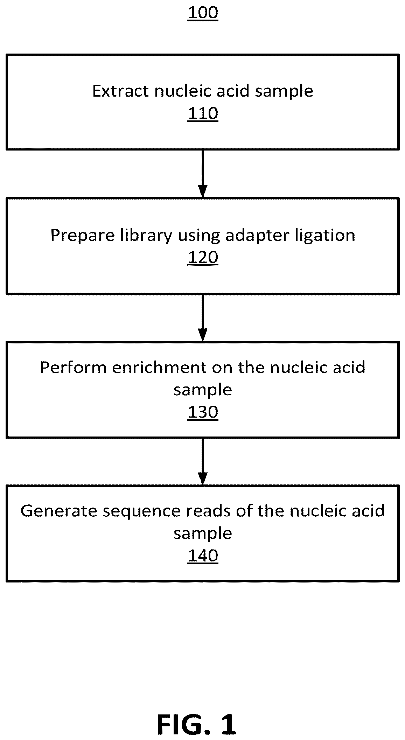

FIG. 1 is a flowchart of a method 100 for preparing a nucleic acid sample for sequencing according to one embodiment. The method 100 includes, but is not limited to, the following steps. For example, any step of the method 100 may comprise a quantitation substep for quality control or other laboratory assay procedures known to one skilled in the art.

In step 110, a nucleic acid sample (DNA or RNA) is extracted from a subject. In the present disclosure, DNA and RNA may be used interchangeably unless otherwise indicated. That is, the following embodiments for using error source information in variant calling and quality control may be applicable to both DNA and RNA types of nucleic acid sequences. However, the examples described herein may focus on DNA for purposes of clarity and explanation. The sample may be any subset of the human genome, including the whole genome. The sample may be extracted from a subject known to have or suspected of having cancer. The sample may include blood, plasma, serum, urine, fecal, saliva, other types of bodily fluids, or any combination thereof. In some embodiments, methods for drawing a blood sample (e.g., syringe or finger prick) may be less invasive than procedures for obtaining a tissue biopsy, which may require surgery. The extracted sample may comprise cfDNA and/or ctDNA. For healthy individuals, the human body may naturally clear out cfDNA and other cellular debris. If a subject has cancer or disease, ctDNA in an extracted sample may be present at a detectable level for diagnosis.

In step 120, a sequencing library is prepared. During library preparation, unique molecular identifiers (UMI) are added to the nucleic acid molecules (e.g., DNA molecules) through adapter ligation. The UMIs are short nucleic acid sequences (e.g., 4-10 base pairs) that are added to ends of DNA fragments during adapter ligation. In some embodiments, UMIs are degenerate base pairs that serve as a unique tag that can be used to identify sequence reads originating from a specific DNA fragment. During PCR amplification following adapter ligation, the UMIs are replicated along with the attached DNA fragment, which provides a way to identify sequence reads that came from the same original fragment in downstream analysis.

In step 130, targeted DNA sequences are enriched from the library. During enrichment, hybridization probes (also referred to herein as "probes") are used to target, and pull down, nucleic acid fragments informative for the presence or absence of cancer (or disease), cancer status, or a cancer classification (e.g., cancer type or tissue of origin). For a given workflow, the probes may be designed to anneal (or hybridize) to a target (complementary) strand of DNA or RNA. The target strand may be the "positive" strand (e.g., the strand transcribed into mRNA, and subsequently translated into a protein) or the complementary "negative" strand. The probes may range in length from 10s, 100s, or 1000s of base pairs. In one embodiment, the probes are designed based on a gene panel to analyze particular mutations or target regions of the genome (e.g., of the human or another organism) that are suspected to correspond to certain cancers or other types of diseases. Moreover, the probes may cover overlapping portions of a target region. By using a targeted gene panel rather than sequencing all expressed genes of a genome, also known as "whole exome sequencing," the method 100 may be used to increase sequencing depth of the target regions, where depth refers to the count of the number of times a given target sequence within the sample has been sequenced. Increasing sequencing depth reduces required input amounts of the nucleic acid sample. After a hybridization step, the hybridized nucleic acid fragments are captured and may also be amplified using PCR. In some examples, the targeted DNA sequences are not enriched and the method 100 moves directly to step 140.

In step 140, sequence reads are generated from the enriched DNA sequences. Sequencing data may be acquired from the enriched DNA sequences by known means in the art. For example, the method 100 may include next-generation sequencing (NGS) techniques including synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators.

In some embodiments, the sequence reads may be aligned to a reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene.

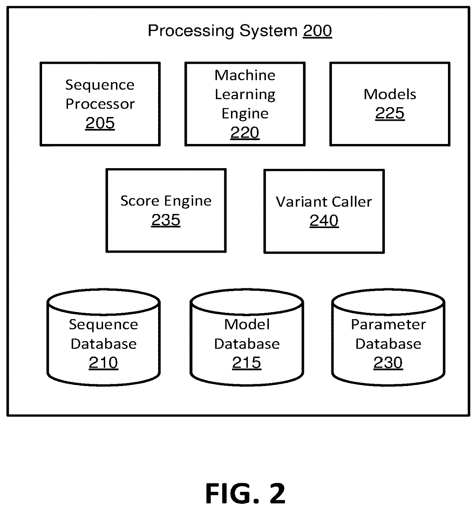

In various embodiments (e.g., in paired-end sequencing), a sequence read is comprised of a read pair denoted as $R_1$ and $R_2$. For example, the first read $R_1$ may be sequenced from a first end of a nucleic acid fragment whereas the second read $R_2$ may be sequenced from the second end of the nucleic acid fragment. Therefore, nucleotide base pairs of the first read $R_1$ and second read $R_2$ may be aligned consistently (e.g., in opposite orientations) with nucleotide bases of the reference genome. Alignment position information derived from the read pair $R_1$ and $R_2$ may include a beginning position in the reference genome that corresponds to an end of a first read (e.g., $R_1$) and an end position in the reference genome that corresponds to an end of a second read (e.g., $R_2$). In other words, the beginning position and end position in the reference genome represent the likely location within the reference genome to which the nucleic acid fragment corresponds. An output file having SAM (sequence alignment map) format or BAM (binary) format may be generated and output for further analysis such as variant calling, as described below with respect to FIG. 2.

III. EXAMPLE PROCESSING SYSTEM

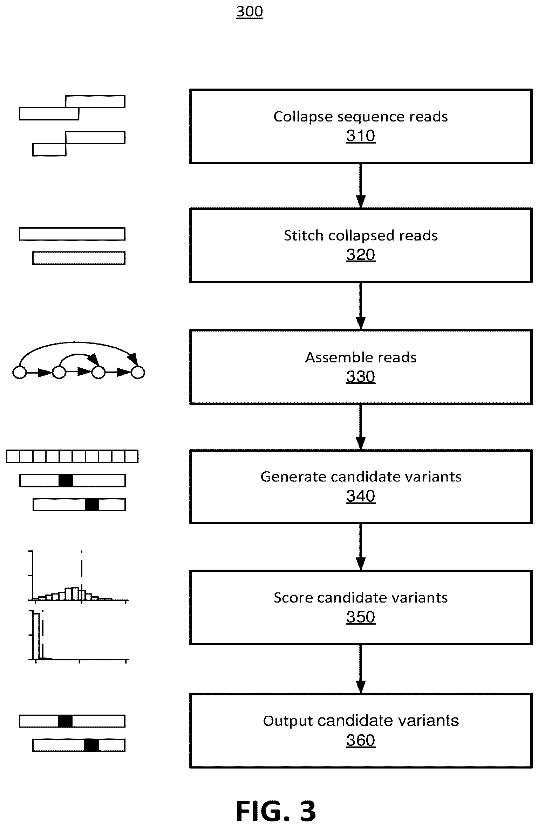

FIG. 2 is a block diagram of a processing system 200 for processing sequence reads according to one embodiment. The processing system 200 includes a sequence processor 205, sequence database 210, model database 215, machine learning engine 220, models 225, parameter database 230, score engine 235, and variant caller 240. FIG. 3 is a flowchart of a method 300 for determining variants of sequence reads according to one embodiment. In some embodiments, the processing system 200 performs the method 300 to perform variant calling (e.g., for SNPs) based on input sequencing data. Further, the processing system 200 may obtain the input sequencing data from an output file associated with a nucleic acid sample prepared using the method 100 described above. The method 300 includes, but is not limited to, the following steps, which are described with respect to the components of the processing system 200. In other embodiments, one or more steps of the method 300 may be replaced by a step of a different process for generating variant calls, e.g., using Variant Call Format (VCF), such as HaplotypeCaller, VarScan, Strelka, or SomaticSniper.

The processing system 200 can be any type of computing device that is capable of running program instructions.

Examples of processing system 200 may include, but are not limited to, a desktop computer, a laptop computer, a tablet device, a personal digital assistant (PDA), a mobile phone or smartphone, and the like. In one example, when processing system is a desktop or laptop computer, models 225 may be executed by a desktop application. Applications can, in other examples, be a mobile application or web-based application configured to execute the models 225.

At step 310, the sequence processor 205 collapses aligned sequence reads of the input sequencing data. In one embodiment, collapsing sequence reads includes using UMIs, and optionally alignment position information from sequencing data of an output file (e.g., from the method 100 shown in FIG. 1) to collapse multiple sequence reads into a consensus sequence for determining the most likely sequence of a nucleic acid fragment or a portion thereof. Since the UMIs are replicated with the ligated nucleic acid fragments through enrichment and PCR, the sequence processor 205 may determine that certain sequence reads originated from the same molecule in a nucleic acid sample. In some embodiments, sequence reads that have the same or similar alignment position information (e.g., beginning and end positions within a threshold offset) and include a common UMI are collapsed, and the sequence processor 205 generates a collapsed read (also referred to herein as a consensus read) to represent the nucleic acid fragment. The sequence processor 205 designates a consensus read as "duplex" if the corresponding pair of collapsed reads have a common UMI, which indicates that both positive and negative strands of the originating nucleic acid molecule are captured; otherwise, the collapsed read is designated "non-duplex." In some embodiments, the sequence processor 205 may perform other types of error correction on sequence reads as an alternative to, or in addition to, collapsing sequence reads.

At step 320, the sequence processor 205 stitches the collapsed reads based on the corresponding alignment position information. In some embodiments, the sequence processor 205 compares alignment position information between a first read and a second read to determine whether nucleotide base pairs of the first and second reads overlap in the reference genome. In one use case, responsive to determining that an overlap (e.g., of a given number of nucleotide bases) between the first and second reads is greater than a threshold length (e.g., threshold number of nucleotide bases), the sequence processor 205 designates the first and second reads as "stitched"; otherwise, the collapsed reads are designated "unstitched." In some embodiments, a first and second read are stitched if the overlap is greater than the threshold length and if the overlap is not a sliding overlap. For example, a sliding overlap may include a homopolymer run (e.g., a single repeating nucleotide base), a dinucleotide run (e.g., two-nucleotide base sequence), or a trinucleotide run (e.g., three-nucleotide base sequence), where the homopolymer run, dinucleotide run, or trinucleotide run has at least a threshold length of base pairs.

At step 330, the sequence processor 205 assembles reads into paths. In some embodiments, the sequence processor 205 assembles reads to generate a directed graph, for example, a de Bruijn graph, for a target region (e.g., a gene). Unidirectional edges of the directed graph represent sequences of k nucleotide bases (also referred to herein as "k-mers") in the target region, and the edges are connected by vertices (or nodes). The sequence processor 205 aligns collapsed reads to a directed graph such that any of the collapsed reads may be represented in order by a subset of the edges and corresponding vertices.

At step 340, the variant caller 240 generates candidate variants from the paths assembled by the sequence processor 205. In one embodiment, the variant caller 240 generates the candidate variants by comparing a directed graph (which may have been compressed by pruning edges or nodes in step 310) to a reference sequence of a target region of a genome. The variant caller 240 may align edges of the directed graph to the reference sequence, and records the genomic positions of mismatched edges and mismatched nucleotide bases adjacent to the edges as the locations of candidate variants. Additionally, the variant caller 240 may generate candidate variants based on the sequencing depth of a target region. In particular, the variant caller 240 may be more confident in identifying variants in target regions that have greater sequencing depth, for example, because a greater number of sequence reads help to resolve (e.g., using redundancies) mismatches or other base pair variations between sequences. In some embodiments, the variants can be SNPs.

Further, multiple different models may be stored in the model database 215 or retrieved for application post-training. For example, a model is trained to model SNP noise rates, a model is trained to filter SNPs, a model is trained to verify contamination detection, a model is trained to detect loss of heterozygosity, etc. Further, the score engine 235 may use parameters of the model 225 to determine a likelihood of one or more true positives or contamination in a sequence read. The score engine 235 may determine a quality score (e.g., on a logarithmic scale) based on the likelihood. For example, the quality score is a Phred quality score $Q = -10 \cdot log_{10} P$, where P is the likelihood of an incorrect candidate variant call (e.g., a false positive).

At step 350, the score engine 235 scores the candidate variants based on the model 225 or corresponding likelihoods of true positives, contamination, quality scores, etc. Training and application of the model 225 are described in more detail below.

At step 360, the processing system 200 outputs the candidate variants. In some embodiments, the processing system 200 outputs some or all of the determined candidate variants along with the corresponding scores. Downstream systems, e.g., external to the processing system 200 or other components of the processing system 200, may use the candidate variants and scores for various applications including, but not limited to, predicting the presence of cancer, predicting contamination in test sequences, predicting noise levels, or germline mutations.

IV. EXAMPLE CONTAMINATION DETECTION WORKFLOW

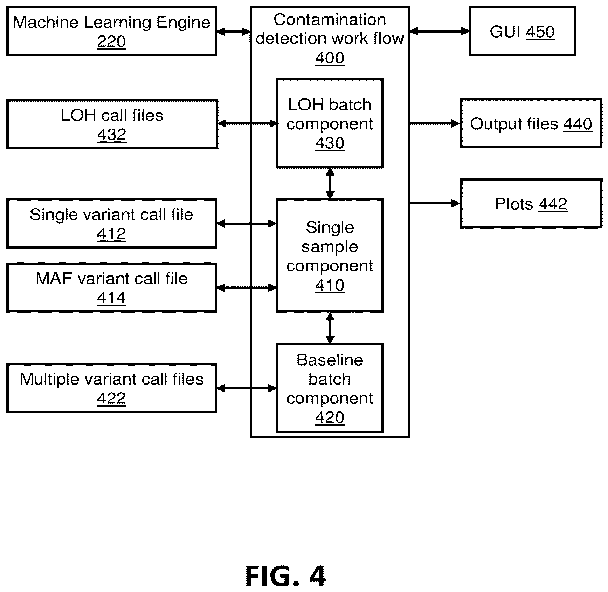

FIG. 4 is a diagram of a contamination detection workflow 400 executing on the processing system 200 for detecting and calling contamination, in accordance with one embodiment.

In the illustrated example, contamination detection workflow 400 includes a single sample component 410, a baseline batch component 420, and a loss of heterozygosity (LoH) batch component 430. Single sample component 410 of contamination detection workflow 400 is informed, for example, by the contents of a single variant call file 442 and a minor allele frequencies (MAF) variant call file 444 called by the variant caller 240. The single variant call file 412 is the variant call file for a single target sample. The MAF variant call file 414 is the MAF variant call file for any number of SNP population allele frequencies AF.

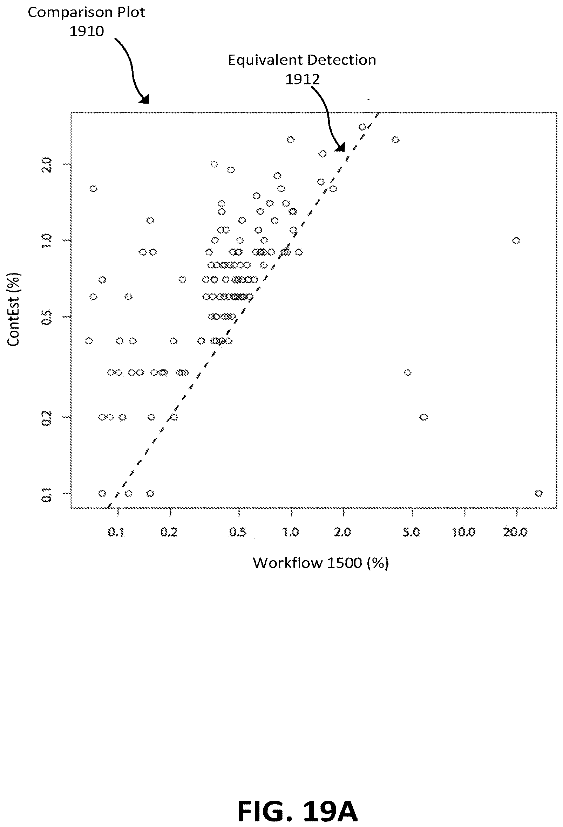

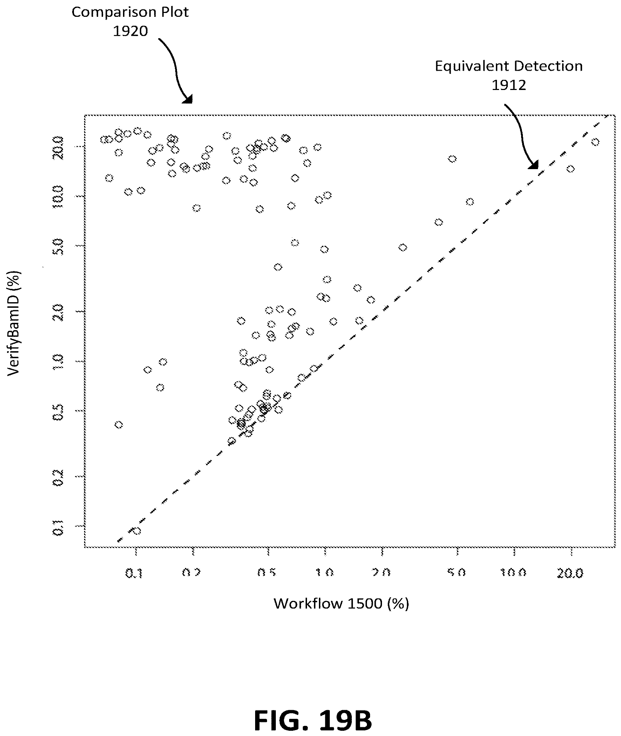

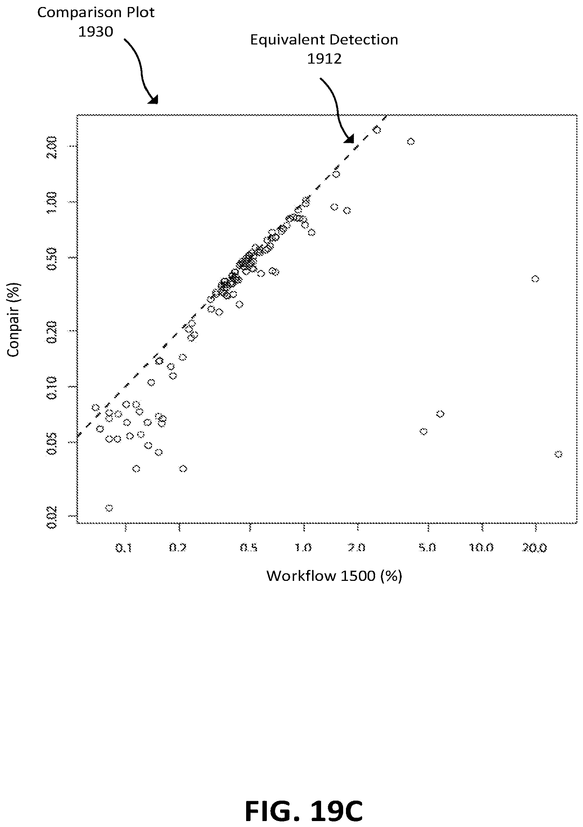

Baseline batch component 420 of contamination detection workflow 400 generates a background noise baseline for each SNP from uncontaminated samples as another input to single sample component 410. Generating a background noise baseline using a contamination noise baseline workflow is described in more detail in regards to FIG. 19. Baseline batch component 420 is informed, for example, by the contents of multiple variant call files 422 called by the variant caller 240. The multiple variant call files 422 can be the variant call files of multiple samples.

LoH batch component 430 of contamination detection workflow 400 determines a LoH in samples as another input to the single sample component 410. LoH batch component 430 is informed, for example, by the contents of LoH call files 432. The LoH call files are call files for a plurality of alleles previously determined to include SNPs with LoH in the sample. The LoH call files can be called by the variant caller 240 and stored in the sequence database 210.

In one embodiment, the contamination detection workflow 400 can generate output files 440 and/or plots from sequencing data processed by contamination detection algorithm. For example, contamination detection workflow 400 may generate log-likelihood data and/or display log-likelihood plots 442 as a means for evaluating a DNA test sample for contamination. Data processed by contamination detection workflow 400 can be visually presented to the user via a graphical user interface (GUI) 450 of the processing system 200. For example, the contents of output files 440 (e.g., a text file of data opened in Excel) and log-likelihood plots 442 can be displayed in GUI 450.

In another embodiment, the contamination detection workflow 400 may use the machine learning engine 220 to improve contamination detection. Various training datasets (e.g., parameters from parameter database 230, sequences from sequence database 210, etc.) may be used to supply information to the machine learning engine 220 as described herein. In accordance with this embodiment, the machine learning engine 220 may be used to train a contamination noise baseline to identify a noise threshold, detect loss of heterozygosity, and determine the limit of detection (LOD) for contamination detection.

Single sample component 410 of contamination detection workflow 400 is, for example, a runnable script that is used to estimate contamination in a sample. By contrast, baseline batch component 430 of contamination detection workflow 400 is, for example, a runnable script that is used for generating estimates across a batch of samples, and may also be used to generate the noise model across these samples (if the input batch is healthy). Similarly, LoH batch component 430 of contamination detection model is, for example, a runnable script that is used for generating estimates across a batch of samples, and may be used to determine the LoH in single samples based on the generated estimates.

V. DETECTING CONTAMINATION OF A SAMPLE

In one embodiment, the contamination detection workflow 400 may be based on a model for estimating contamination. In one embodiment, the model is a maximum likelihood model (herein referred to as the likelihood model) for detecting contamination in sequencing data from a test sample. However, in other examples, the model can be any other estimation model such as an M-estimator, maximum spacing estimation, method of support, etc.

In one example, the likelihood model determines contamination by calculating the probability of observing a MAF of a sample at a given contamination level $\alpha$ and, subsequently, determining if the sample is contaminated. In some embodiments, the likelihood model is informed by prior probabilities of contamination that are first calculated for each SNP in the sample based on the genotype of previously observed contaminated samples.

Further, the contamination detection workflow 400 can, in some cases, determine the likely source of contamination for the observed sample. That is, the likelihood model can compare sequencing data from several contaminated samples to determine a source of contamination. The likelihood model can be informed by prior probabilities of contamination from other samples with a known genotype to identify a likely source of contamination.

V.A PROBABILITY OF CONTAMINATION FOR A SINGLE SNP

The contamination detection workflow 400 determines a probability that a sample is contaminated using prior probabilities and observed sequencing data (e.g., in a population of samples). In some examples, the observed sequencing data can be included in a test sample call file (such as single variant call file 412), a LoH call file (such as LoH call file 432) and a population call file (such as MAF call file 414). The prior probabilities of contamination can be determined based on the observed sequencing data. Here, for purpose of example, the probability of contamination for a single SNP is based on a samples minor allele frequency MAF and the error rate of previously observed homozygous SNPs. In some embodiments, the contamination detection workflow 400 can additionally or alternatively use alternate allele frequency, noise rates, read depths, etc. to determine a contamination probability.

Contamination detection workflow 400 compares the probability of observing data in the test sequences in the sample and population using two different models. In one model, there is no contamination and any reads with alternative alleles at the site i are either the result of noise in the test sequences corresponding to site i or of heterozygosity of the test sequences at the site i. In the other model, there is contamination of the test sample and test sequences with alternative alleles that can be the result of correctly reading a contaminating DNA strand. In this context, contamination detection workflow 400 calculates a ratio between the likelihood the test sample is contaminated and the likelihood the test sample is uncontaminated using the two models. Based on the ratio, contamination detection workflow can determine if the test sample is contaminated or uncontaminated.

In one embodiment, the probability of contamination at a single SNP site for a given set of data D is calculated as:

$$P(\alpha|D) = P(D|\alpha) \cdot P(\alpha) \qquad (1)$$

where $P(\alpha|D)$ is the probability of observing the contamination level $\alpha$ given the data D, $P(D|\alpha)$ is the probability of observing the data given the contamination level $\alpha$, and $P(\alpha)$ is the probability of the contamination level $\alpha$. Therefore, in an example where there is no contamination in the test sample, the probability of contamination in a test sample can be represented as:

$$P(\alpha = 0|D) = P(D|\alpha = 0) \cdot P(\alpha = 0) \qquad (2)$$

where $\alpha = 0$ indicates that the contamination level $\alpha$ is 0.0%

In one example, the data D for an SNP in a test sample is represented below in

Table 1.

| Table 1: Data for an SNP in a test sample. | |
|---|---|
| SNP Identifier | RS2237290 |
| Chromosome Position | 7 : 13952609 |
| Global MAF | 0.3896 |
| $\epsilon$ for A→T | $3 \cdot 10^{-5}$ |
| $\epsilon$ for T→A | $9 \cdot 10^{-5}$ |
| Test Sample Depth | 50 |
| Minor Allele Depth | 5 |

where $\epsilon$ is the probability of a specific error or mutation at the SNP site. In other examples the data D can include any number of additional or fewer elements such that contamination detection workflow 400 can determine contamination in a test sample.

In one embodiment, in test samples where the contamination level is non-zero, the probability of observing data D with a contamination level $\alpha$ for a given set of data D (i.e., $P(D|\alpha)$) is further based on the genotype of the contaminant Gc and the genotype of the host $G_H$ (the source of the test sample). That is, the probability of observing data D given a contamination level $\alpha$ can be represented as:

$$P(D|\alpha) = \sum_{G_H, G_C} P(G_H) \cdot P(G_C) \cdot P(D|\alpha p) \qquad (3)$$

where $P(Gc)$ is the probability that the contamination at the SNP site will be the type associated with the genotype of the contaminant at that site, $P(G_H)$ is the probability that the contamination at the site will be the genotype of the host at that site, and $P(D|\alpha)$ is the probability of observing the data D at a contamination level $\alpha$. Here, the set of characteristics p include the specified genotypes GH and GC, probability of an SNP mutation $\epsilon$ for the SNP site and the contamination level $\alpha$ but can include any other characteristics of the test sample. Here, the summation sign is not wholly the same as a mathematical summation (i.e., not over the *variable* $G_H$). Instead, the summation over the genotypes indicates that the probability of observing data at a contamination level $\alpha$ across the sites includes contributions based on the three possible genotypes of the contaminant and host (A/A, A/B, and B/B).

For a given site the probability of observing the data at a given contamination level alpha can be represented with a generic site specific model. The generic site specific model can be represented as:

$$P(D|\alpha) =$$

$$P(AA_{host}) \cdot P(AA_{cont}) \cdot P(D|p = \varepsilon) + \ldots$$

$$P(AA_{host}) \cdot P(AB_{cont}) \cdot P\left(D\middle|p = \varepsilon + \frac{\alpha}{2}\right) + \ldots$$

$$P(AA_{host}) \cdot P(BB_{cont}) \cdot P(D|p = \varepsilon + \alpha) +$$

$$\ldots$$

$$P(BB_{host}) \cdot P(BB_{cont}) \cdot P(D|p = \varepsilon) \tag{4}$$

where AA is a homozygous reference allele, AB is a heterozygous allele, BB is a homozygous alternative allele, the subscript "host" represents the genotype of the host $G_H$, the subscript "cont" represents the genotype of the contaminant, $\varepsilon$ is the probability of observing a specific mutation, and $\alpha$ is the contamination level.

V.A.I BINOMIAL DISTRIBUTIONS

In some cases, the generic site specific model can be modeled with a binomial distribution. For example, for a specific case from the generic site specific model, the probability of observing the data D at a given contamination level alpha can be represented as:

$$P(D|\alpha) = P(D|AA_{host}, AB_{cont}, \alpha) = binomial\left(DP, MAD, \frac{\alpha}{2} + \varepsilon\right) \tag{5}$$

where "binomial" is the binomial probability of observing the data based on depth DP and minor allele depth MAD (minor allele depth) of the test sample, the genotype of the host (A/A), the genotype of the contaminant (A/B), the contamination level $\alpha$, and the probability of observing a specific error or mutation $\varepsilon$.

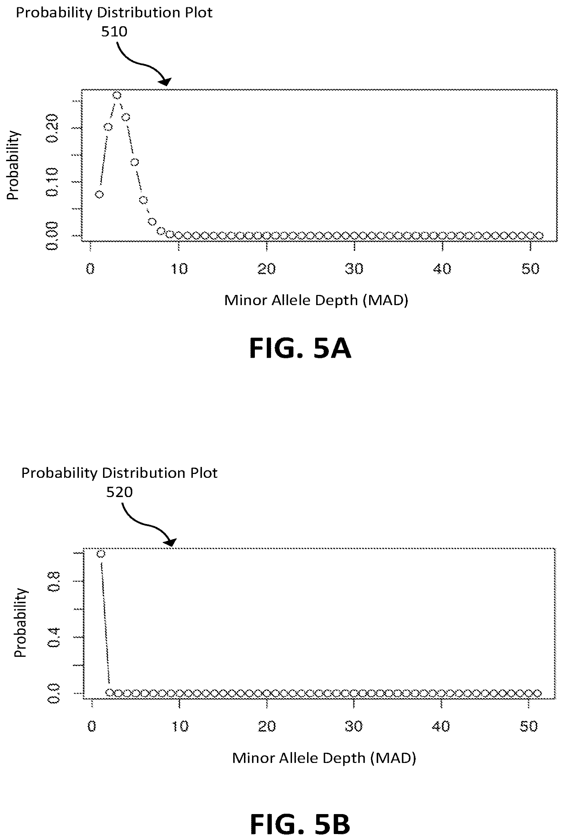

For example, FIG. 5A is probability distribution plot 510 for a test sample with a contamination level $\alpha$ of 10% and a probability of observing a specific mutation $\varepsilon$ of 0.01%. In this plot, the minor allele depth is on the x-axis and the probability based on the binomial distribution (similar to Eqn. 4) is on the y-axis. Therefore, the plot shows the probability of observing a minor allele depth MAD given the contamination level alpha, the SNP mutation probability epsilon, a genotype of the host of A/A and a genotype of the contaminant of A/B.

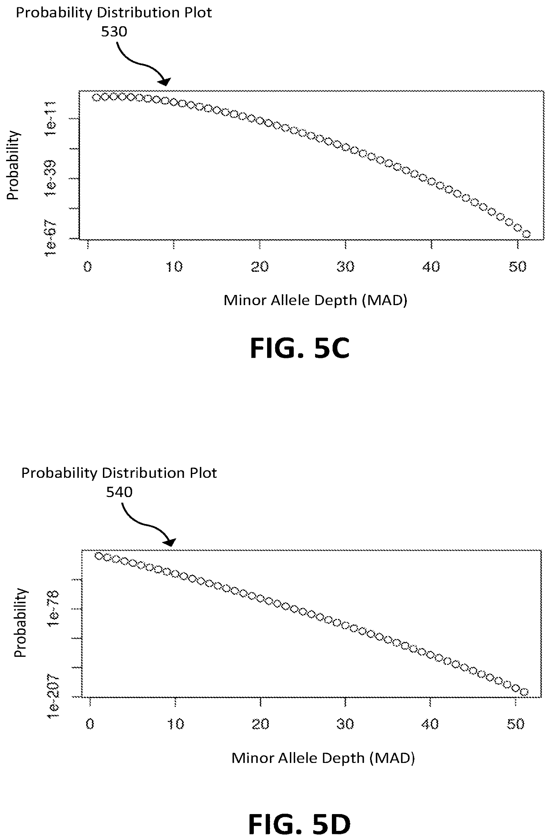

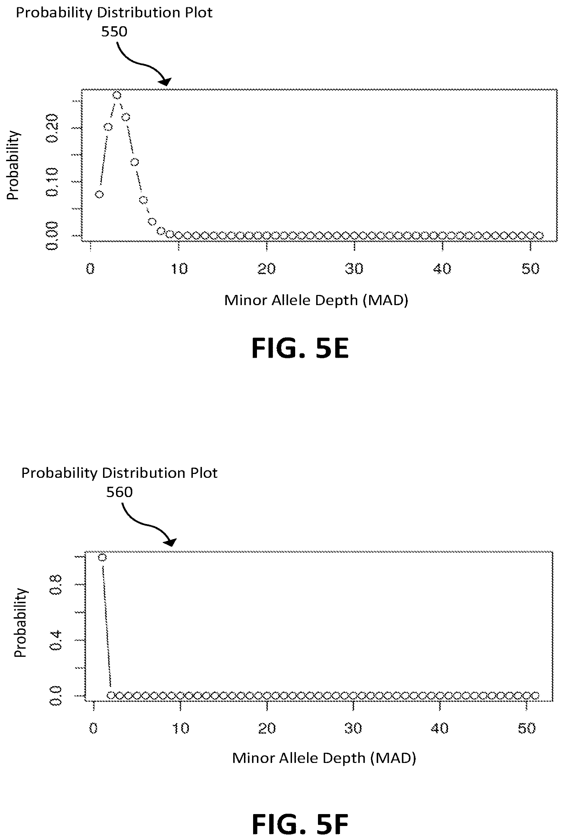

FIG. 5B - FIG. 5F are probability distribution plots for different contamination levels $\alpha$ and mutation probabilities $\varepsilon$. FIG. 5B is a probability distribution plot 520 for a test sample with a contamination level $\alpha$ of 0% and a probability of observing a specific mutation $\varepsilon$ of 0.01%. FIG. 5C is a probability distribution plot 530 for a test sample with a contamination level $\alpha$ of 10% with a logarithmically scaled y axis. FIG. 5D is a probability distribution plot 540 for a test sample with a contamination level $\alpha$ of 0% and a probability of observing a specific mutation $\varepsilon$ of 0.01% with a logarithmically scaled y-axis. FIG. 5E is a probability distribution plot 550 for a test sample with a contamination level $\alpha$ of 10% and a blackswan value of 0.002 (a minimum contribution level of 0.002). FIG. 5F is a probability distribution plot 560 for a test sample with a contamination level $\alpha$ of 0%, a probability of observing a specific mutation of 0.01% and a blackswan value of 0.002.

V.A.II NEGATIVE BINOMIAL DISTRIBUTIONS

In other cases, the generic site-specific model can be modeled with a negative binomial distribution. For example, the probability of observing the data D at a given contamination level alpha at a specific site can be represented as:

$$P(D|\alpha) = P(D|AB_{host}, BB_{cont}, \alpha) = nbinomial\left(DP, MAD, \frac{\alpha}{2} + \varepsilon\right) \tag{6}$$

where "nbinomial" is the negative binomial probability of observing the data based on depth DP and minor allele depth (MAD) of the test sample, the genotype of the host (A/B), the genotype of the contaminant (B/B), the contamination level $\alpha$, and the probability of observing a specific error or mutation $\varepsilon$.

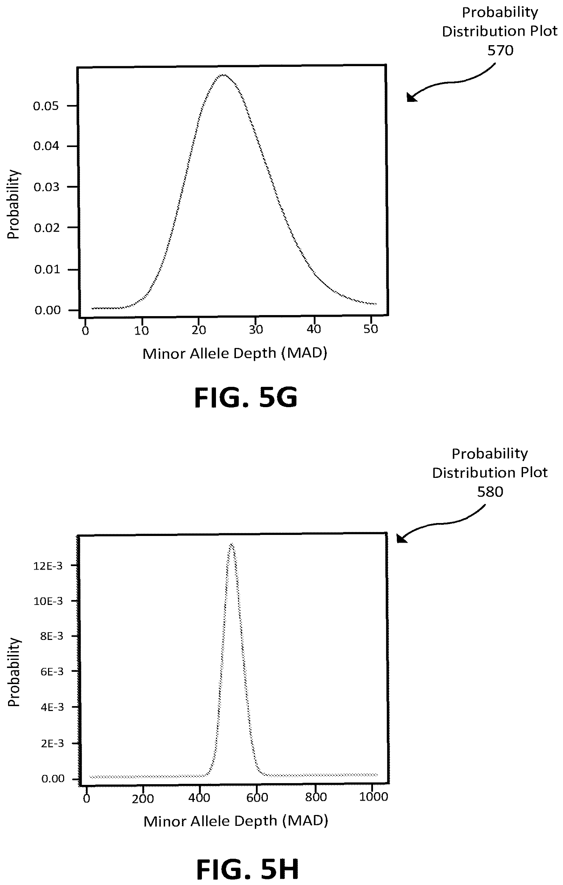

To illustrate, FIG. 5G is a probability distribution plot 570 for a test sample with a contamination level $\alpha$ of X% and a probability of observing a specific heterozygous SNP ε of Y%. The depth of samples is 50. Further, the pull down and enrichment efficiency of each allele is about 50 percent. In this plot, the minor allele depth is on the x-axis and the probability based on the negative binomial distribution is on the y-axis. Therefore, the plot shows the probability of observing a minor allele depth (MAD) given the contamination level α, the heterozygous SNP probability ε, a genotype of the host of A/B and a genotype of the contaminant of B/B. FIG. 5H is a probability distribution plot 580 similar to FIG. 5G, but the depth of samples is 1000 rather than 50.

V.A.III SIMPLIFICATIONS

The generic site-specific model can be simplified using prior probabilities of contamination. The simplified model can be represented as:

$$P(D|\alpha) = P_C \cdot P(D|\alpha, C) + (1 - P_C)P(D|\alpha = 0, !\,C) \qquad (7)$$

where $P_C$ is the probability of contamination of the test sample based on a prior observation of a contaminant with a genotype different from the host genotype C, P(D|α,C) is the probability of observing the data D with a contamination level α given the SNP is contaminated, (1-$P_c$) is the probability of no contamination and P(D|α=0, !C) is the probability of observing data D with a contamination level α of 0% (i.e., no contamination, denoted as ! C).

Alternatively stated, Pc is the probability that an SNP at a site is contaminated with a contaminant of a different allele type than the host given a contamination level α. In one example, the simplified model determines the prior probability of contamination Pc using the following:

$$P_C = \begin{cases} 1 - (1 - MAF)^2 & if\ host\ is\ A/A \\ 1 - MAF^2 & if\ host\ is\ B/B \end{cases} \qquad (8)$$

where MAF is the minor allele frequency, A/A is a homozygous reference allele, and B/B is an homozygous alternative allele. Here, heterozygous alleles are removed and are not considered in determining the probability of contamination for a test sample.

V.B PROBABILITY OF CONTAMINATION FOR A SAMPLE

As previously described, in one embodiment, the contamination detection workflow 400 uses a likelihood model to determine contamination in a sample. Here, to determine contamination in a sample, the likelihood model determines a level of contamination α that maximizes a likelihood function L(α). The likelihood function L(α) can be written as:

$$L(\alpha) \propto P(D|\alpha) = \prod_{i=1}^{N} max\,(P(D_i|\alpha), \beta) \qquad (9)$$

where P(D|α) is the probability of observing data D given contamination level α, β is a minimum allowable probability, N is the number of homozygous (A\A or B\B) SNPs of the sample, and $D_i$ is the observed data for a given SNP.

The likelihood function L(α) is proportional to the probability of observing data D given a contamination level α (P(D|α)). The probability of the data D given a contamination level α takes into account all SNPs of the sample. That is, L(α) is the product over each SNP in the sample of the maximum of the probability of the data in that SNP given the contamination level α (P($D_i$|α)). For each SNP, if the probability of the data D given a contamination level α is below a threshold, the probability for that SNP can be assigned a value β. The value β is a minimum probability that is set as a black swan term (e.g., $\beta = 3.3 \times 10^{-7}$) which limits the lowest value each SNP evaluated can contribute to the likelihood function L(α). The probability of contamination at of a single SNP site (P($D_i$|α)) is described in more detail in Section V.a.

V.C PROBABILITY OF CONTAMINATION FOR A SAMPLE USING LIKELIHOOD TESTS

In one example of determining the likelihood of contamination, the contamination detection workflow 400 applies a likelihood model including two separate likelihoods tests.

In the first likelihood test, the product term of the likelihood function L(α) is used to calculate a first likelihood ratio (LR) representing the maximum contamination likelihood that is obtained from testing a series of contamination levels $\alpha_i$ against the minor allele frequency in a sample. That is, which level of contamination α gives the highest contamination likelihood.

The first likelihood ratio $LR_1$ uses a first null hypothesis that the sample is contaminated at a maximum of a series of contamination levels α (L(α = $\alpha_i$)) based on the MAF of the observed SNPs. That is, the sample is contaminated at a

contamination level $\alpha_{max}$ giving the highest likelihood of contamination. Therefore, the first null hypothesis can be written as:

$$L_{max} = max[L_1(\alpha = .001), L_2(\alpha = .002), ... L_i(\alpha = .5)] \qquad (10)$$

The first likelihood ratio also uses a first hypothesis that there is no contamination in the sample ($L(\alpha = 0.000)$). Therefore, the first likelihood ratio test $LR_1$ can be written as:

$$LR_1 = \frac{max[L(\alpha=0.001), L(\alpha=0.002), L(\alpha=0.003) ... L(\alpha=.5)]}{L(\alpha=0.000)} \qquad (11)$$

Generally, the first likelihood ratio $LR_1$ results in a value. The sample is considered to pass the first likelihood test if the value of the first likelihood ratio $LR_1$ is above a threshold level. That is, it is likely that the sample is contaminated at a contamination level $\alpha$.

In the second likelihood test, the likelihood function $L(\alpha)$ is used to calculate a second likelihood ratio $LR_2$ representing a likelihood that observed minor allele frequencies are due to contamination rather than due to a constant increase in noise across all SNPs.

The second likelihood ratio $LR_2$ uses a second null hypothesis $L_{max}$ MAF that is the same as the first null hypotheses (Eqn. 4). Additionally, the second likelihood ratio $LR_2$ uses a second hypothesis $L_{noise}$ that a sample contaminated at contamination level $\alpha_{max}$ includes minor allele frequencies at an average allele frequency of previously observed SNPs (uniform(MAF)). The second null hypothesis can be written as:

$$L_{noise} = L(\alpha_{max}|uniform(MAF)) \qquad (12)$$

Accordingly, the second likelihood ratio can be written as:

$$LR_2 = \frac{L_{max}}{L_{noise}} = \frac{max[L_1(\alpha=0.001), L_2(\alpha=0.002), ... L_i(\alpha=.5)]}{L(\alpha_{max}|uniform(mAF))} \qquad (13)$$

The second likelihood ratio $LR_2$ results in a value. The sample is considered to pass the second likelihood test $LR_2$ if the value is above a threshold. That is, it is likely that the observed MAF is due to contamination and not due to noise. Alternatively stated, the second likelihood test passes when a specific arrangement of previously observed MAFs are significant in determining the contamination likelihood, while a random distribution of previously observed MAFs are insignificant in determining contamination likelihood.

If a test sample passes both of the likelihood tests, then the sample is called as contaminated at contamination level $\alpha$ which passes the tests. If a test sample fails either of the likelihood tests, then it is not called as contaminated.

In other configurations, the contamination detection workflow can use additional or fewer likelihood tests to determine if a sample is contaminated.

V.D DETERMINING A CONTAMINATION SOURCE

In one example of determining the likelihood of contamination, the likelihood model of the contamination detection workflow 400 can additionally determine a likely source of contamination. Detecting the source of contamination enables the assessment of risk introduced by the contaminant, as well as the point in sample process in which it happened, such as, for example, any step of process 100 or 300. In contamination detection workflow 400, the genotypes of likely contaminants may be used in place of prior probabilities from population SNPs. Introduction of prior probabilities of contamination will either increase or decrease the likelihood ratio relative to the likelihood ratio obtained by for probabilities based on the population.

The likelihood model can be informed by the prior probabilities of SNPs from the known genotypes of samples that were processed in the same batch as the test sample (or a set of related batches). A likelihood test is then performed to determine if knowing the exact genotype probabilities gives a higher value than the likelihood obtained using the population MAF probability. If the difference is significant, it can be concluded that a given sample is the contaminant.

For a given SNP, three observed genotypes are possible: homozygous reference 0/0, heterozygous 0/1, and homozygous alternative 1/1, where 0 represents the reference allele and 1 the alternative allele. In a normal (uncontaminated) sample, the expected allele frequency values observed are expected to be close to 0, 0.5 and 1 for genotypes 0/0, 0/1 and 1/1, respectively. However, in a contaminated sample, the observed allele frequency values can be expected to shift from 0, 0.5, and 1, as the SNPs vary across the population, and thus, have a higher likelihood of being present in a contaminating sample.

V.E CONTAMINATION DETECTION USING LIKELIHOOD TESTS

It is important to be able to distinguish between contamination and noise. As noted above, processing system 200 can be used to detect contamination in a test sample. For example, using the contamination detection workflow 400 a contamination event can be detected based on a plurality (or set) of observed variant allele frequencies in a test sample. In one embodiment, the observed variant allele frequencies can be compared to population MAFs from a plurality of SNPs for the detection of cross-sample contamination.

V.E.I SINGLE SAMPLE CONTAMINATION DETECTION

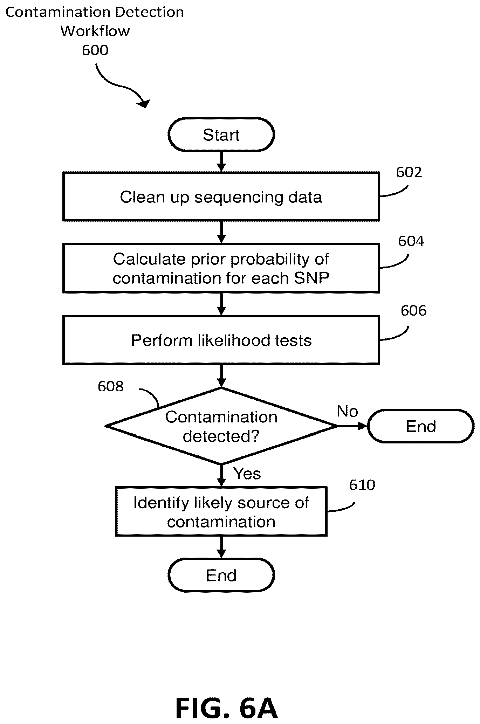

FIG. 6A illustrates a flow diagram illustrating a contamination detection workflow 630 performed in accordance with the workflow 400 of FIG. 4. The detection workflow 630 of this embodiment includes, but is not limited to, the following steps. The detection workflow detects contamination in a sample obtained from a person for a disease diagnosis.

At step 610, sequencing data obtained from a sample (e.g., using the process 300) is cleaned up and genotypes are neutralized. For example, data cleaning may include filtering out non-informative SNPs, removing SNPs with no coverage, removing SNPs with high error frequencies (e.g., > 0.1%), removing SNPs with high variance, removing SNPs with a depth less than a threshold, removing any heterozygous SNPs, removing SNPs with low coverage, and removing any SNPs that have a high heterogeneity rate. In other examples, homozygous alternative SNPs with variant frequency 0.8 to 1.0 can be negated (e.g., variant frequency 0.95 becomes 0.05) in order to put all the variant frequency data in one scale that can be linearly compared to minor allele frequency values. Further, the MAF values can be negated based on a samples genotype.

At step 604, a prior probability of contamination is calculated for each SNP based on host sample's genotype and minor allele frequency as described in Section V.b

At step 606, a likelihood model including a maximum likelihood estimation is applied to determined contamination based on the prior probability of contamination for the SNPs. The likelihood model includes a first and a second likelihood test as described in Section V.c.

At a decision step 608, it is determined whether the test sample is contaminated. If a test sample passes both likelihood tests (e.g., probabilities above a threshold), then the sample is contaminated and workflow 600 proceeds to a step 610. If a test sample does not pass either likelihood test, then the sample is not contaminated and workflow 600 ends.

At step 612, a likely source of contamination is identified based on the prior probabilities of SNPs from known genotypes of other samples that were processed in the same batch as the test sample (or a set of related batches) as described in Section V.d.

V.E.II SAMPLE CONTAMINATION DETECTION IN A BATCH OF SAMPLES

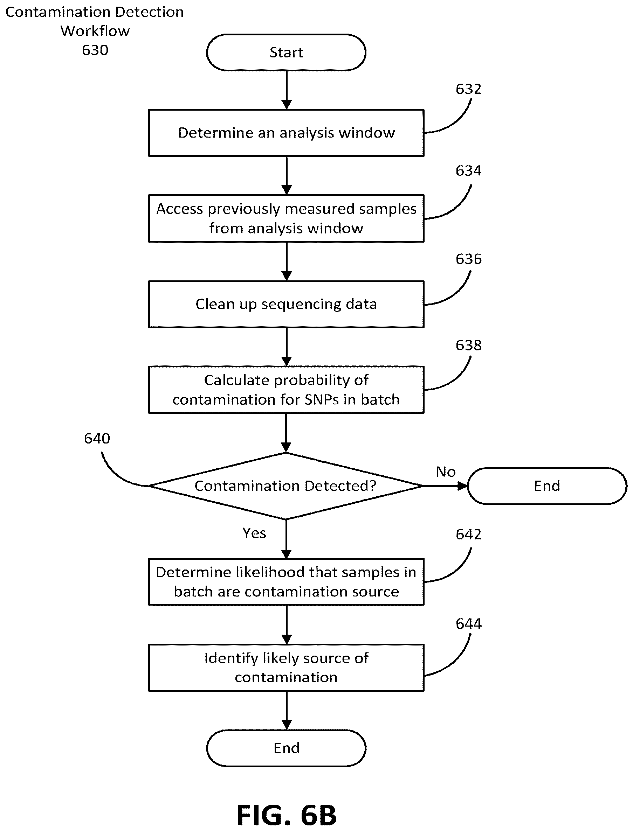

FIG. 6B illustrates a flow diagram showing a contamination detection workflow 630 performed in accordance with the workflow 400 of FIG. 4. The detection workflow 630 of this embodiment may include, but is not limited to, the following steps. The detection workflow detects contamination in a contamination source in a group of samples. The samples in the group are obtained from one or more persons for a disease diagnosis. The workflow may be implemented by a processing system (e.g., processing system 200).

At step 632, the system determines an analysis window. An analysis window, for example, defines a subset of samples from a whole set of samples that were obtained and/or processed similarly. For example, the analysis window may be a subset of samples from a sample batch, or a subset of batches from a batch set. In other embodiments, the analysis window may be: (i) a number of samples from a whole sample set, (ii) a period of samples obtained from a longer period of sample taking, (iii) samples associated with a testing location of a number of testing locations, (iv) samples associated with a testing apparatus, etc.

At step 634, the system accesses test sequences within the analysis window. For example, the system may access all of the samples obtained within a five-day window. In another example, the system may access all samples from a particular sample batch of a number of sample batches.

At step 636, the system cleans (or pre-processes) sequencing data from samples in the analysis window and neutralizes genotypes. For example, data cleaning may include filtering out non-informative SNPs, removing SNPs with no coverage, removing SNPs with high error frequencies (e.g., > 0.1%), removing SNPs with high variance, removing SNPs with a depth less than a threshold, removing any heterozygous SNPs, removing SNPs with low coverage, and removing any SNPs that have a high heterogeneity rate.

In other examples, homozygous alternative SNPs with variant frequency 0.8 to 1.0 can be negated (e.g., variant frequency 0.95 becomes 0.05) in order to put all the variant frequency data in one scale that can be linearly compared to minor allele frequency values. Further, the MAF values can be negated based on a samples genotype.

In other examples, data cleaning may include removing non-informative SNPs based on the methylation processes indicated in the sequencing data. Filtering data based on methylation processes are described in more detail below.

At step 638, the system determines a prior probability of contamination for each SNP in the analysis window. The prior probability of contamination is based on a host sample's genotype and minor allele frequency as described in, for example, Section V.b. Here, the "host" sample may be one or more of the samples within the analysis window, or a sample known to have contamination. To do so, the system applies a likelihood model to the test sequences to determine contamination based on the prior probability of contamination for the SNPs. The likelihood model can include a maximum likelihood estimation as described herein. The likelihood model can include a first and a second likelihood test as described in, for example, Section V.c.

At a decision step 640, the system determines whether one or more of the samples in the analysis window is contaminated. The system determines a sample in the analysis window is contaminated if the test sequence passes both likelihood tests. The system determines a sample in the analysis window is not contaminated if the test sequences fails one or more of the likelihood tests. If there is contamination, the workflow 630 proceeds to step 642, and if there is no contamination the workflow ends.

At step 642, the system determines a likelihood that a contaminated test sample within the analysis window is a contamination source. To do so, the system determines the likelihood using genotypes from samples identified as contaminated (rather than the minor allele frequency). In this manner, the system can determine if a particular contaminated sample within the analysis window is the source of contamination in other contaminated samples within the analysis window.

At step 644, the system determines a likely source of contamination. To do so, the system can rank the likelihoods that each contaminated sample in the analysis window is the contamination source. The system can determine that any number of the samples in the ranked list are a contamination source (e.g., the top 3, those having a likelihood above a threshold, etc.). In response, the system may remove the contaminated test sequences such that they are not further analyzed by the system. Further, the system may utilize the determined likely source of contamination to understand systematic sources of contamination that exist and, if those sources exist, to remove them.

### V.F SUPPORTING DATA

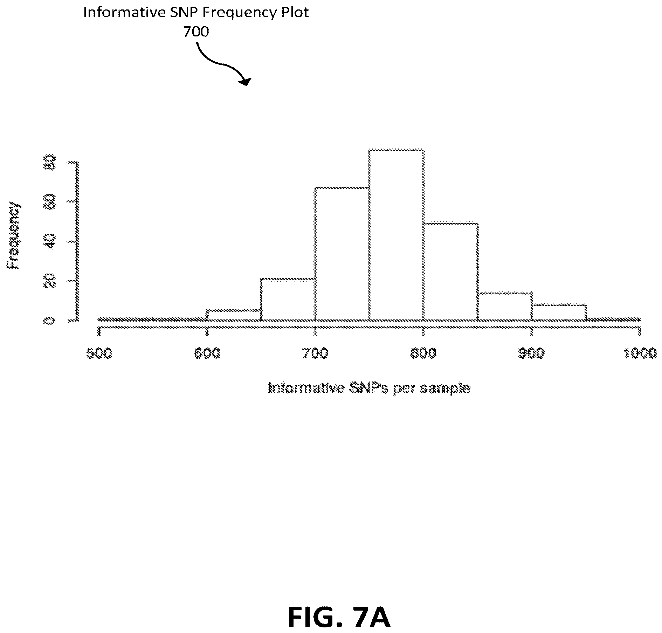

FIG. 7A is an informative SNP frequency plot 700 showing an example number of informative SNPs for a given sample pair for a contamination event. In an example set of 12174 SNPs, about 700 SNPs are informative SNPs. That is, for example, the SNPs are homozygous in the host and a different genotype in the contaminant.

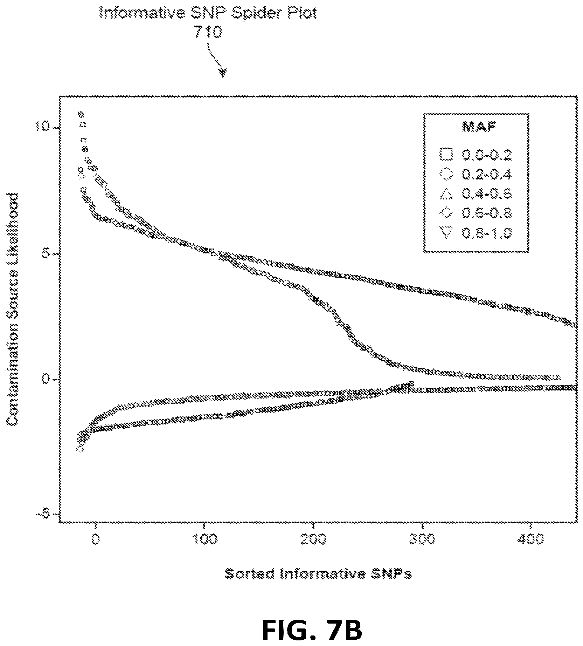

FIG. 7B is an informative SNP spider plot 710 for a contamination source event. In the SNP spider plot 710, the x-axis are the SNPs sorted by their source likelihoods, and the y-axis is the actual contamination likelihood values. Square data points represent the possible source of contamination being tested, and triangle data points represent the population average likelihood. The MAF for each SNP are indicated by the color of the triangle and square icons according to the legend. Within the SNP spider plot 710, the upper section of the plot represents SNPs with positive evidence, and the lower section represent the SNPs with negative evidence for contamination.

The SNP spider plot 710 shows, per SNP, the likelihood that a test sample is a contamination source. In this SNP spider plot 710, the positive likelihoods and fast drop in negative likelihoods, points in the direction of a true positive call. By examining this time of plot for the other candidates (out of a top number of candidates), the control system can understand how the SNP compares to other possible SNPs that may be a contamination source. The likelihoods are based on the genotypes of the top three samples identified as a likely contamination source. The spider plot shows SNPs supporting and disproving the source hypothesis.

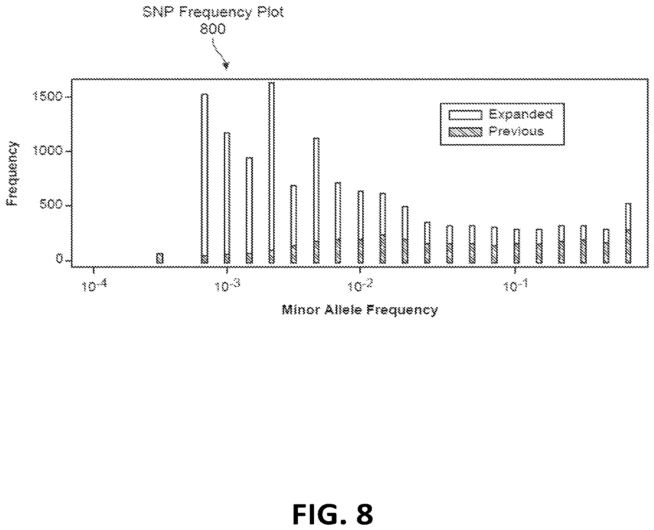

FIG. 8 is an SNP frequency plot 800 showing the number of SNPs for each frequency bin for a first SNP set comprising 2718 SNPs ("Previous", indicated by solid lines) and for a second SNP set comprising 12174 SNPs ("Expanded", indicated by dashed lines). The minor allele frequencies for this SNP set range from about $10^{-3}$ to about 1. The data show that most of the SNPs in the SNP set are in the lower frequency range.

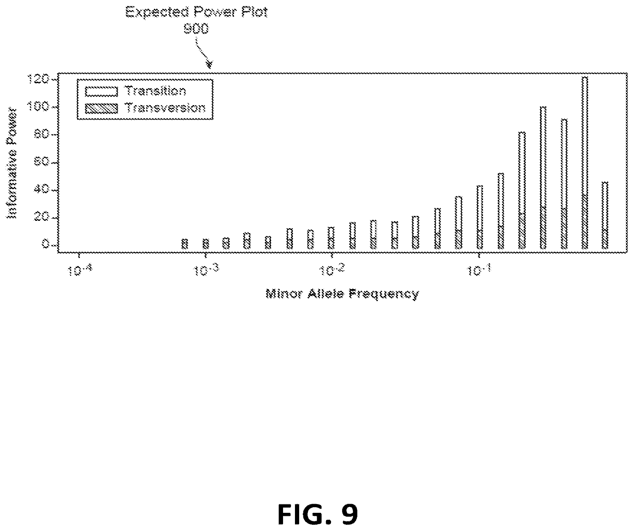

FIG. 9 is an expected power plot 900 showing the expected power of informative SNPs based on population minor allele frequency (MAF). The expected power is: Power $= n \times ((1 - p)^2 \times (1 - (1 - p)^2 + p^2 \times (1 - p^2))$, where p is the MAF and n is the number of SNPs in a MAF bin. The data show that the highest power is for SNPs with higher MAFs. Transitions (dashed lines) correspond to substitutions that are between purine bases (A, G) or between pyrimidine bases (C, T), whereas transversions (solid lines) are interchanges of purine and pyrimidine bases.

### V.G LIMIT OF DETECTION

To determine the limit of detection (LOD) of contamination detection workflow 400, two clean samples were mixed *in silico* at different contamination levels (ranging from 50% down to 0.01% contamination level $\alpha$). Here, the limit of detection is considered to be the lowest contamination level at which the specificity is above 95%.

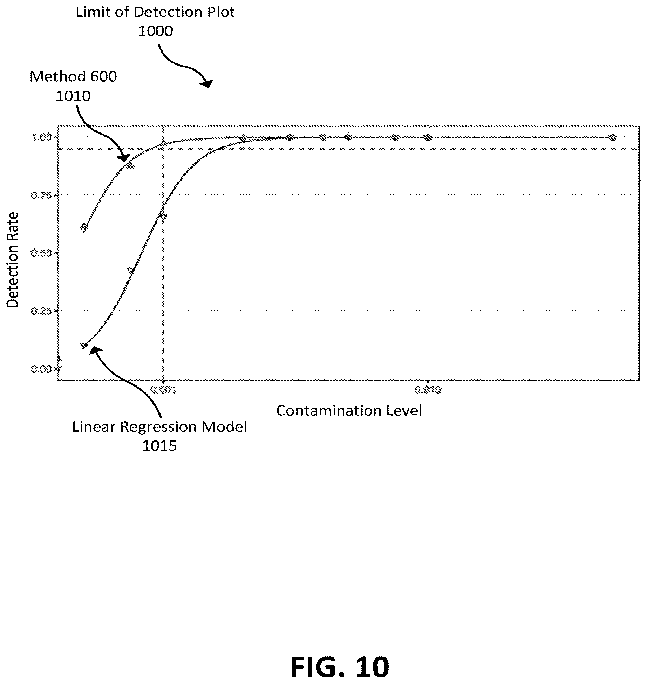

FIG. 10 is a limit of detection plot 1000 showing the limit of contamination detection obtained using detection workflow 400 (e.g., workflow 600). In plot 1000 the x-axis is the contamination level and the y-axis is the detection rate. Limit of detection for detection workflow 400 was compared against the limit of detection for a robust linear regression model for contamination detection (see, e.g., U.S. Patent Appl. No. 62/460,268, entitled "Detecting cross contamination in sequencing data"). Plot 1000 shows a line 910 of the detection rate obtained using contamination detection workflow 600. The LOD using contamination detection workflow 500 is about 0.1% contamination level. Plot 1000 also shows a line 1015 of the detection rate obtained using the robust linear regression model. The LOD using the linear regression model is about 0.2% contamination level.

## VII. CONTAMINATION DETECTION VALIDATION

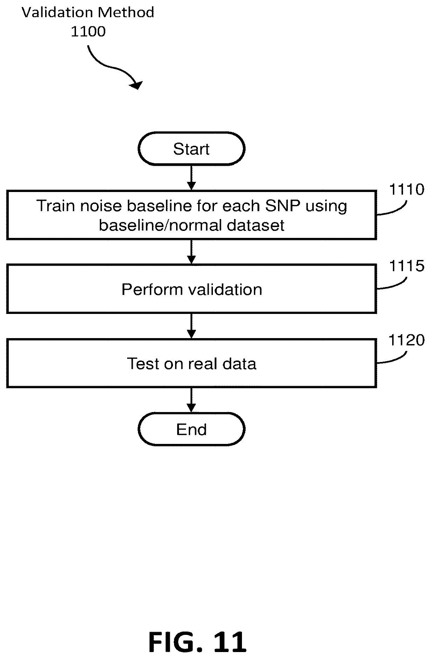

Detection workflow 400 was validated using a three-step process. FIG. 11 illustrates an example of a method 1100 for validating contamination detection workflow 400 (e.g., workflow 600). Validation method 1100 may include, but is not limited to, the following steps.

At a step 1110, a background noise baseline for each SNP is generated using a set of normal training samples (e.g., 80 normal, uncontaminated samples). The noise baseline provides an estimate of the expected noise for each SNP and is used to distinguish a contamination event from a background noise signal. Generation of a noise (contamination) baseline is described in more detail with reference to FIG. 19.

At a step 1115, a 5-fold cross-validation process is performed. For example, datasets of 24 normal samples and *in silico* titrations are partitioned into a validation set and a training set. Here, the contamination levels ranges from 0.05% to 50%. The training set is used to train detection workflow 400 and set a threshold for calling a contamination event versus normal background noise. That is, detection workflow 400 can include a different threshold for each threshold and repeat of an SNP. The threshold is then tested on the validation set. This process is repeated a total of 10 times to identify a final threshold and LOD for calling a contamination event.

At a step 1120, the final threshold and LOD are tested on a real dataset (e.g., a cfDNA dataset from cancer patient samples).

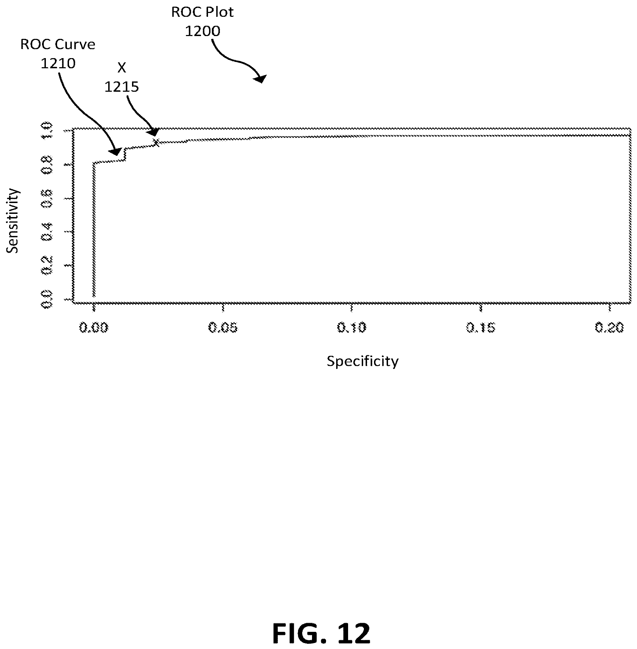

FIG. 12 is a receiver operating characteristic (ROC) plot 1200 showing an example of a ROC curve 1210 generated during cross-validation for threshold evaluation. In plot 1200 the x-axis is the specificity and the y-axis is the sensitivity. The "x" 1215 on ROC curve 1210 indicates the sensitivity and specificity level observed when the optimal threshold was applied. In this example, the optimal threshold (that has specificity above 95% and highest sensitivity) was 70 and the target specificity level was 0.97.

## VIII. Loss OF HETEROZYGOSITY IN A SAMPLE

Loss of heterozygosity (LoH) is an event that occurs in DNA which results in the gain or loss of a piece or whole chromosome, while the other chromosome stays intact, causing a loss of allelic balance at heterozygous sites. In some cases, the chromosome does not stay intact but still indicates LoH if the allelic balance is no longer 1:1. To explain in more simple terms, human DNA contains two copies of the genome, one from each chromosome pair. For the majority of positions in the genome, the base present in each copy is consistent between chromosomes; however, a small percentage may contain different bases than the reference chromosome (e.g., a SNP). Generally, copies from a chromosome pair are balanced. However, in some cases, one chromosome pair's copy of a region can be gained or lost resulting in a region having less or extra copies of one of the chromosomes. When this balance between chromosomes is lost, the region is said to show loss of heterozygosity.

LoH is a common occurrence in cancer and can be used in early cancer detection. Due to the loss of a copy pair, LoH can be read as a homozygous state of an allele. However, LoH does not necessarily imply an actual homozygous state (which would require the presence of two identical alleles in the cell). In particular, LoH creates an allelic state between heterozygote and homozygote (when sequencing cancer samples mixed with normal samples. In this case, if the deviation from heterozygosity is high enough, the allele appears as a contaminated homozygote state for a likelihood model. Thus, LoH in samples can generate false positives in contamination detection workflows (e.g., workflows 400 and 500) based on allele frequency of homozygous SNPs. That is, homozygous SNPs that are an indicator of cancer (via LoH) can also be an indicator of sample contamination. Thus, it is advantageous to remove homozygous SNPs caused by LoH from a sample before executing a contamination detection workflow.

## VIII.A DETERMINING Loss OF HETEROZYGOSITY IN A SAMPLE

In one embodiment, the contamination detection workflow 400 may also detect contamination including samples with loss of heterozygosity. When detecting contamination, the contamination detection workflow calculates the probability that SNPs of the sample indicate loss of heterozygosity and removes the detected SNPs from the sample.

To determine if SNPs of a sample contain loss of heterozygosity, the contamination detection workflow 400 can perform a LoH likelihood test. The LoH likelihood test determines a likelihood that SNPs of the sample are indicative of LoH rather than contamination. The LoH likelihood test includes a null hypothesis, a first hypothesis, and a second hypothesis.

The null hypothesis $H_0$ represents the probability of observing a minor allele depth AD and total depth DP, indicating no loss of heterozygosity with heterozygosity level $\gamma$ ($P(AD|DP, y)$). That is, the null hypothesis $H_0$ indicates the probability that the observed number of minor alleles indicate heterozygosity. Generally, the heterozygosity level $\gamma$ is 0.5 but can be any other value. Here, the heterozygosity level is the ration of reference alleles when the chromosomes are balanced. In one configuration, the probability of observing a minor allele depth indicating no LoH can be represented by a binomial distribution based on the AD, DP, and heterozygosity level $\gamma$. Thus, the null hypothesis $H_0$ can be written as:

$$H_0 = P(AD|DP, \gamma) = dbinom(AD, DP, \gamma) \qquad (14)$$

where AD is the minor allele depth, DP is the total depth (of both major and minor alleles, or "population"), $\gamma$ is the heterozygosity level, and dbinom is a binomial distribution function.

The first hypothesis $H_1$ represents the probability of observing a minor allele depth MAD indicating a LoH at a loss of heterozygosity level $\Delta$. That is, the first hypothesis $H_1$ illustrates the likelihood that the observed number of minor alleles indicate LoH at LoH level $\Delta$. In one example, $\Delta$ is a value determined empirically from estimations using the maximum likelihood models described herein. In one configuration, the probability of observing a minor allele depth indicating LoH can be represented by a binomial distribution based on the MAD, AD, the heterozygosity level $\gamma$, and a tested LoH level $\Delta$. Accordingly, the first hypothesis can be written as:

$$H_1 = P(AD|DP, \text{LoH}_\Delta) = dbinom(AD, DP, \gamma - \Delta) \qquad (15)$$

where AD is the minor allele depth, and DP is the total depth, $\gamma$ is the heterozygosity level, dbinom is a binomial distribution function, and $\Delta$ is a LoH level.

The second hypothesis $H_2$ represents the probability of observing a minor allele depth AD with a given contamination level $\alpha$ of the sample. That is, the second hypothesis $H_2$ gives the probability that the observed number of minor alleles indicate a contamination at level $\alpha$. In one configuration, the probability of observing a minor allele depth AD indicating a contamination at level $\alpha$ can be informed by the probability that the sample is contaminated based on the genotype of the contaminant (cP).

$$H_2 = (1 - \alpha) \cdot P(AD|DP, \gamma) + \dots$$
$$\alpha((1 - \text{cP}) \cdot P(AD|DP, \gamma) + \text{cP} \cdot P(AD|DP, \text{LoH}_\alpha)) \qquad (16)$$

$$H_2 = P(AD|DP, \alpha) = (1 - \alpha) \cdot H_0 + \alpha((1 - \text{cP}) \cdot H_0 + \text{cP} \cdot H_1) \qquad (17)$$

where AD is the minor allele depth, and DP is the total depth, $\gamma$ is the heterozygosity level, $\Delta$ is a LoH level, and cP is the contamination probability.

The LoH likelihood test $L_{\text{LoH}}$ compares the second hypothesis to the first hypothesis for each SNP of the population. For a given SNP, if the first hypothesis $H_1$ less the second hypothesis $H_2$ is above a threshold, then the SNP is removed from the population before determining if the sample is contaminated, otherwise, the SNP remains in the population. That is, if the SNP is more likely to include LoH than contamination, the SNP is removed from the population. The LoH likelihood test can be represented by the expression:

$$L_{LOH}(i) \rightarrow \begin{cases} if\ H_1 - H_2 \geq \varphi & remove\ SNP_i \\ else & maintain\ SNP_i \end{cases} \qquad (18)$$

where $L_{\text{LoH}}(i)$ represents the LoH likelihood test taken for each SNP i, $H_2$ is the second hypothesis, $H_1$ is the first hypothesis, and $\varphi$ is a threshold value. In one example embodiment, threshold value $\varphi$ is determined from simulation tests for contamination detection but can be determined based on any other analysis. In some cases, the LoH likelihood test is performed for a set of SNPs representing large sections of chromosomes.

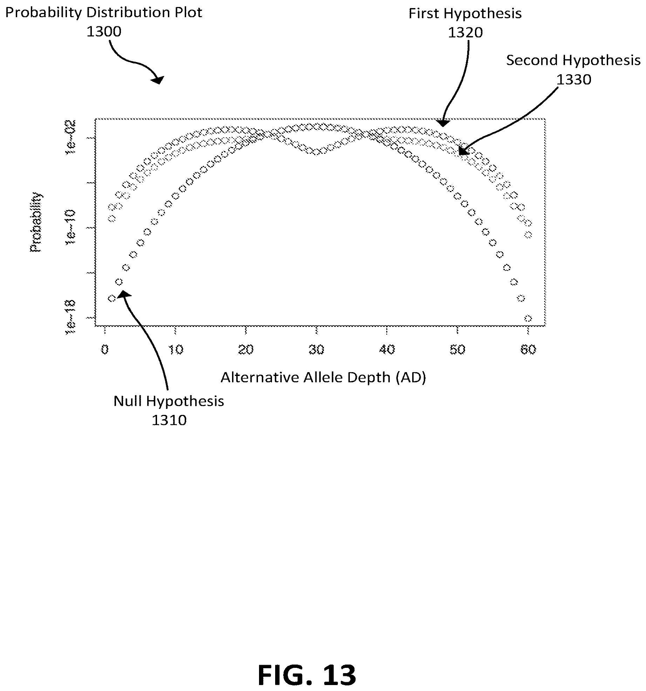

FIG. 13 shows a probability distribution plot 1300 of example probability distributions used for determining the LoH in a sample using the LoH likelihood test $L_{\text{LoH}}$. The x-axis is the allele depth for a sample and the y-axis is the determined probability for a hypothesis of the likelihood test $L_{LoH}$. The line 1310 shows the probability distribution of the null hypothesis that the sample does not include loss of heterozygosity or contamination. Alternatively stated, the line 1310 is the probability of observing a number of reads in the sample which indicate normal heterozygosity. The line 1320 represents the probability distribution of the first hypothesis $H_1$ that the sample includes loss of heterozygosity at a given LoH level $\Delta$. That is, the line 1320 represents the probability of observing a number of reads in the sample that include reads indicative of loss of heterozygosity in the sample. The line 1330 shows the probability distribution of the second hypothesis $H_2$ that the sample is contaminated at a contamination level $\alpha$ with contamination probability cP. That is, the line 1330 represents the probability of observing a number of reads in the sample that show contamination at a contamination level $\alpha$.

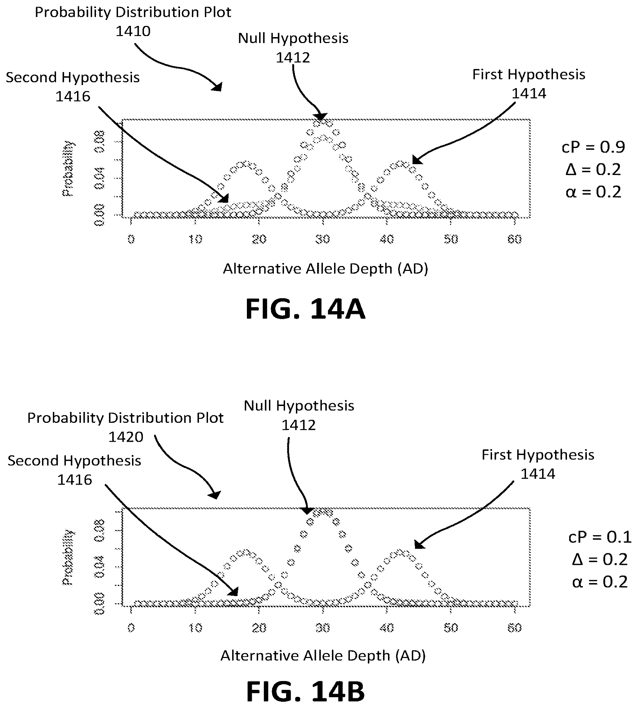

FIG. 14A and 14B show probability distribution plots of the probability distributions for the null hypothesis $H_0$ (line 1412), the first hypothesis $H_1$ (line 1414), and the second hypothesis $H_2$ (red line 1416) using different values for the contamination probability cP, the loss of heterozygosity level $\Delta$, and contamination level $\alpha$. Plot 1410 shows the probability distributions with a contamination probability cP of 0.9, a LoH level $\Delta$ of 0.2, and a contamination level $\alpha$ of 0.2. Plot 1420 shows the probability distributions with a contamination probability cP of 0.1, a LoH level $\Delta$ of 0.2, and a contamination level $\alpha$ of 0.2. For each SNP of the sample, the LoH likelihood test $L_{LoH}$ compares the probability distributions for the first and second hypothesis. For SNPs of the sample with a difference between the LoH probability (Hi) and the contamination probability ($H_2$) above a threshold ($H_1$ - $H_2 \geq \phi$), the LoH likelihood test $L_{LoH}$ can remove the likely LoH SNPs (or sequences including LoH SNPs) based on the analysis.

## VIII.B NEGATIVE BINOMIAL DISTRIBUTION

The negative binomial distribution described above can also be applied to a loss of heterozygosity tests. That is, the null hypothesis, the first hypothesis, and the second hypothesis are:

$$H_0 = P(AD|DP, \gamma) = dbinom(AD, DP, \gamma) \qquad (19)$$

$$H_1 = P(AD|DP, \text{LoH}_\Delta) = dnbinom(AD, DP, \gamma - \Delta) \qquad (20)$$

$$H_2 = P(AD|DP, \alpha) = (1 - \alpha) \cdot H_0 + \alpha((1 - \text{cP}) \cdot H_0 + \text{cP} \cdot H_1) \qquad (21)$$

where the variables are similarly defined as those above.

Accordingly, the LoH likelihood test $L_{LoH}$ can still be employed to filter samples based on a test samples LoH. The $L_{LoH}$ still compares the second hypothesis to the first hypothesis for each SNP of the population. Therefore, $L_{LoH}$ can still be represented by the expression:

$$L_{LOH}(i) \rightarrow \begin{cases} if\ H_1 - H_2 \geq \varphi & remove\ SNP_i \\ else & maintain\ SNP_i \end{cases} \qquad (22)$$

where the variables remain similarly defined as those above. $L_{LoH}$(i) represents the LoH likelihood test taken for each SNP i, $H_2$ is the second hypothesis, $H_1$ is the first hypothesis, and $\varphi$ is a threshold value. In one example embodiment, the threshold value $\varphi$ is determined from simulation tests for contamination detection but can be determined based on any other analysis. In some cases, the LoH likelihood test is performed for a set of SNPs representing large sections of chromosomes.

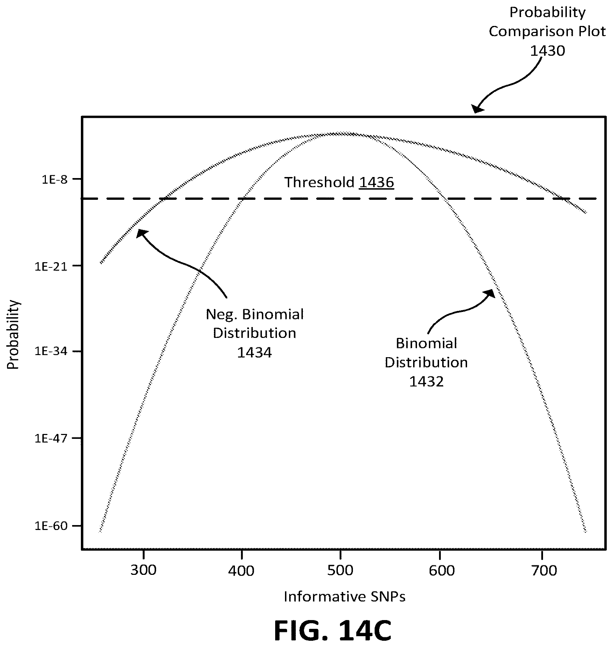

FIG. 14C illustrates a probability comparison plot 1430 comparing loss of heterozygosity models employing a negative binomial distribution to those employing a binomial distribution. In the probability comparison plot 1430, the x-axis provides an ordered list of informative SNPs, and the y-axis is the probability of calling a loss of heterozygosity for those SNPs based on likelihood tests. The probability comparison plot has a distribution line for a negative binomial distribution 1434 and a binomial distribution 1432. The threshold 1436 indicates that SNPs having a probability below the threshold are not considered. Here, the negative binomial distribution provides a higher variance alternative to the binomial distribution.

## VIII.C CONTAMINATION DETECTION USING LOH LIKELIHOOD TESTS

It is important to be able to distinguish between contamination and noise without calling false positives. Detection workflow 400 including workflow 1500 can include detecting LoH in the sample and filtering the samples including the LoH to improve the accuracy of contamination detection.

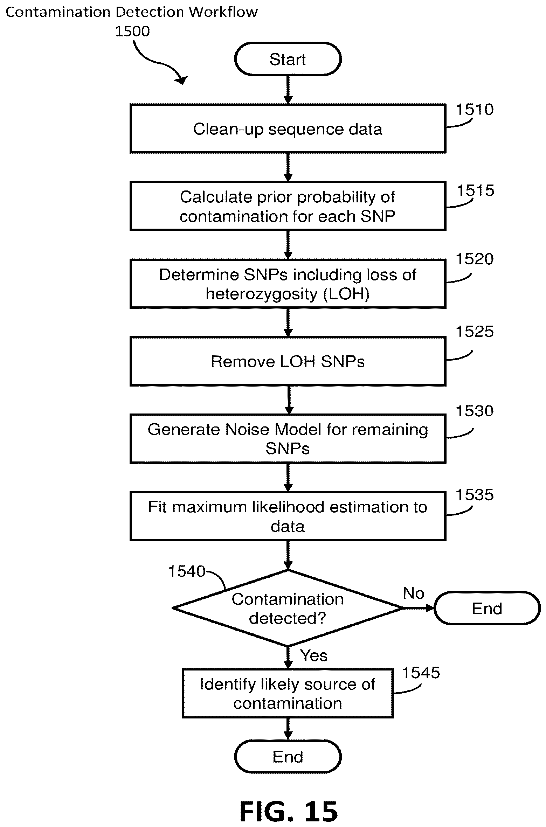

FIG. 15 illustrates a flow diagram of a workflow 1500 for detecting contamination performed in accordance with one embodiment. The contamination detection method of this embodiment includes, but is not limited to, the following steps.

At step 1510, the sequencing data is cleaned up and genotypes are normalized similarly to the clean-up 610 step of the workflow 600 in FIG. 6.

At step 1515, the workflow calculates a prior probability of contamination for each SNP based on the genotype of the contaminant similar to step 615 of FIG. 6.

At step 1520, a loss of heterozygosity likelihood test is performed to determine SNPs that include LoH. The LoH likelihood test is based on the LoH level $\Delta$, a contamination level $\alpha$, and a prior probability of contamination cP for the SNPs.

At step 1525, SNPs that are more likely to include loss of heterozygosity than contamination are removed from the population. In some cases, the difference in likelihood for each SNP is above a threshold level when removed.

At step 1530, a background noise model generates a background noise baseline calculated from a mean allele frequency of the SNPs across healthy samples. The background noise model generates a noise coefficient, which provides an estimate of the expected noise for each of the SNPs.

Following the generation of the noise model, the workflow 1500 proceeds similarly to the workflow 600 of FIG. 6. That is, detection workflow 1500 fits 1535 maximum likelihood estimation to data using likelihood tests, detects 1540 contamination, and identifies 1545 the likely source of contamination analogously to the corresponding steps of detection workflow 600.

Notably, these steps in workflow 1500 are performed using a population of SNPs in which sequences including LoH are removed. The resulting contamination detection 1540 achieves a higher specificity than the workflow 600 of FIG. 6.

## IX. VALIDATION OF CONTAMINATION DETECTION WITH LOH DETECTION

### IX.A IN-VITRO TITRATION

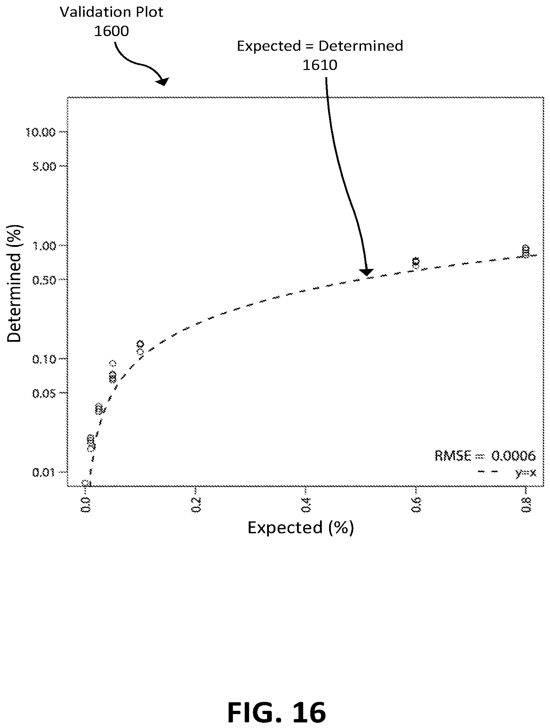

FIG. 16 is a validation plot showing the validation of the detection workflow 1500. Here, in-vitro titration is used to introduce contamination to targeted sequences of 35 samples. Seven contamination levels (0.00%, 0.01%, 0.025%, 0.05%, 0.1%, 0.6% and 0.8%) are introduced into five samples each. The detection workflow 1500 is applied to the 35 samples to call contamination and determine the contamination level. The x-axis of the plot 1600 is the expected contamination level introduced to a sample via titration and the y-axis is the determined contamination level determined by a detection workflow. The dotted line 1610 on the plot 1600 indicates where the expected contamination level is equivalent to the detected contamination level. The data points on plot 1600 show the contamination levels determined by the detection workflow 1500. In this case the detection workflow 1500 called contamination with a specificity of 97.1% (in this example, 34/35). Error in the contamination detection was measured as 0.0006 using root mean square error.

Three alternate contamination workflows known in the art were used to measure the contamination in the samples. The three alternate workflows include: 1) "ContEst: estimating cross-contamination of human samples in next-generation sequencing data" from Cibulskis, K. et. al., Bioinformatics, 2011 (herein referred to as "ContEst"); 2) "Detecting and Estimating Contamination of Human DNA Samples in Sequencing and Array-Based Genotype Data" from Jun, G. et. al., American Journal of Human Genetics, 2012 (herein referred to as "VerifyBamID"); and 3) "Conpair: concordance and contamination estimator for matched tumor-normal pairs" from Bergmann, E.a. et. al., Bioinformatics, 2016 (herein referred to as "Conpair"). The RMSE errors for the three detection workflows were 0.001, 0.03, and 0.003, respectively.

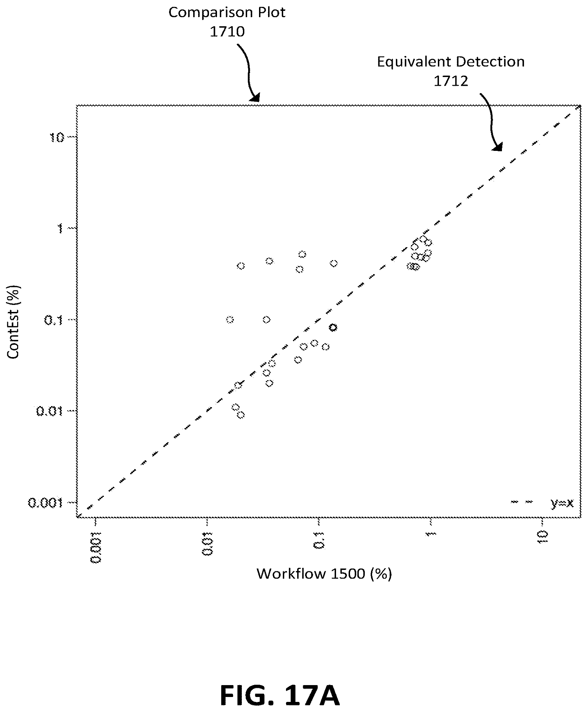

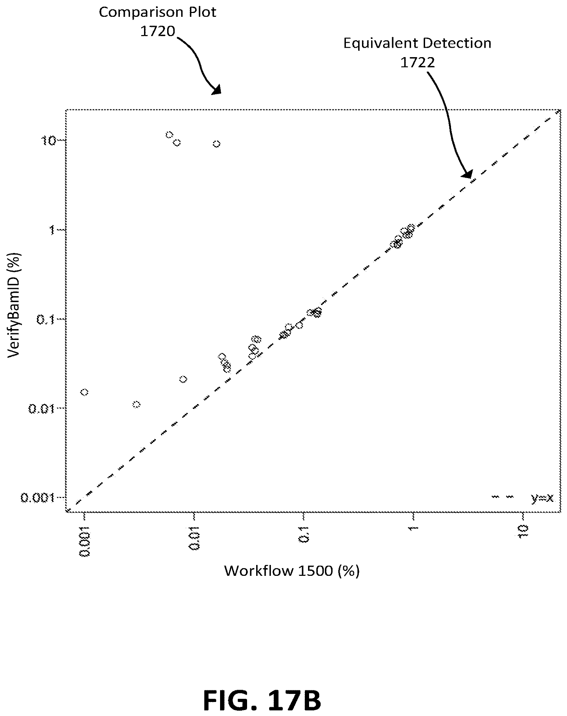

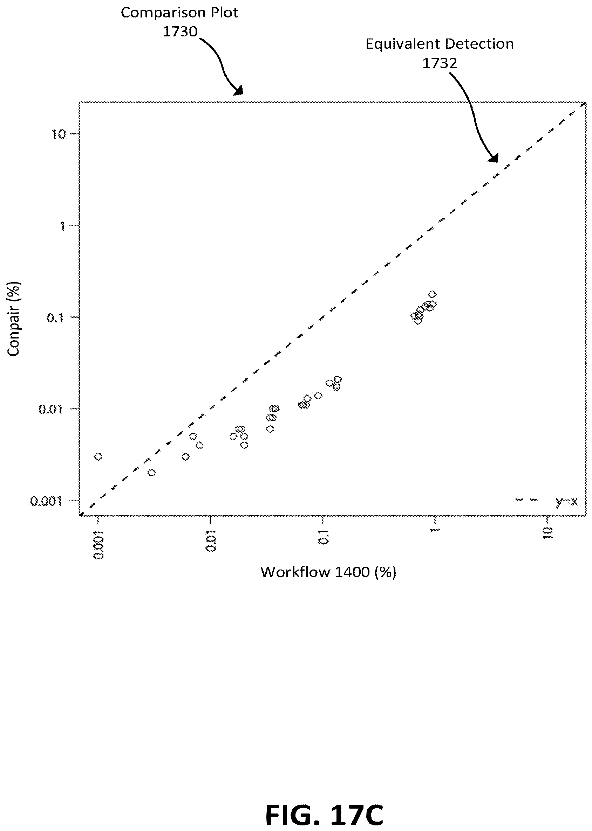

FIGs. 17A-17C are comparison plots illustrating the differences in the detected contamination level between workflow 1500 and the three alternate workflows 1-3. In FIGs. 17A-17C, the detected contamination level for workflow 1500 is the x-axis and the detected contamination level for the alternate workflow is the y-axis. The dotted line is a visual aid representing equivalent contamination detection between the workflow 1500 and the alternate workflow.

In plot 1710, the alternate workflow is ContEst. The plot 1710 illustrates that ContEst is not able to detect contamination below 0.01%. Line 1712 indicates where ContEst and contamination detection workflow 1500 detect contamination equally. Further, the error in detected contamination levels less than 0.5% is high. In plot 1720, the alternate workflow is VerifyBamID. Line 1722 indicates where VerifyBamID and contamination detection workflow 1500 detect contamination equally. The plot illustrates that VerifyBamID is not able to detect contamination below 0.01%. Further, contamination levels below 0.025% can sometimes call abnormally large contamination levels. In plot 1730, the alternate workflow is Conpair. Line 1732 indicates where Conpair and contamination detection workflow 1500 detect contamination equally. The plot 1730 illustrates that Conpair generally determines a contamination level lower than the contamination level determined by workflow 1400.

### IX.B NON-CANCER SAMPLES FROM 1000 GENOMES DATA

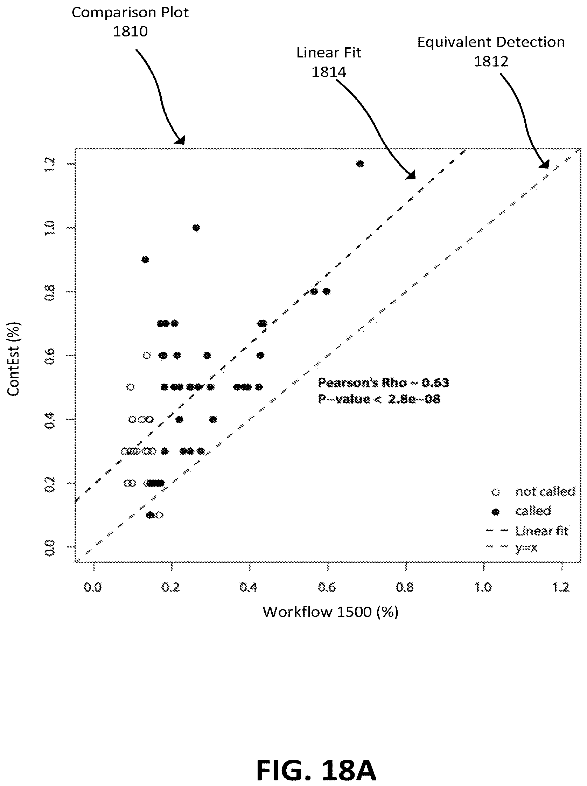

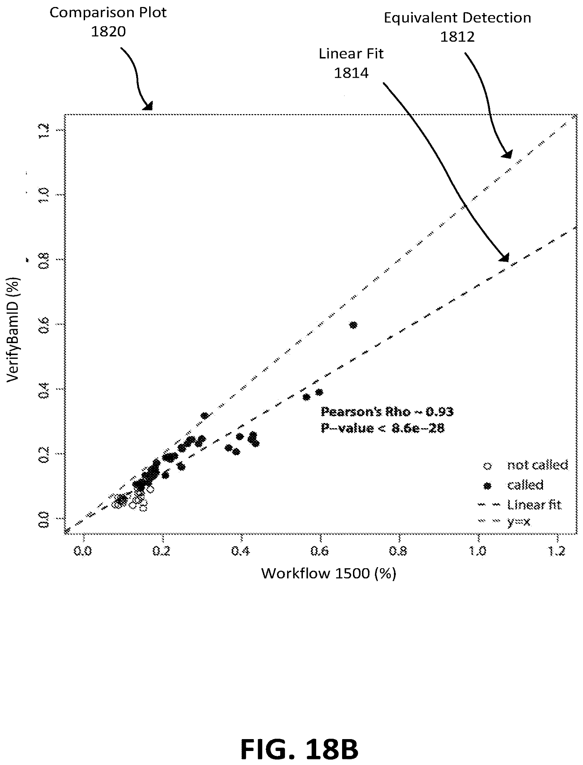

FIG. 18A-18B are comparison plots showing a comparison of contamination calls by detection workflow 1500 and alternate workflows 2 and 3. The detection workflow and alternate workflows are applied to 63 CEU samples from the 1000 genomes project to determine if the sample is contaminated and at what contamination level the sample is contaminated. Here, the samples are known to not include cancer. The closed circles are contamination events detected by both the alternate workflow and the detection workflow 1500. The open circles are contamination events detected by detection workflow 1500 and not the alternate workflow. The x-axis is the contamination level detected by contamination workflow 1500 and the y-axis is the contamination level detected by the alternate workflow. The line 1812 represents equivalent detected contamination levels and the line 1814 represents a linear fit of the determined contamination levels.

Plot 1810 compares alternate workflow 1 to the detection workflow 1500. Plot 1810 illustrates that both workflows call similar contamination events when the contamination level is above ~0.2%. Additionally, ContEst overestimates detected contamination levels when compared to workflow 1400. Plot 1820 compares alternate workflow 2 to the detection workflow 1400. Plot 1820 illustrates that both workflows call similar contamination events when the contamination level is above ~0.1%. Additionally, VeirifyBamID slightly underestimates detected contamination levels when compared to workflow 1500.

IX.C CANCER SAMPLES FROM TUMORS

FIGs. 19A-19C are plots showing a comparison of contamination calls and detected contamination levels by detection workflow 1400 and alternate workflows 1-3. The detection workflow and alternate workflows are applied to 120 tumor samples from Exome sequence data to determine if the sample is contaminated and at what contamination level the sample is contaminated. Here, all the samples are known to include cancer. The x-axis is the contamination level detected by contamination workflow 1500 and the y-axis is the contamination level detected by the alternate workflow. The line 1912 represents equivalent detected contamination levels.

Plot 1910 compares alternate workflow 1 to the detection workflow 1500. Plot 1910 illustrates that alternate workflow 1 overestimates the contamination level compared to workflow 1500. Additionally, ContEst substantially underestimates samples contaminated at less than ~2%. Plot 1920 compares alternate workflow 2 to the detection workflow 1500. Plot 1920 illustrates that alternate workflow 2 overestimates the contamination level compared to workflow 1500. Additionally, VeirifyBamID substantially overestimates some samples with a contamination level less than ~1%. Plot 1930 illustrates that alternate workflow 3 determines similar contamination levels in samples with contamination levels between 0.2% and 2.0%. However, Conpair generally underestimates contamination levels outside that range of contamination levels.

X. BACKGROUND NOISE BASELINE

It is important to distinguish between a contaminant signal and noise. A background noise baseline can be used to distinguish static noise that is generated during sequencing of each SNP. The background noise may be from the sequence context of a variant; some regions will have a higher noise level and some regions will have a lower noise level. In one embodiment, the noise baseline can be determined from the mean allele frequencies observed for a plurality of SNPs across healthy samples.

The background noise baseline is a noise baseline for each SNP that is based on the expected noise across a plurality of normal (uncontaminated) samples. As noted above, the background noise baseline can be captured in the background noise model of baseline batch component 420. Further, generating the contamination noise baseline can be used in any of the various contamination detection methods described herein (e.g., workflow 400 of FIG. 4, workflow 600 of FIG. 6, and workflow 1500 of FIG. 15).

In one embodiment, determining a contamination baseline can be based on the probability of observing a noise level due to errors for a homozygous sample genotype.

X.A BACKGROUND NOISE WORKFLOW

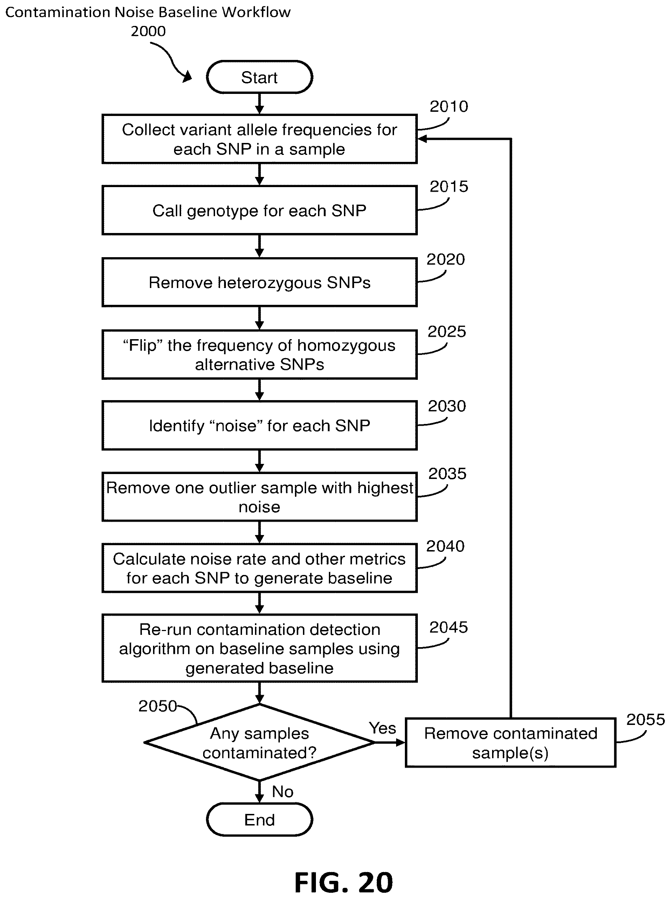

FIG. 20 illustrates a flow diagram of an example workflow 2000 of generating a contamination noise baseline. Workflow 2000 may include, but is not limited to, the following steps.

At a step 2010, variant allele frequencies for each SNP are collected from pileup files from a set of normal baseline samples (n = 80 normal samples).

At a step 2015, the genotype for each SNP in a sample is called. For example, an allele frequency range from about 25% to about 75% is called as a heterozygous allele; an allele frequency from about less than 25% is called as a homozygous reference allele, and an allele frequency from about greater than 75% is called as a homozygous alternative allele.

At a step 2020, the heterozygous SNPs are removed.

At a step 2025, the frequency of each homozygous alternative SNP is flipped subtracting this allele frequency from 1, for example, 99.9% allele frequency becomes 0.1%. Therefore variant allele frequency from this step on corresponds to noise frequency.

At a step 2030, the deviation of the flipped frequency from 0 is determined and identified as "noise" for that SNP.

At a step 2035, for each SNP, one outlier sample with the highest noise is removed.

At a step 2040, the noise rate and other metrics for each SNP are calculated using the remaining samples to generate a baseline. Some example metrics include a heterozygous rate, homozygous rate, and compliance with Hardy-Weinberg equation and observed noise frequency.

At a step 2045, the contamination detection algorithm is run on the baseline samples using the generated baseline.

At a decision step 2050, it is determined whether any of the baseline samples are contaminated. If yes, then workflow 200 proceeds to a step 2055. If no, then the generated baseline becomes the final baseline and workflow 2000 ends.

At step 2055, the contaminated noise baseline sample(s) is removed and workflow 2000 returns to step 2010.

X.B BACKGROUND NOISE DATA

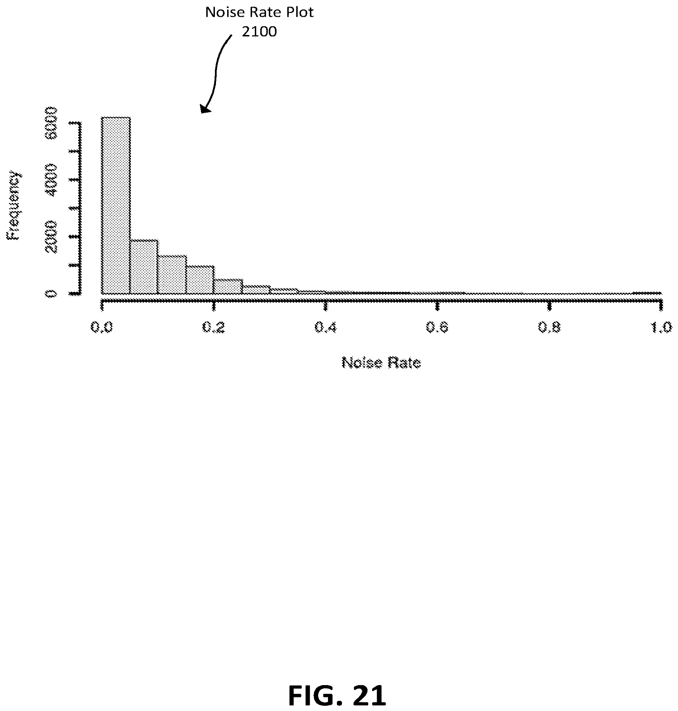

FIG. 21 is a noise rate plot 2100 showing an example of the noise rate of SNPs. The data show that about half of the SNPs (6189 SNPs (53.71%)) had less than 5% of samples with any error. That is, for 95% of samples SNPs were called as the expected value.

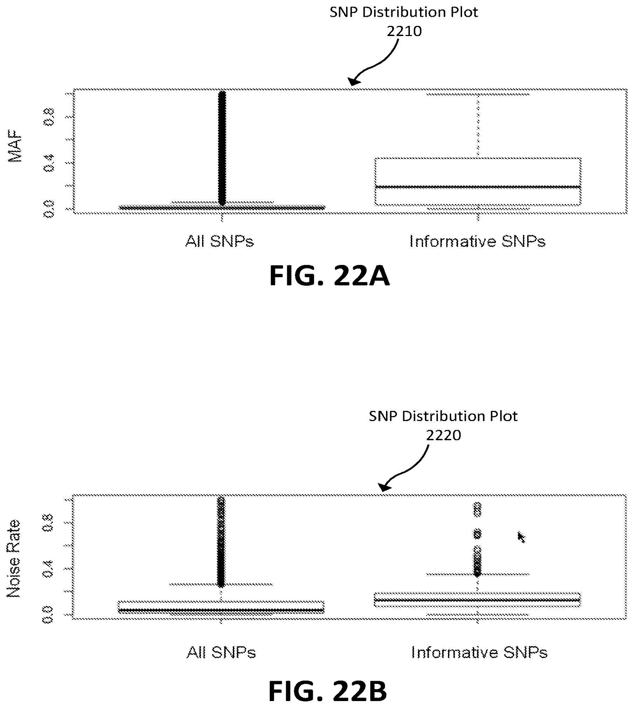

FIG. 22A is a SNP distribution plot 2210 showing the MAF distribution of informative SNPs compared to all SNPs. As shown, informative SNPs have higher MAFs.

FIG. 22B is a SNP distribution plot 2220 showing the noise rate distribution of informative SNPs compared to all SNPs. As shown, informative SNPs have a higher noise rate.

FIG. 22 is a Venn diagram 2300 showing a comparison of the contamination (noise) baselines generated for three separate studies (designated A, B, and C). The data show that the noisy SNP sites (4934) are consistent across different sample sets and are not due to some random calculation (noise rate > 0.05).

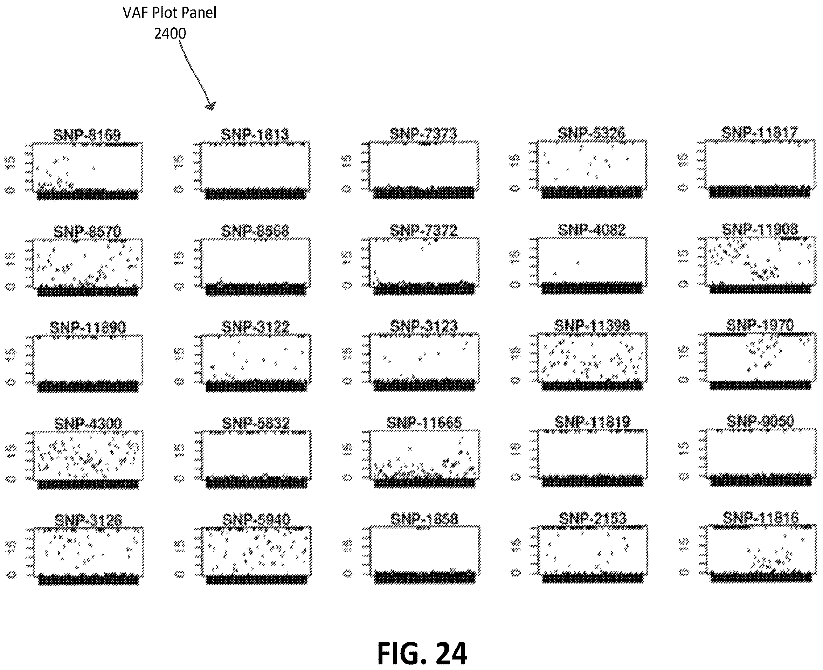

FIG. 24 is a panel 2400 of variant allele frequency (VAF) plots showing the variant allele frequencies for 25 SNPs. For each SNP, 24 samples were assessed. The divergence in the observed variant allele frequency away from zero represents noise. Some of the SNPs have very high noise across samples and may be filtered out during analysis.

Table 2 below shows an example of filtering statistics for an example starting set of 12174 SNPs. SNPs that were filtered out include 651 SNPs that had no coverage in pileup files, 57 SNPs with high error frequencies (more than 0.1% average error), 44 SNPs with high variance, 28 SNPs with low coverage, and 9 SNPs had a higher heterozygous rate (MAF) than expected.

| Table 2: SNP filtering statistics | |
| --- | --- |
| Total SNPs | 12174 |
| No.coverage | 651 |
| High.error | 57 |
| High.variance | 44 |
| Low.coverage | 28 |
| High.Het.Rate | 9 |
| Total.filtered | 769* |
| Kept | 11405 |
| *Some SNPs may be removed by more than one filter | |

X.C BACKGROUND NOISE IN CONTAMINATION DETECTION

Contamination detection workflow 600 of FIG. 6 was tested using a baseline/normal sample dataset (n = 84). Table 2 below shows a summary of the contaminated samples identified in the baseline/normal dataset using contamination detection method 200. Out of 84 baseline/normal samples tested, 4 samples were found to be contaminated at about 0.1% contamination level. For 2 of the samples (B2_6_CR_13 and B6_14_WO44216552592), the sources of contamination were identified (B2_6_CR_13 contaminated with B2_5_WO44216569928, and B6_14_WO44216552592 contaminated with B6_10_WO44216575078). The contaminated samples in the baseline/normal sample dataset were not detected using a robust linear regression model with a LOD of about 0.2%.

| Table 3: Contaminated samples in baseline/normal dataset | |
|---|---|
| Sample | Source |
| B2_14_CR_08 | Undetermined |
| B2_6_CR_13 | B2_5_WO44216569928 |
| B6_02_WO44216564538 | Undetermined |
| B6_14_WO44216552592 | B6_10_WO44216575078 |

X.D TRI-NUCLEOTIDE CONTEXT ERROR

In some embodiments, the system may employ a noise detection model that accounts for the tri-nucleotide context ("TNC error model") of SNPs in a sample. That is, the TNC error model generates an expected error rate for a substitution error and its flanking nucleotides, rather than just the substitution error itself. For example, an error model can determine a likelihood than an A to G substitution in a test sample is an error based on previous occurrences of that substitution error in the population. A TNC error model determines the likelihood that an A to G substitution within its flanking nucleotides (e.g., AAA to AGA, CAC to CGC, etc.) is an error, and the determination is based on a previous occurrence of that substitution error within its flanking nucleotides in the population. A TNC error model grants more granularity in detecting substitution errors in a contamination detection workflow.

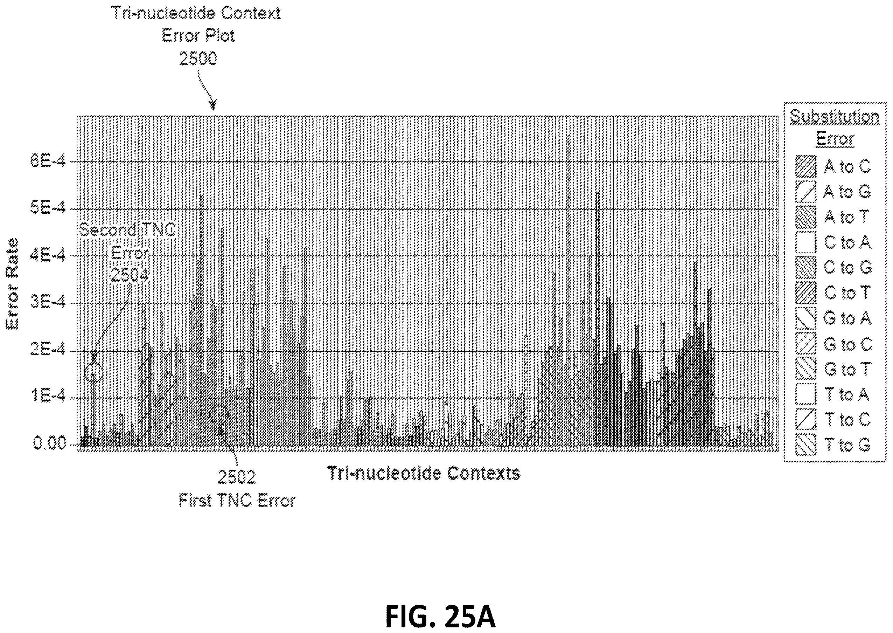

FIG. 25A illustrates a tri-nucleotide context error plot 2500. In a tri-nucleotide error plot 2500, the y axis is the error rate for a specific tri-nucleotide context error, and the x-axis is a series of bars representing tri-nucleotide contexts for substitution errors. The bars on the x-axis are grouped according to color, with each color representing a specific substitution error. In this manner, bars on the x-axis having the same color represent different trinucleotide contexts for that substitution error. For example, the leftmost set of bars represent the trinucleotide contexts for the A to C substitution error (e.g., AAA to ACA, CAC to CCC, etc.). The plot demonstrates the capability of the TNC error model to determine errors (rather than contamination) at a more granular level.

Noticeably, certain types of substitution errors (e.g., a shading band) have higher error rates than other substitution errors. However, within that error type, the substitution error occurs at a much lower rate than the aggregate given its tri-nucleotide context. For example, the first indicated tri-nucleotide context error 2502 is a substation error of this type.

The converse example is also seen. That is, certain substitution errors have lower error rates than other substitution errors. However, within its error type, that substitution error occurs at a much higher rate than the aggregate given its trinucleotide context. For example, the second indicated tri-nucleotide context error 2504 is a substitution error of this type.

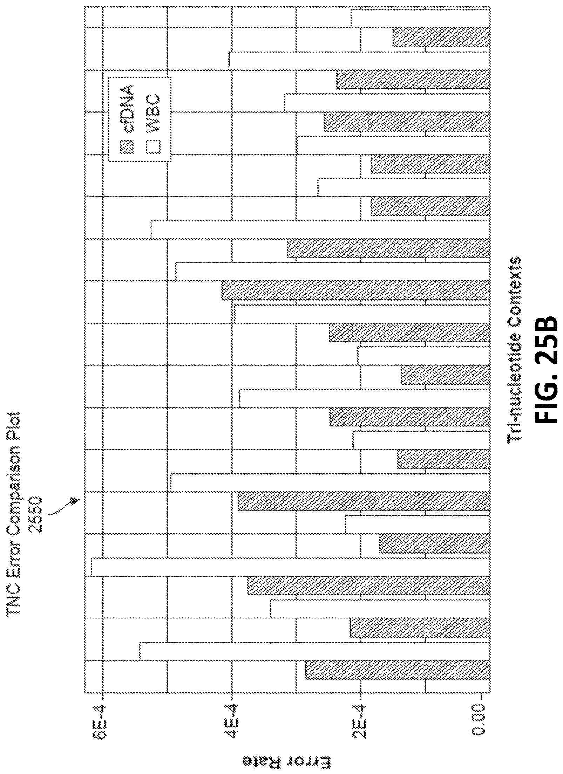

FIG. 25B illustrates a tri-nucleotide context error comparison plot 2550, according to one example embodiment. In the tri-nucleotide error comparison plot, the y-axis is the error rate for a specific tri-nucleotide context error, and the x-axis is a series of bar sets representing tri-nucleotide contexts for substitution errors. Each bar set compares the error rate for cfDNA and WBC DNA for each tri-nucleotide context of a C to A substitution errors. The error rate for WBC DNA is higher than that of cfDNA for all substitution types.

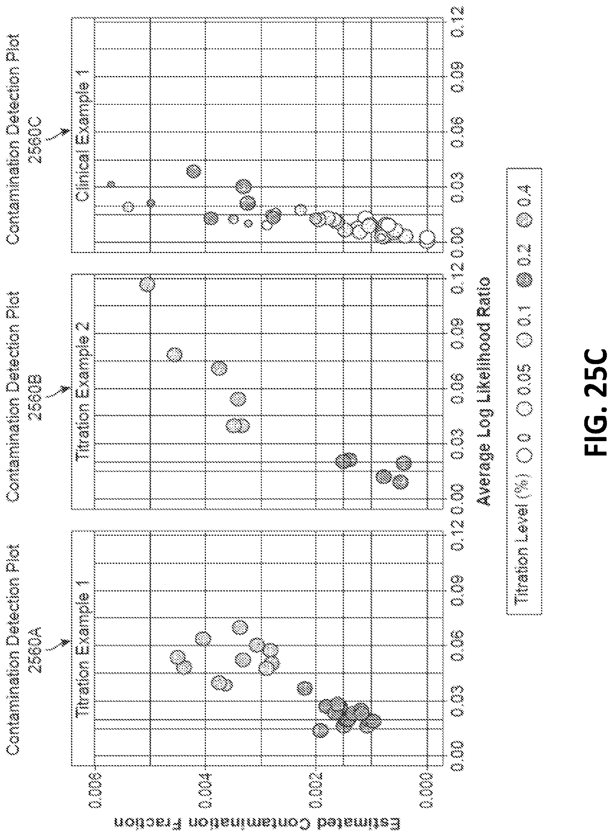

FIG. 25C illustrates a trio of contamination detection plots, according to one example embodiment. The contamination detection plots illustrate the limit of detection for samples employing tri-nucleotide context error. In these plots the x-axis is the average log likelihood ratio, and the y-axis is the estimated contamination fraction. The color of the data points represents the actual contamination fraction. Contamination detection plots 2560A and 2560B map data for samples whose contamination was introduced into samples via titration. Contamination plot 2560C maps data for clinical samples.

Within the samples illustrated herein, the tri-nucleotide context error process was able to detect 100% of samples contaminated at 0.4%, and approximately 50% of the samples contaminated at 0.2%. The tri-nucleotide context error process was unable to detect samples contaminated at 0.1% and 0.05%. Given this, the likely limit of detection for the tri-nucleotide context error process is around 0.3%. Notably, for the clinical samples, the effective conta depth was lower than the titration examples, indicated by the greater spread than the titration data.

XI. OUTPUT FILES

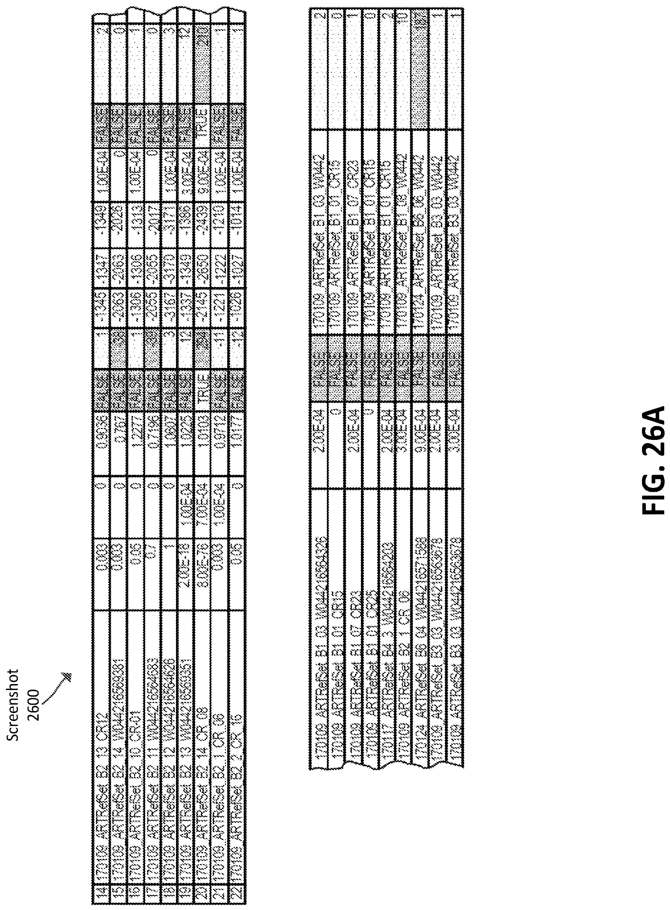

FIG. 26A is a screenshot 2600 of an example of an output file 440 opened in Microsoft (MS) Excel that includes information for each baseline/normal sample tested. Each row represents a sample. In this example, both a robust linear regression model and contamination detection method 200 of FIG. 2 were used to call contamination events, and genotype probability was used as prior probability in a likelihood estimation to identify the source of contamination. Output data for the linear regression model includes, for example, MAFpvalue, MAFcoef, Noisecoef, and Call; output data for contamination detection method 200 (maximum likelihood estimation) includes LhDiff, Lh, Lh0, Lhunif, MaxLh, and LhCall; and output data for the genotype method for contaminant source identification includes bestGtSample, best GtMaxLh, and best GtCall. Samples that are called as contaminated using the linear regression model and contamination detection algorithms are indicated by "TRUE" in column E "Call" and column K "LhCall", respectively.

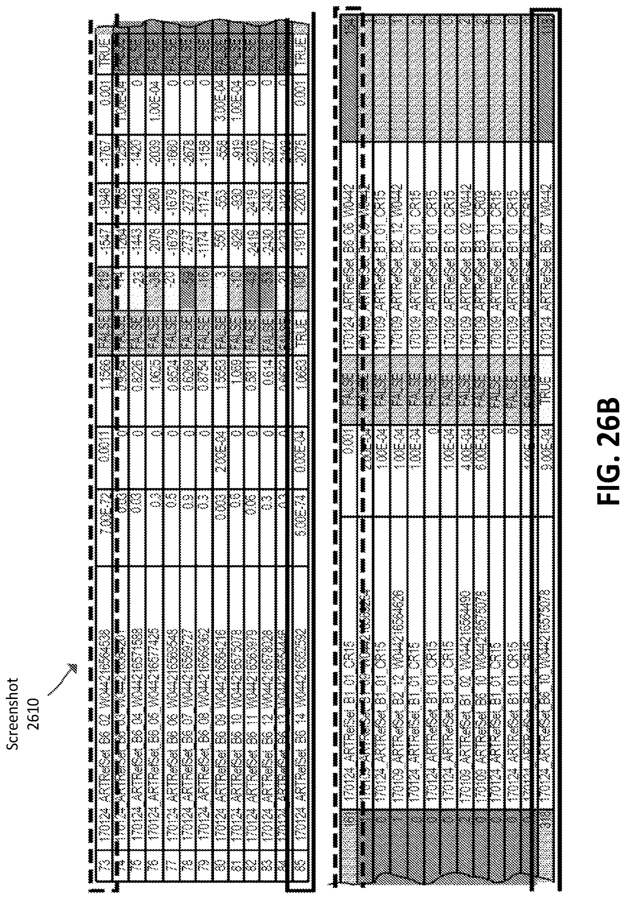

FIG. 26B is a screenshot 2610 of a portion of output file 440 of FIG. 25 that shows the analysis data for two contamination events in the baseline/normal sample dataset. Contamination of sample B6_14_WO44216552592 (row 85, indicated by boxed area) was called with the linear regression method (column E "TRUE") and detection workflow 500 (column K "TRUE"). The likelihood ratio (column F "LhDiff") was 165 compared to zero contamination hypothesis. Using genotype probabilities as prior probabilities in the likelihood estimation, one of the genotypes B6_10_WO44216575078 gave a likelihood ratio of 318 (column L "bestGtLhDiff"), which is significantly higher than the original likelihood ratio of 165. From this difference in likelihood ratios, it can be concluded that the B6_10_WO44216575078 sample is the contaminant.

Contamination of sample B6_02_WO44216564538 (row 73, indicated by dashed box area) was not called with the linear regression method (column E "FALSE"), but was called with contamination detection method 200 (column K "TRUE"). The likelihood ratio (column F "LhDiff") was 219 compared to zero contamination hypothesis. From the original likelihood ratio, it is concluded that the B6_02_WO44216564538 sample is contaminated, but the source of contamination was not identified based on genotype data for the baseline/normal dataset. That is, in this case, the genotype likelihood ratio of 161 is lower than the original likelihood ratio of 219.

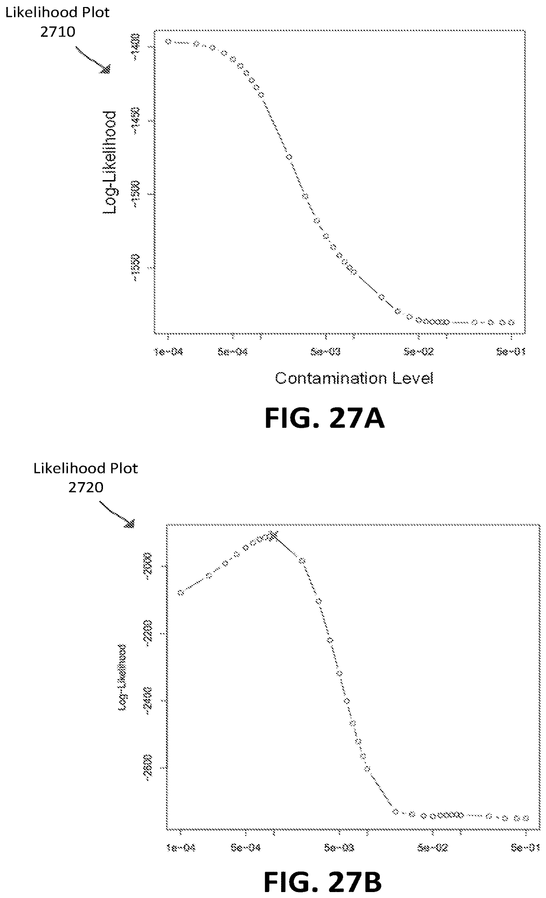

FIG. 27A and 27B are likelihood plots 2710 and 2720, respectively, showing the log-likelihood plots 442 of different hypotheses of contamination levels for baseline/normal samples B1_6_WO44216569493 and B6_14_WO44216552592. Referring to FIG. 27A, plot 2710 shows no likelihood of contamination at different hypothesis levels of sample B1_6_WO44216569493. Referring to FIG. 27B, plot 2720 shows a peak (indicated by an "x") that indicates contamination of sample B6_14_WO44216552592.

As described above, log-likelihood plots (e.g., plot 2710 of FIG. 27A and plot 2720 of FIG. 27B) can be generated as output by the contamination detection workflow 400 of FIG. 4 and provide a ready means to visualize a contamination event.

## XII. FILTERING BASED ON BISULFITE CONVERSION

In some embodiments, the contamination detection workflow can filter call files (such as called SNPs) to minimize the impact of bisulfite conversion. Bisulfite conversion can modify some of the nucleobases in a sequence and lead to false positive calls for contamination detection. More particularly, bisulfite conversion can cause a T to C conversion in an SNP which leads to a higher chance of incorrectly detecting contamination. Therefore, the contamination detection workflow 400 can filter the received sequences and include only SNPs that accurately reflect contamination events. Filtering the received sequences that may have been modified by bisulfite conversion also decreases the limit of detection of the contamination detection workflow 400.

To illustrate, in an example embodiment, the contamination detection workflow can filter received sequences to include only those with A to T and T to A SNPs. In another example, the contamination detection workflow may remove SNPs in a strand specific manner. That is, the workflow only removes those SNPs from a forward (or reverse) sequence read that indicates a methylation error, while maintaining the corresponding reverse (or forward) sequence read for calling contamination events. In this case, which SNPs are removed or maintained may be indicated in a rules table accessible by the contamination detection workflow. Accordingly, the system implementing the workflow may access the rules table and remove or maintain SNPs in sequencing data based on the rules in the table.

### XII.A FILTERING DUAL-STRAND BISULFITE CONVERSION SAMPLES

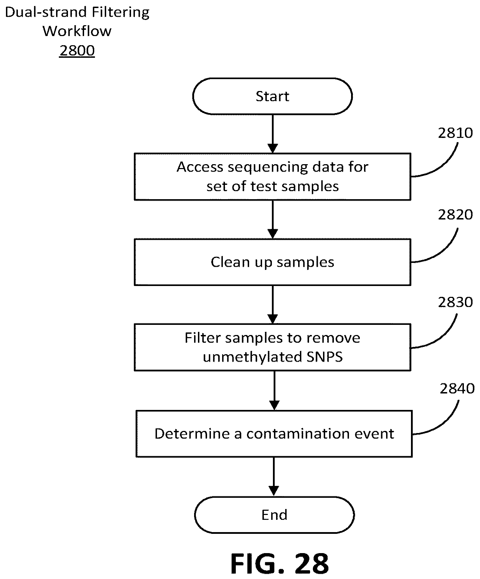

FIG. 28 shows a flow diagram illustrating a dual-strand filtering workflow 2800 performed in accordance the workflow 400 of FIG. 4. The dual-strand filtering workflow 2800 of this embodiment may include, but is not limited to, the following steps. The dual-strand filtering workflow removes sequencing data that may incorrectly call a contamination event due to errors in a methylation process applied to the sequencing data. The workflow may be implemented by a processing system (e.g., processing system 200).

At step 2810, the system accesses a set of test samples. For example, the system may access samples obtained for determining a presence and/or type of a disease in a sample. In preparing the samples for calling disease presence, the samples undergo bisulfite conversion such that methylation may be used to determine disease presence.

At step 2820, the system cleans sequencing data from samples in the analysis window and neutralizes genotypes. For example, data cleaning may include filtering out non-informative SNPs, removing SNPs with no coverage, removing SNPs with high error frequencies (e.g., > 0.1%), removing SNPs with high variance, removing SNPs with a depth less than a threshold, removing any heterozygous SNPs, removing SNPs with low coverage, and removing any SNPs that have a high heterogeneity rate. Any cleaning step described herein may also be applied.

At step 2930, the system filters the sequencing data to remove sequencing data that may indicate a contamination even due to an error in the bisulfite conversion process. For example, the system may remove all A to T or T to A SNPs from the sequencing data because those sites may have been unmethylated during the bisulfite conversion process.

At step 2940, the system determines a probability of contamination for the test samples. To do so, the system may apply, for example, contamination detection workflow 600 or contamination detection workflow 630.

After step 2940, the workflow ends.

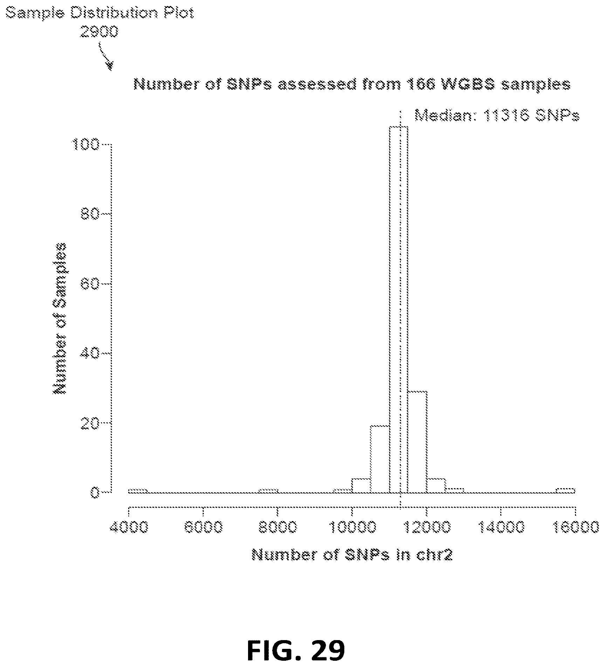

FIG. 29 is a sample distribution plot 2900 illustrating the average number of SNPs in a chromosome after the sample has been filtered based on bisulfite conversion. For example, the sample may be filtered according to dual-strand filtering workflow 2800. The x-axis is the number of SNPs in a chromosome after filtering, and the y axis is the number of samples containing that number of SNPs. Here the threshold mapQ is 60 and threshold baseQ is 36. Phred scale quality values are calculated as *-10log10p* where *p* is the probability of alignment or base being incorrect. For example, if a base has probability 0.01 of being incorrect, the corresponding baseQ will be 20. MapQ considers the repeat structure of the genome, and a low value means the alignment may have multiple candidate locations. BaseQ is calculated by the instrument for a given sequencing cycle, also considering phase errors from previous cycles and represents the confidence in the base call). The only SNPs in the sample are A to T or T to A SNPs. The average number of SNPs in a sample meeting these criteria is 11,316.

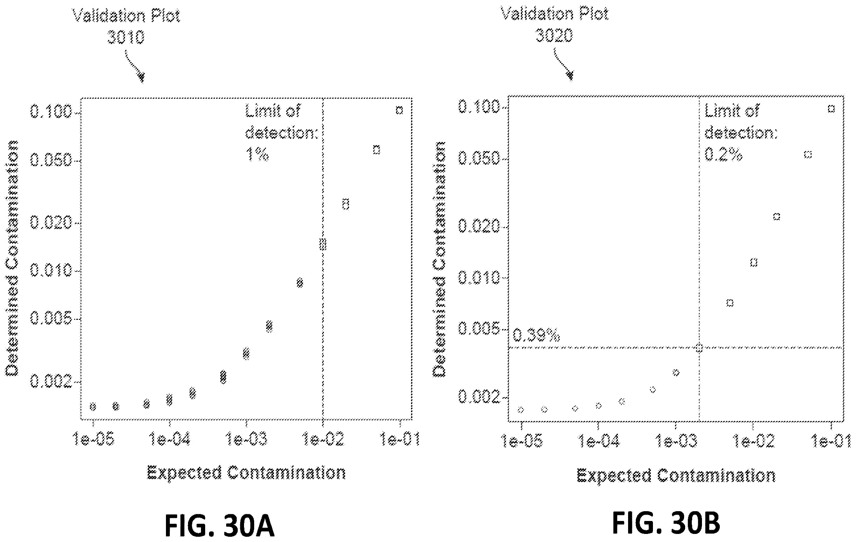

FIGS. 30A and 30B are validation plots showing the improvement in the limit of detection of contamination detection when filtering SNPs associated with bisulfite conversions. In this example, the filtering is done according to dual-strand filtering workflow 2800. Each plot shows a titration experiment with a series of contamination levels between 0.001% and 0.1%. The x-axis is the expected contamination level introduced during titration and the y-axis is the measured contamination level. The limit of detection of contamination is illustrated as a horizontal dashed line in each plot. Plot 3010 shows a limit of detection of ~1% and plot 3020 shows a limit of detection ~0.2% for data using different alignment filtering paradigms.

XII.A FILTERING SINGLE-STRAND BISULFITE CONVERSION SAMPLES

In some embodiments, the system can filter single-strand bisulfite conversion samples rather than dual-strand samples.

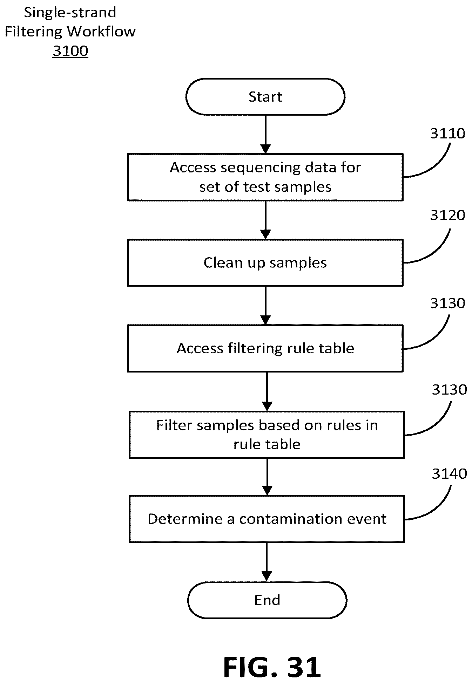

FIG. 31 shows a flow diagram illustrating a dual-strand filtering workflow 3100 performed in accordance the workflow 400 of FIG. 4. The dual-strand filtering workflow 3100 of this embodiment may include, but is not limited to, the following steps. The dual-strand filtering workflow removes sequencing data that may incorrectly call a contamination event due to errors in a methylation process applied to the sequencing data. The workflow may be implemented by a processing system (e.g., processing system 200).

At step 3110, the system accesses a set of test samples. For example, the system may access samples obtained for determining a presence and/or type of a disease in a sample. In preparing the samples for calling disease presence, the samples undergo bisulfite conversion such that methylation may be used to determine disease presence.

At step 3120, the system cleans sequencing data from samples in the analysis window and neutralizes genotypes. For example, data cleaning may include filtering out non-informative SNPs, removing SNPs with no coverage, removing SNPs with high error frequencies (e.g., > 0.1%), removing SNPs with high variance, removing SNPs with a depth less than a threshold, removing any heterozygous SNPs, removing SNPs with low coverage, and removing any SNPs that have a high heterogeneity rate. Any cleaning step described herein may also be applied.

At step 3130, the system accesses a filtering rule table. The filtering rule table includes one or more filtering rulesets for filtering the sequencing data. When implemented, rules of the ruleset filter SNPs in the sequencing data based on their methylation status. In an example, the rule table includes a ruleset for both forward reads and reverse reads. In some cases, the rule table includes a ruleset to be applied to both forward reads and reverse reads.

The tables below show example rulesets from a rule table. Other rulesets are also possible, although they are not enumerated here. Each ruleset includes a column for a reference allele and an alternate allele. The columns indicate nucleobases of an SNP at a given location in a test sample. The reference allele is located on either a forward read or a reverse read. The alternate allele is the nucleobase at the same location on a corresponding read. The corresponding read is one generated by a polymerase chain reaction on the forward or reverse reference read. The tables also have a column indicating whether the allele will be removed or maintained (e.g., not counted / counted) when calling a contamination event.

Table 4 is a ruleset applied to reverse reads of a test sample.

| Table 4: Reverse read ruleset for SNPs | | |
|---|---|---|
| Reference | Alternate | Remove / Maintain |
| C | T | Maintain |
| C | A | Maintain |
| A | C | Maintain |
| T | C | Maintain |
| G | A | Remove |
| G | T | Remove |
| A | G | Remove |
| T | G | Remove |

Table 5 is a ruleset applied to forward reads of a test sample.

| Table 5: Forward read ruleset for SNPs | | |
|---|---|---|
| Reference | Alternate | Remove / Maintain |
| G | A | Maintain |
| G | T | Maintain |
| A | G | Maintain |
| T | G | Maintain |
| C | T | Remove |
| C | A | Remove |
| A | C | Remove |
| T | C | Remove |

Table 6 is a ruleset applied to both forward and reverse reads of a test sample.

| Table 6: Forward and reverse read ruleset for SNPs | | |
|---|---|---|
| Reference | Alternate | Remove / Maintain |
| C | G | Remove |
| G | C | Remove |
| A | T | Maintain |
| T | A | Maintain |

At step 3140, the system determines a probability of contamination for the test samples. To do so, the system may apply, for example, contamination detection workflow 600 or contamination detection workflow 630.

After step 3140, the workflow ends.

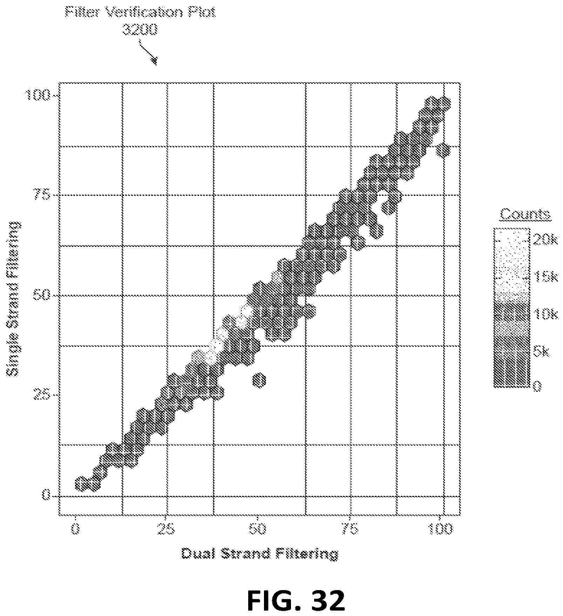

FIG. 32 is a filter verification plot 3200 comparing the single-strand workflow of FIG. 32 to the dual-strand filtering workflow of FIG. 28. In the filter verification lot, the x-axis shows SNPs filtered according to the dual strand workflow, while the y-axis shows SNPs filtered according to the single-strand workflow. The gradient of each location on the plot indicates a sum of the number of SNPs filtered according to the workflows.

Here, the plot shows only A to T and T to A SNPs, which are handled similarly in each of the workflows. Therefore, for a given SNP, if the workflows are functioning correctly, the SNPs should be counted by each workflow the same number of times. Accordingly, the expected data would be a linear plot of counts with a slope of one. Here, the line is approximately linear with each workflow counting SNPs in a similar manner.

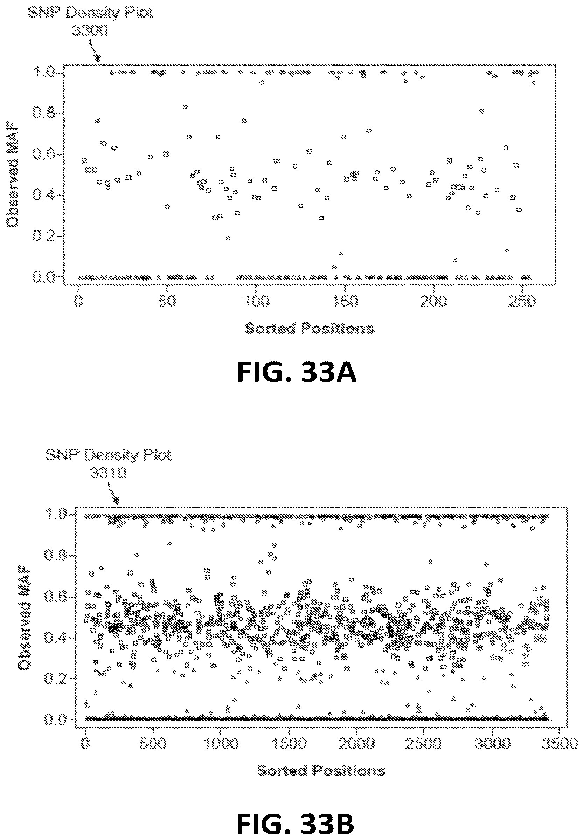

FIG. 33A is a SNP density plot 3300 for a test sample filtered according to the dual-strand workflow, and FIG. 33B is a SNP density plot 3310 for the same sample filtered according to the single-strand workflow. In an SNP density plot, the x-axis indicates the sorted positions of SNPs in the sample remaining after filtering. The y-axis gives the observed minor allele frequency for the given SNP.

In the SNP density plot for the dual-strand workflow 3300, the filtering process maintained 175 non-heterozygous SNPs with an average depth of 174. In the SNP density plot for the single-strand workflow 3310, the filtering process maintained 1545 non-heterozygous remaining SNPs with an average depth of 110. In other words, the dual-strand workflow greatly increased the number of SNPs maintained for contamination detection. Correspondingly, the limit of detection was also decreased.

In some embodiments, the system may employ SNPs from differing sources when determining a contamination event. That is, SNPs used to call a contamination may be filtered according to one or more contamination detection workflows. For example, the SNPs may be filtered using dual-strand workflow 3100 and a contamination filter used to generate PRS SNPs. In this case, the rest of the SNPs targeted in the panel can be used for contamination calling. The SNPS may include PRS regions. Some of the PRS regions targeting some weakly cancer associated SNPs, but also more of regions which are targeting abnormal cancer methylation targets.

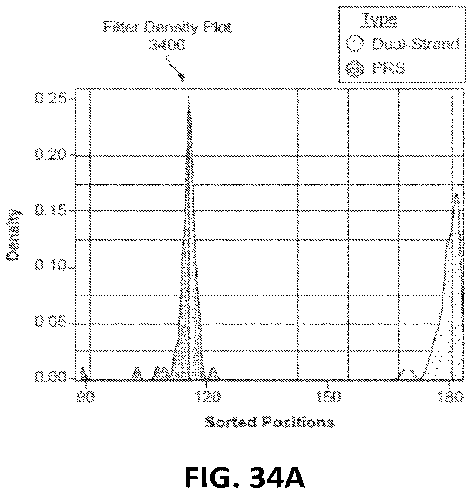

FIG. 34A is a filter density plot comparing SNP density resulting from a dual-strand workflow and a PRS workflow. In the filter density plot 3400, the x-axis is the number of maintained SNPs in a test sample and the y-axis is the density of those SNPs. The color grade of the plot indicates which workflow generated the SNPs. Here, the dual-strand workflow generates more SNPs, although the SNPs have a lower density in the test samples.

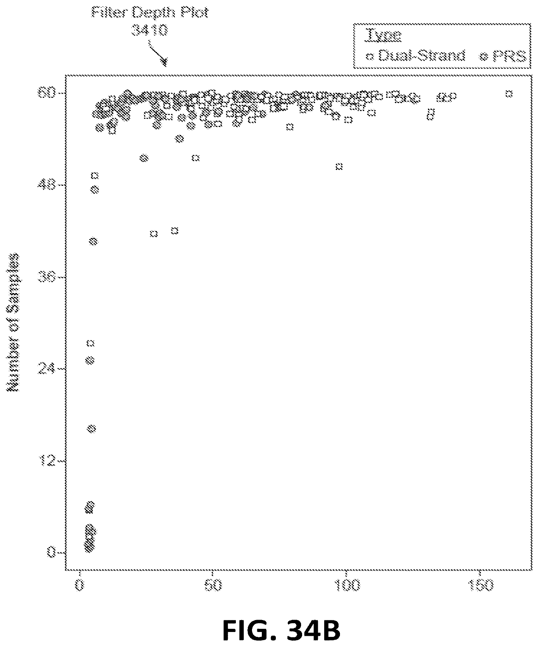

FIG. 34B is a filter depth plot comparing SNP depth resulting from a dual-strand workflow and a PRS workflow. In the filter depth plot 3410, the x-axis is the depth of SNPs, and the y-axis is the number of samples having that depth. Each dot on the plot represents an SNP, and the color grade of the dot indicates which workflow generates the SNP. Here, a vast majority of SNPs from both workflows have a sample depth above a depth threshold (e.g., 15, 20, 30, etc.). The depth threshold is the minimum depth necessary for the SNP to meaningfully indicate the presence or absence of a disease. Further, the dual strand workflow increases a greater number of SNPs above the depth threshold than the PRS workflow. Based on the depth and density of SNPs generated from the samples using the dual-strand and PRS workflows, SNPs from both workflows could be used to detect a disease presence.

## XIII. SNP BLACKLISTS

In some embodiments, the system can filter samples according to SNP blacklists for calling a contamination event. As described below, filtering samples according to SNP blacklists improves the specificity and sensitivity of contamination detection as well as decreasing the limit of detection for contamination.

## XIII.A FILTERING SEQUENCING DATA FOR CONTAMINATION CALLS

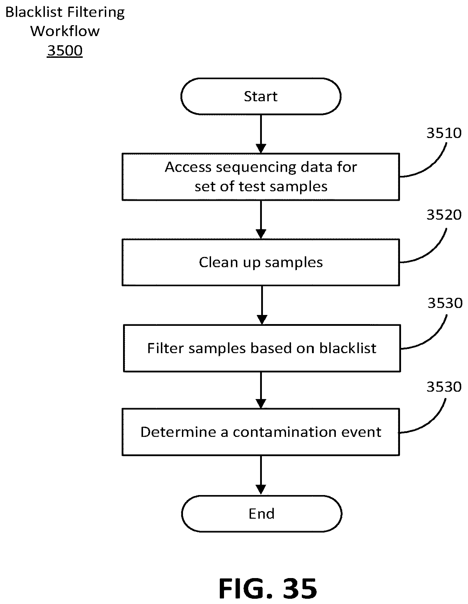

FIG. 35 shows a flow diagram illustrating a blacklist filtering workflow 3500 performed in accordance the workflow 400 of FIG. 4. The blacklist filtering workflow 2800 of this embodiment may include, but is not limited to, the following steps. The blacklist filtering workflow removes sequencing data (e.g., SNPs) that are more likely to incorrectly call a contamination event. The workflow may be implemented by a processing system (e.g., processing system 200).

At step 3510, the system accesses a set of test samples. For example, the system may access samples obtained for determining a presence and/or type of a disease in a sample. In an embodiment, the samples undergo bisulfite conversion to prepare the sample, and the resulting methylation information may be used to determine disease presence.

At step 3520, the system cleans sequencing data and neutralizes genotypes. For example, data cleaning may include filtering out non-informative SNPs, removing SNPs with no coverage, removing SNPs with high error frequencies (e.g., > 0.1%), removing SNPs with high variance, removing SNPs with a depth less than a threshold, removing any heterozygous SNPs, removing SNPs with low coverage, and removing any SNPs that have a high heterogeneity rate.

At step 3530, the system filters SNPs in the test samples according to an SNP blacklist. For example, the system may access a list of SNPs and remove all SNPs on the list from the test samples. The blacklist can be in a library located on the system, accessible by the system, known in the art, or some other SNP blacklist.

At step 3540, the system determines a probability of contamination for the test samples. To do so, the system may apply, for example, contamination detection workflow 600 or contamination detection workflow 630.

After step 3540, the workflow ends.

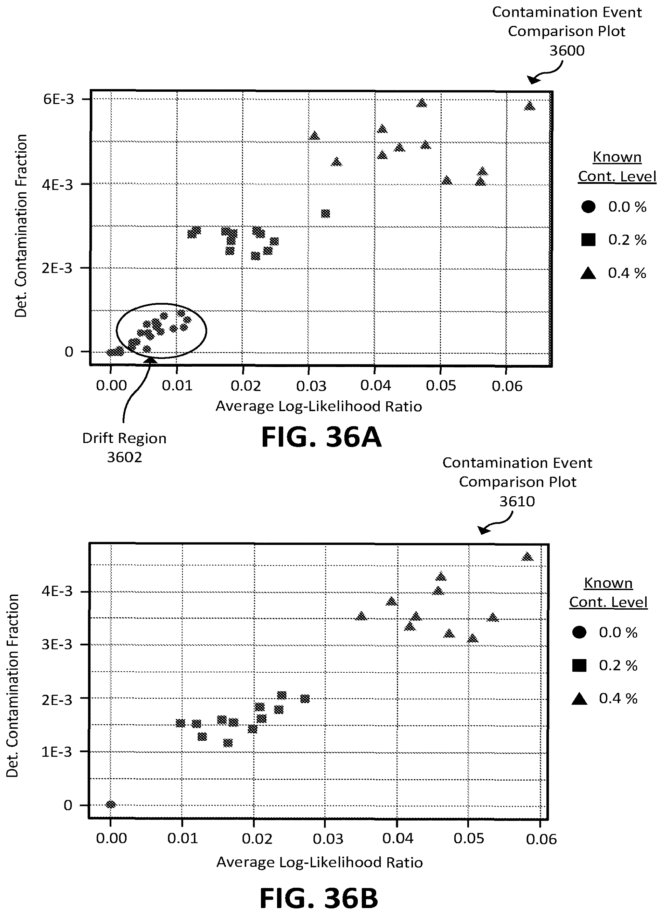

FIG. 36A illustrates a contamination event comparison plot 3600 where the test samples are not filtered according to blacklist filtering workflow 3500, and FIG. 36B illustrates a contamination comparison plot 3610 where the test samples are filtered according to the blacklist filtering workflow 3500.

In a contamination event comparison plot, the x-axis is the average LLR of a test sample, and the y-axis is the determined contamination fraction. Each of the indicia on the graph represents a test sample, and the shape of the indicia gives the known contamination fraction for that sample. Therefore, with perfect contamination detection, an SNP at a given point has a determined contamination fraction equivalent to the known contamination fraction. However, the determined contamination fraction is dissimilar to the known contamination fraction for many of the test samples.

The mismatch between determined and known contamination fractions is evident within the drift region 3602 of the contamination event comparison plot 3600. Without applying SNP blacklist workflow, test samples call a contamination even despite not having contamination. However, as seen in the contamination even comparison plot 3610, the drift region does not occur when applying the SNP blacklist workflow. In other words, uncontaminated samples do not call a contamination event when applying the blacklist filtering workflow 3500.

XIII.B GENERATING BLACKLISTS

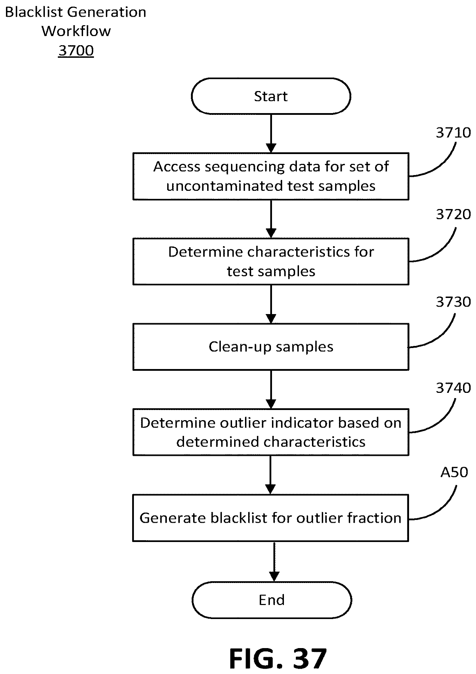

FIG. 37 shows a flow diagram illustrating a blacklist generation workflow 3700 performed in accordance with workflow 400 of FIG. 4. The blacklist generation workflow 3700 of this embodiment may include, but is not limited to, the following steps. The blacklist generation workflow generates SNP blacklists, with SNPs on the blacklist more likely to incorrectly indicate a contamination event in sequencing data. The workflow may be implemented by a processing system (e.g., processing system 200).

At step 3710, the system accesses a cohort of test samples. For example, the system may access samples obtained for determining a presence and/or type of a disease in a sample. Test samples in the cohort are known to not include contamination. **In an** embodiment, the samples undergo bisulfite conversion to prepare the sample, and resulting methylation information may be used to determine disease presence.

At step 3720, the system determines characteristics for the cohort of test samples. For example, the system may determine an observed minor allele frequency for each SNP in the cohort of test samples. **In** some cases, SNPs having a high observed minor allele frequency may indicate a contamination even in uncontaminated samples. Other determinative characteristics are also possible.

At step 3730, the system cleans sequencing data from samples in the cohort and neutralizes genotypes. For example, data cleaning may include filtering out non-informative

SNPs, removing SNPs with no coverage, removing SNPs with high error frequencies (e.g., > 0.1%), removing SNPs with high variance, removing SNPs with a depth less than a threshold, removing any heterozygous SNPs, removing SNPs with low coverage, removing SNPs with 0 allelic fraction, and removing any SNPs that have a high loss of heterogeneity rate.

At step 3740, the system determines an outlier indicator for the cohort of SNPs based on the determined characteristics. For example, the outlier indicator can be a variant threshold level, but other representations of outliers are also possible. The variant threshold may be, for example, 10%, but could be some other threshold level.

At step 3750, the system generates a SNP blacklist using the outlier indicator. That is, the system adds all SNPs within the cohort indicated by the outlier indicator to the SNP blacklist. The SNP blacklist can be maintained on the system, an accessible remote system, or some other system.

To illustrate previous steps, consider an example where the determined characteristics are observed minor allele frequency, a variant threshold is the outlier indicator, and the variant threshold is 10%. Here, SNPs having an observed minor allele frequency above 10% are added to the SNP blacklist. Thus, if an SNP is likely to incorrectly call a contamination event in a test sample, the SNP is added to the blacklist, and those SNPs are removed from the test sample before applying a contamination detection workflow (e.g., contamination detection workflow 630). Therefore, an uncontaminated sample is less likely to call a contamination event.

After step 3740, the workflow ends. The generated SNP blacklist can be used for the blacklist filtering workflow 3500. In various embodiments, the system may apply the blacklist generation workflow to different cohorts of samples such that the SNP blacklist can be targeted to specific sets of test samples. For example, an SNP blacklist can be targeted to a cohort of test samples obtained by a specific panel, a set of individuals, etc.

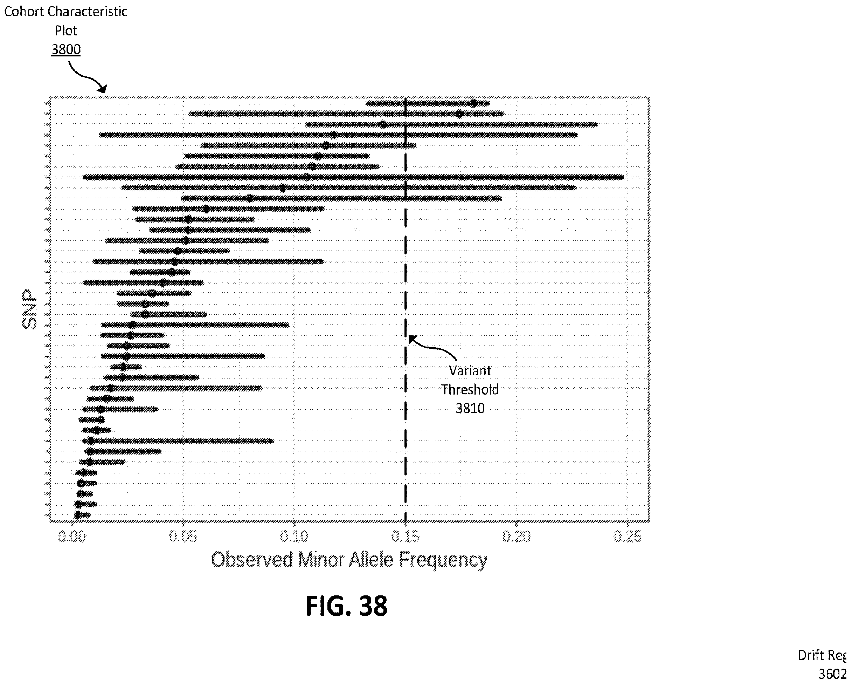

FIG. 38 is a cohort characteristic plot 3800 showing the observed minor allele frequency for SNPs in a cohort of test samples. In the cohort characteristic plot, the x-axis is the observed minor allele frequency, and the y-axis is an ordered list of SNPs in the cohort. Many of the SNPs in the cohort are not shown. In this example, an example outlier fraction 3810 as a variant threshold, e.g., 15% observed minor allele frequency. Here, top two SNPs would be added to an SNP blacklist while, with the remainder being maintained in the population.

When generating an SNP blacklist, several parameters affect how well a resulting blacklist functions to reduce incorrect contamination calls.

One parameter that affects performance is the outlier indicator. Changing the outlier fractions modifies how often a contamination detection workflow calls a contamination event for an uncontaminated example.

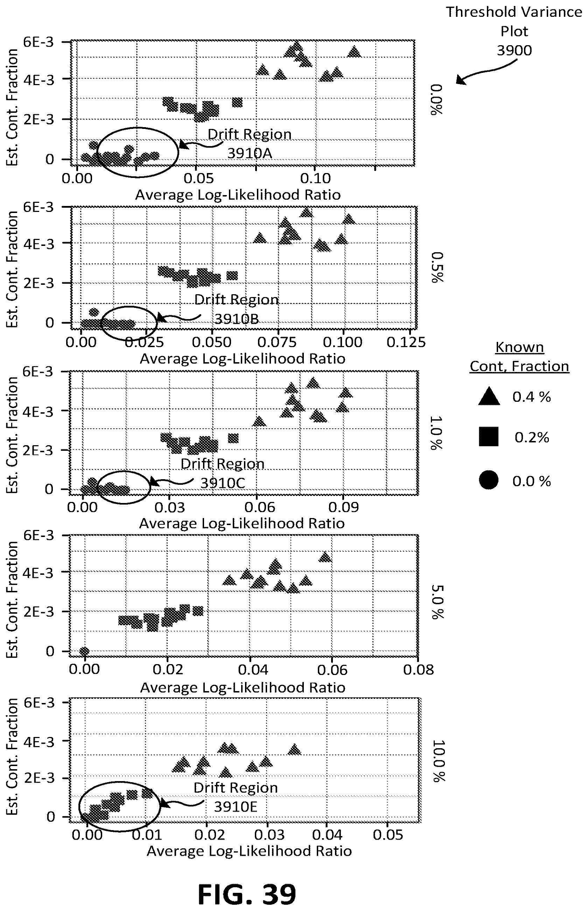

To illustrate, FIG. 39 shows a threshold variance plot 3900 illustrating how changing the variant threshold affects incorrectly calling an uncontaminated sample. Each panel in the outlier variance plot has similar x and y axes. The x-axes are the average LLR of a test sample, and the y-axes are the determined contamination fraction. Each of the indicia on a panel represent a test sample, and the shape of the indicia gives the known contamination fraction for that sample. Each panel has a blacklist generated from different variant thresholds. From top to bottom, the variant thresholds are 0.0%, 0.50%, 1.0%, 5.0%, and 10.0%.

Again, drift regions 3910 in the panels illustrate how accurately a contamination detection workflow calls a contaminated sample. Ideally, there should be a large amount of separation between uncontaminated samples on both the x and y axes. That is, the circles and the triangles should be separated as much as possible. Further, the circles should be localized to 0 on both the x and y axes for accurate calling of uncontaminated samples.

In the drift region 3910A for 0.0% and the drift region 3910E for 10.0% variant threshold samples, the uncontaminated samples are not localized on the x and y axis. In the drift region 3910B for the 0.5% and the drift region 3910C for the 1.0% variant threshold samples, the uncontaminated samples have average log-likelihood ratios greater than 0. Accordingly, the drift region for the 5.0% variant threshold gives the best performance and indicates that samples with a 5.0% variant threshold increase specificity and sensitivity of a contamination detection workflow.

In another embodiment, the system may add SNPs to the blacklist until the blacklist reaches a threshold SNP size. In this case, SNPs are added to the SNP blacklist based on their determined characteristics. For example, if the determined characteristic is the minor allele frequency, SNPs are added to the SNP blacklist according to descending frequency. That is, SNPs with higher minor allele frequencies are added to the blacklist before those with lower minor allele frequencies. SNPs are continuously added to the blacklist in this manner until the blacklist reaches the desired size.

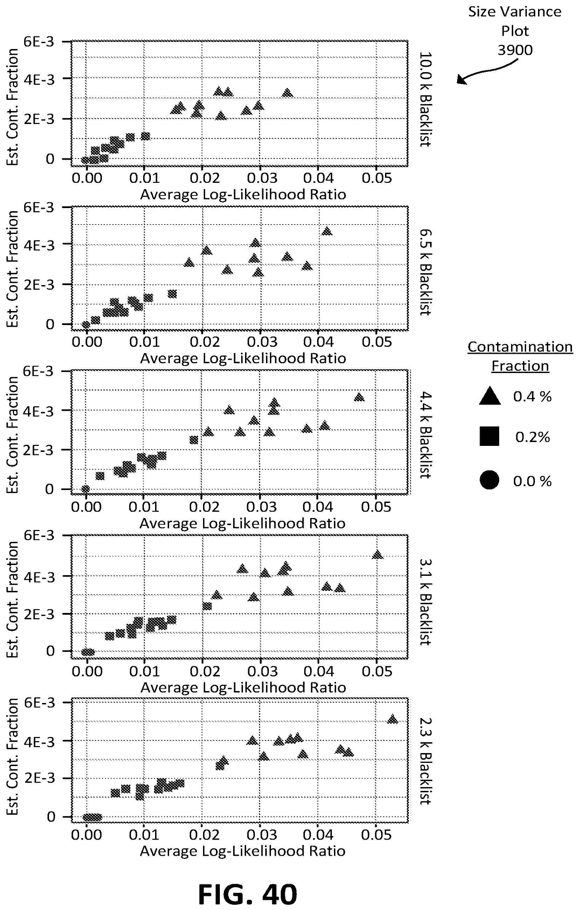

To illustrate, FIG. 40 shows a size variance plot 4000 illustrating how changing the size of the SNP blacklist affects incorrectly calling an uncontaminated sample. Each panel in the outlier variance plot has similar x and y axes. The x-axes are the average LLR of a test sample, and the y-axes are the determined contamination fraction. Each of the indicia on a panel represent a test sample, and the shape of the indicia gives the known contamination fraction for that sample. Each panel has a blacklist of a different size but are generated with the same variant threshold. From top to bottom, the blacklist sizes are 10.0k, 6.5k, 4.4k, 3.1k, and 2.3k SNPs.

Like the threshold variance plot 3900, drift regions 4010 in the panels illustrate how accurately a contamination detection workflow calls a contaminated sample. Ideally, there is a large amount of separation between uncontaminated samples on both the x and y axes. That is, the circles and the triangles should be separated as much as possible. Further, the circles should be localized to 0 on both the x and y axes for accurate calling of uncontaminated samples. Here, blacklists of 3.1k are likely to most accurately call uncontaminated samples.

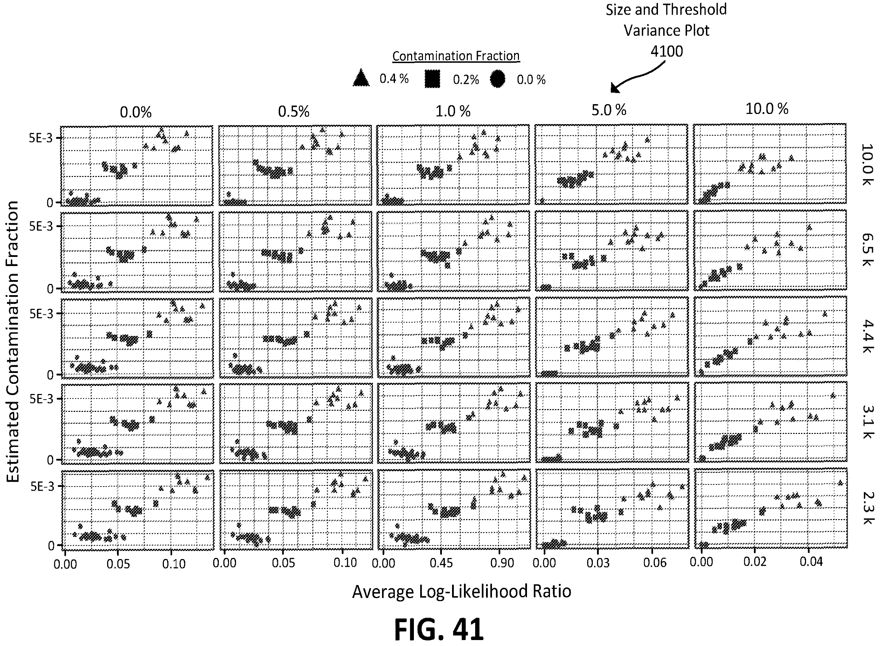

FIG. 41 shows a size and threshold variance plot illustrating how changing both the size of the SNP blacklist and the variant threshold of an outlier indicator affects incorrectly calling an uncontaminated sample.

In the size and threshold variance plot 4100, each panel has similar x and y axes. The x-axes are the average LLR of a test sample, and the y-axes are the determined contamination fraction. Each of the indicia on a panel represent a test sample, and the shape of the indicia gives the known contamination fraction for that sample. Each panel has different variant thresholds and blacklist sizes. From left to right the variant thresholds are 0.0%, 0.50%, 1.0%, 5.0%, and 10.0%, and from top to bottom the blacklist sizes are 10.0k, 6.5k, 4.4k, 3.1k, and 2.3k SNPs. Thus, the middle plot has a variant threshold of 1.0% and a blacklist size of 4.4k SNPs.

Here, the size and threshold variance plot 4100 indicates that a variant threshold of 5.0% and an SNP blacklist size of 10.0k provides the highest accuracy calling uncontaminated samples for the cohort of samples analyzed. However, other examples may find different thresholds and/or blacklist sizes based on the sequencing data in the test samples.

## XIV. AUTOMATICALLY SELECTING CONTAMINATION DETECTION THRESHOLDS

As described herein, the system can determine a contamination event in a sample or set of samples. Generally, the system calls a contamination when the contamination level is above a threshold contamination level (e.g., 0.1%, 0.5%, 1.0%, 3.0 % etc.). However, as the processes for generating and testing those samples for a disease presence changes, the likelihood of a contamination event changes. Therefore, the system may implement a method for automatically modifying the contamination threshold used when calling a contamination event.

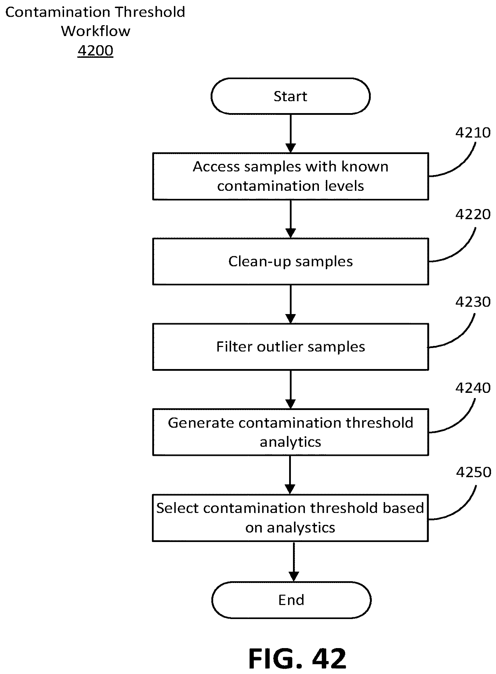

FIG. 42 illustrates a flow diagram illustrating a contamination threshold determination workflow 4200. The determination workflow 4200 of this embodiment includes, but is not limited to, the following steps. The detection workflow determines a contamination threshold for calling a contamination event in one or more samples obtained from a subject, or subjects, for disease diagnosis. The detection workflow can be implemented by a system for sequencing test samples and calling variants (e.g., processing system 200).

At step 4210, the system accesses one or more samples with known contamination levels ("contaminated samples"). The contaminated samples can be simulated, fabricated, or real samples. Simulated samples can include sequencing data from uncontaminated test samples, with that sequencing data manipulated to simulate a contamination event. Fabricated samples can include sequencing data from uncontaminated test samples that have been contaminated in a laboratory setting using in-vitro titration processes. Real samples can include sequencing data form test samples that have been previously determined to include a contamination event. The system then calculates a log likelihood ratio ("LLR") for the contaminated samples using the sequencing data. The LLR is a quantification of how likely a sample is to be contaminated.

At step 4220, the system cleans (or pre-processes) sequencing data of the contaminated samples. Contaminated samples are cleaned up in a manner similar to the process used to determine the presence or absence of a disease. Some example methods of cleaning up samples are described herein. For example, step 610 of FIG. 6A, step 632 of FIG. 6B, etc.

At step 4230, the system filters outlier contaminated samples. Filtering outlier samples can include removing (i) samples having a LLR greater than a threshold LR value (e.g., 1.5, 2.0, 5.0, etc.) (ii) samples having a LLR in the top threshold percentage of determined LLRs, (e.g., 1%, 2%, 5%, etc.) or (iii) samples having a LLR a threshold statistical difference from other samples (e.g., three mean absolute difference from the median, two sigma from mean, etc.). Other filtering of outlier contaminated samples is also possible.

At step 4240, the system determines a set of contamination threshold analytics for the contaminated samples. Contamination threshold analytics quantify how well different contamination thresholds call a contamination event when implemented. That is, for example, the analytics quantify, for a given contamination level, what LLR is sufficient to call a contamination event.

Contamination threshold analytics can include a variety of heuristics quantifying a contamination threshold. For example, the contamination analytics may include, for a given contamination threshold, a limit of detection, a sensitivity of detecting a contamination event, a specificity of detecting a contamination event, an average LLR for test samples, an observed minor allele frequency, etc.

At step 4250, the system determines a contamination threshold to implement based on the contamination threshold analytics. For example, the system can select a contamination threshold that gives the lowest limit of detection. In another example, the system can select a contamination threshold that generates the highest sensitivity at a given specificity.

The system can select a global contamination threshold or select a contamination threshold for different contamination levels. For example, the contamination threshold may be 10.5 E-3 for a contamination level of 5E-3, while it is 10.3 E-3 for a contamination level of 2E-3. In some cases, an administrator of the system can select the contamination threshold.

After the system selects the contamination threshold(s) the workflow ends.

As an example, the following table demonstrates contamination thresholds determined for different contamination levels. The table also show the sensitivity and specificity of detecting contamination events at the specified contamination level using the contamination threshold.

| Table 6: Cont. thresholds from workflow 4200. | | | |
|---|---|---|---|
| Threshold | Cont. Level | Sensitivity | Specificity |
| 0.0102 | 0.001 | 0.569 | 0.954 |
| 0.0105 | 0.001 | 0.569 | 0.985 |
| 0.0102 | 0.002 | 0.846 | 0.954 |
| 0.0103 | 0.002 | 0.846 | 0.985 |
| 0.0102 | 0.005 | 0.985 | 0.954 |
| 0.0105 | 0.005 | 0.985 | 0.985 |
| 0.0102 | 0.010 | 1.000 | 0.954 |
| 0.0105 | 0.010 | 1.000 | 0.985 |

Within this example, the limit of detection was: (i) 3.2 E-3 with a specificity of 0.954 and a sensitivity of 0.954, (ii) 3.2 E-3 with a specificity of 0.984 and a sensitivity of 0.954, and (iii) 3.5 E-3 with a specificity of 1.00 and a sensitivity of 0.95.

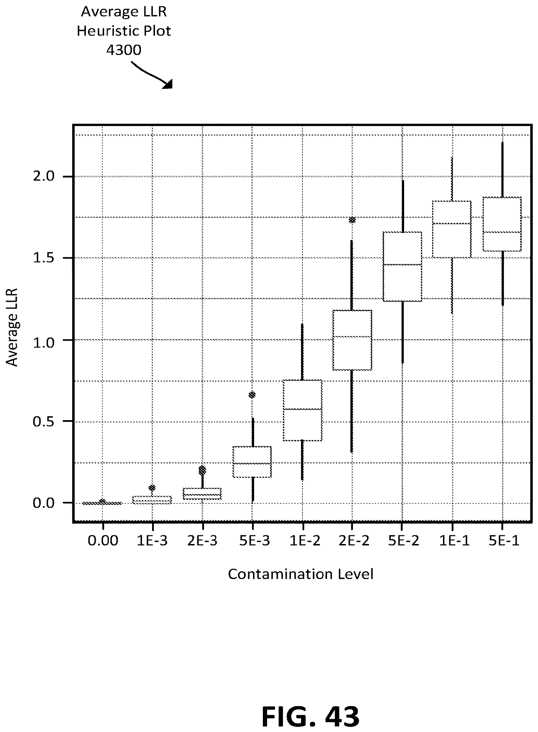

FIG. 43 illustrates an average LLR heuristic plot 4300. The average LLR heuristic plot is a bar and whisker plot with the x-axis illustrating contamination levels in contaminated samples and the y-axis indicating average LLRs of samples. This LLR heuristic plot 4300 shows how selecting a contamination threshold for each contamination level is important. For example, a contamination threshold that calls a contamination for a sample contaminated at 1E-3 based on its LLR would be different than the contamination threshold for a sample contaminated at 1E-1.

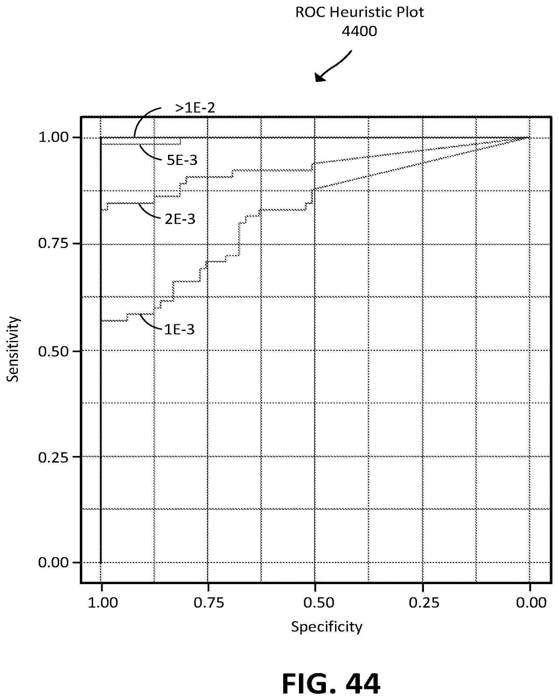

FIG. 44 illustrates a ROC heuristic plot 4400. The ROC heuristic plot 4400 is a ROC plot illustrating specificity on the x-axis and sensitivity on the y-axis. Each of the lines in the ROC heuristic plot represents the capability of the system in calling a contamination event at different contamination levels. Each contamination level has a different contamination threshold.

XV. ADDITIONAL CONSIDERATIONS

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above disclosure.

Some portions of this description describe the embodiments of the invention in terms of algorithms and symbolic representations of operations on information. These algorithmic descriptions and representations are commonly used by those skilled in the data processing arts to convey the substance of their work effectively to others skilled in the art. These operations, while described functionally, computationally, or logically, are understood to be implemented by computer programs or equivalent electrical circuits, microcode, or the like. Furthermore, it has also proven convenient at times, to refer to these arrangements of operations as modules, without loss of generality. The described operations and their associated modules may be embodied in software, firmware, hardware, or any combinations thereof.

Any of the steps, operations, or processes described herein may be performed or implemented with one or more hardware or software modules, alone or in combination with other devices. In one embodiment, a software module is implemented with a computer program product including a computer-readable non-transitory medium containing computer program code, which can be executed by a computer processor for performing any or all of the steps, operations, or processes described.

Embodiments of the invention may also relate to a product that is produced by a computing process described herein. Such a product may include information resulting from a computing process, where the information is stored on a non-transitory, tangible computer-readable storage medium and may include any embodiment of a computer program product or other data combination described herein.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based hereon. Accordingly, the disclosure of the embodiments of the invention is intended to be illustrative, but not limiting, of the scope of the invention, which is set forth in the following claims.

Various embodiments of the invention are recited below as embodiments E1 to E102:

E1. A method for identifying contamination in a test sample, the method comprising:

receiving a plurality of sequence read pairs comprising a plurality of forward strand sequence reads and a plurality of reverse strand sequence reads, wherein:

each of the plurality of sequence read pairs are obtained from methylation-aware sequencing, and
each of the plurality of sequence read pairs comprise at least one single nucleotide polymorphism (SNP);

filtering the plurality of sequence read pairs to generate a population of sequence read pairs by:

filtering forward strand sequence reads according to a first ruleset describing forward strand sequence reads of the test sample that indicate contamination, and
filtering reverse strand sequence reads according to a second ruleset describing reverse strand sequence reads of the test sample that indicate contamination;

determining a prior contamination probability for each SNP of the population of sequence read pairs based on a minor allele frequency for each SNP;
applying a contamination model including at least one likelihood test to a sequence read pair of the population using the contamination probabilities for the SNPs in that sequence read pair, each likelihood test configured to produce a test contamination probability representing the likelihood that the sequence read pair indicates a contamination in the test sample; and
identifying the contamination in the test sample when the test contamination probability is above a likelihood threshold.

E2. The method of E1, further comprising:

determining, for each of the plurality of forward strand sequence reads, a nucleotide base at an SNP site; and
determining, for each of the plurality of corresponding reverse strand sequence reads, a corresponding nucleotide base at the SNP site.

E3. The method of E2, wherein filtering the plurality of sequence read pairs further comprises:

    identifying a sequence read pair in the population, wherein:

        the nucleotide base is a cytosine base at the SNP site in the forward strand sequence read, and the corresponding nucleotide base is a guanine base at the corresponding SNP site in the corresponding reverse strand sequence read;

    removing the sequence read pair from the population.

E4. The method of E2, wherein filtering the plurality of sequence read pairs further comprises:

    identifying a sequence read pair in the population, wherein:

        the nucleotide base is a guanine base at the SNP site in the forward strand sequence read, and the corresponding nucleotide base is a cytosine base at the corresponding SNP site in the corresponding reverse strand sequence read;

    removing the sequence read pair from the population.

E5. The method of E2, wherein filtering the plurality of sequence read pairs further comprises:

    identifying a sequence read pair in the population, wherein:

        the nucleotide base is an adenine base at the SNP site in the forward strand sequence read, and the corresponding nucleotide base is a thymine base at the corresponding SNP site in the corresponding reverse strand sequence read;

    maintaining the sequence read pair in the population.

E6. The method of E2, wherein filtering the plurality of sequence read pairs further comprises:

    identifying a sequence read pair in the population, wherein:

        the nucleotide base is a guanine base at the SNP site in the forward strand sequence read, and the corresponding nucleotide base is a cytosine base at the corresponding SNP site in the corresponding reverse strand sequence read;

    maintaining the sequence read pair in the population.

E7. The method of E2, wherein filtering the plurality of sequence read pairs further comprises:

    identifying a forward strand sequence read from a sequence read pair in the population wherein:

        the nucleotide base is an adenine base at the SNP site in the forward strand sequence read, and the corresponding nucleotide base is a guanine base at the corresponding SNP site in the corresponding reverse strand sequence read;

    maintaining the forward strand sequence read of the sequence read pair in the population.

E8. The method of E2, wherein filtering the plurality of sequence read pairs further comprises:

    identifying a forward strand sequence read from a sequence read pair in the population wherein:

        the nucleotide base is a thymine base at the SNP site in the forward strand sequence read, and the corresponding nucleotide base is a guanine base at the corresponding SNP site in the corresponding reverse strand sequence read;

maintaining the forward strand sequence read of the sequence read pair in the population.

E9. The method of E2, wherein filtering the plurality of sequence read pairs further comprises:

identifying a forward strand sequence read from a sequence read pair in the population wherein:

the nucleotide base is a guanine base at the SNP site in the forward strand sequence read, and
the corresponding nucleotide base is an adenine base at the corresponding SNP site in the corresponding
reverse strand sequence read;

maintaining the forward strand sequence read of the sequence read pair in the population.

E10. The method of E2, wherein filtering the plurality of sequence read pairs further comprises:

identifying a forward strand sequence read from a sequence read pair in the population wherein:

the nucleotide base is a guanine base at the SNP site of the forward strand sequence read, and
the corresponding nucleotide base is a thymine base at the corresponding SNP site in the corresponding
reverse strand sequence read;

maintaining the forward strand of the sequence read pair in the population.

E11. The method of E2, wherein filtering the plurality of sequence read pairs further comprises:

identifying a forward strand sequence read from a sequence read pair in the population wherein:

the nucleotide base is a cytosine base at the SNP site of the forward strand sequence read, and
the corresponding nucleotide base is a thymine base at the corresponding SNP site in the corresponding
reverse strand sequence read;

removing the forward strand of the sequence read pair in the population.

E12. The method of E2, wherein filtering the plurality of sequence read pairs further comprises:

identifying a forward strand sequence read from a sequence read pair in the population wherein:

the nucleotide base is a cytosine base at the SNP site of the forward strand sequence read, and
the corresponding nucleotide base is an adenine base at the corresponding SNP site in the corresponding
reverse strand sequence read;

removing the forward strand of the sequence read pair in the population.

E13. The method of E2, wherein filtering the plurality of sequence read pairs further comprises:

identifying a forward strand sequence read from a sequence read pair in the population wherein:

the nucleotide base is an adenine base at the SNP site of the forward strand sequence read, and
the corresponding nucleotide base is a cytosine base at the corresponding SNP site in the corresponding
reverse strand sequence read;

removing the forward strand of the sequence read pair in the population.

E14. The method of E2, wherein filtering the plurality of sequence read pairs further comprises:

identifying a forward strand sequence read from a sequence read pair in the population wherein:

the nucleotide base is a thymine base at the SNP site of the forward strand sequence read, and
the corresponding nucleotide base is a cytosine base at the corresponding SNP site in the corresponding reverse strand sequence read;

removing the forward strand of the sequence read pair in the population.

E15. The method of E2, wherein filtering the plurality of sequence read pairs further comprises:

identifying a reverse strand sequence read from a sequence read pair in the population wherein:

the nucleotide base is an adenine base at the SNP site of the forward strand sequence read, and
the corresponding nucleotide base is a cytosine base at the corresponding SNP site in the corresponding reverse strand;

maintaining the reverse strand sequence read of the sequence read pair in the population.

E16. The method of E2, wherein filtering the plurality of test sequences further comprises:

identifying a reverse strand sequence read from a sequence read pair in the population, wherein:

the nucleotide base is a thymine base at the SNP site of the forward strand sequence read, and
the corresponding nucleotide base is a cytosine base at the corresponding SNP site in the corresponding reverse strand;

maintaining the reverse strand sequence read of the sequence read pair in the population.

E17. The method of E2, wherein filtering the plurality of sequence read pairs further comprises:

identifying a reverse strand sequence read from a sequence read pair in the population, wherein:

the nucleotide base is a cytosine base at the SNP site of the forward strand sequence read, and
the corresponding nucleotide base is an adenine base at the corresponding SNP site in the corresponding reverse strand;

maintaining the reverse strand sequence read of the sequence read pair in the population.

E18. The method of E2, wherein filtering the plurality of sequence read pairs further comprises:

identifying a reverse strand sequence read from a sequence read pair in the population, wherein:

the nucleotide base is a cytosine base at the SNP site of the forward strand sequence read, and
the corresponding nucleotide base is a thymine base at the corresponding SNP site in the corresponding reverse strand;

maintaining the reverse strand sequence read of the sequence read pair in the population.

E19. The method of E2, wherein filtering the plurality of sequence read pairs further comprises:

identifying a reverse strand sequence read from a sequence read pair in the population, wherein:

the nucleotide base is a guanine base at the SNP site of the forward strand sequence read, and
the corresponding nucleotide base is an adenine base at the corresponding SNP site in the corresponding reverse strand;

removing the reverse strand sequence read of the sequence read pair in the population.

E20. The method of E2, wherein filtering the plurality of sequence read pairs further comprises:

identifying a reverse strand sequence read from a sequence read pair in the population, wherein:

the nucleotide base is a guanine base at the SNP site of the forward strand sequence read, and the corresponding nucleotide base is a thymine base at the corresponding SNP site in the corresponding reverse strand;

removing the reverse strand sequence read of the sequence read pair in the population.

E21. The method of E2, wherein filtering the plurality of sequence read pairs further comprises:

identifying a reverse strand sequence read from a sequence read pair in the population, wherein:

the nucleotide base is a adenine base at the SNP site of the forward strand sequence read, and the corresponding nucleotide base is a guanine base at the corresponding SNP site in the corresponding reverse strand;

removing the reverse strand sequence read of the sequence read pair in the population.

E22. The method of E2, wherein filtering the plurality of sequence read pairs further comprises:

identifying a reverse strand sequence read from a sequence read pair in the population, wherein:

the nucleotide base is a thymine base at the SNP site of the forward strand sequence read, and the corresponding nucleotide base is a guanine base at the corresponding SNP site in the corresponding reverse strand;

maintaining the reverse strand sequence read of the sequence read pair in the population.

E23. The method of E1, wherein filtering the plurality of sequence read pairs to generate the population of sequence read pairs further comprises: removing sequence read pairs comprising one or more nucleotide bases at one or more SNP sites included in an SNP removal table, the SNP removal table indicating SNPs that inaccurately indicate the contamination.

E24. The method of E1, wherein filtering the plurality of sequence read pairs to generate the population of sequence read pairs further comprises: removing sequence read pairs comprising one or more corresponding nucleotide bases at one or more corresponding SNP sites included in an SNP removal table, the SNP removal table indicating SNPs that inaccurately indicate the contamination.

E25. The method of E1, wherein applying a contamination model comprises: applying a negative binomial probability distribution to the population.

E26. The method of E1, wherein the test sample is a plasma sample.

E27. The method of E1, wherein the test sample comprises a plurality of cell-free DNA molecules.

E28. The method of E1, wherein the plurality of sequence read pairs obtained from methylation-aware sequencing comprise a plurality of cell-free DNA (cfDNA) molecules treated such that unmethylated cytosine bases in the cfDNA molecules are converted to uracil bases, and wherein the sequence read pairs are the treated cfDNA molecules.

E29. The method of E1, wherein the plurality of sequence read pairs obtained from methylation-aware sequencing comprise a plurality of cell-free DNA (cfDNA) molecules treated with sodium bisulfite, and wherein the sequence read pars are the treated cfDNA molecules.

E30. The method of E1, wherein the plurality of sequence read pairs obtained from methylation-aware sequencing comprise a plurality of cell-free DNA (cfDNA) molecules treated with a cytidine deaminase and sequencing the treated cfDNA molecules.

E31. The method of E1, wherein the plurality of sequence read pairs obtained from methylation-aware sequencing are obtained via genome-wide bisulfite sequencing.

E32. The method of E1, wherein the plurality of sequence read pairs obtained from methylation-aware sequencing are obtained via paired-end massively parallel sequencing.

E33. The method of E1, further comprising:

enriching the test sample for a plurality of targeted cfDNA molecules before performing the methylation-aware sequencing.

E34. A non-transitory computer-readable storage medium storing computer program instructions executable by a processor of a system to perform steps for identifying contamination in a test sample, the instructions, when executed by the processor, causing the processor to:

receive a plurality of sequence read pairs comprising a plurality of forward strand sequence reads and a plurality of reverse strand sequence reads, wherein:

each of the plurality of sequence read pairs are obtained from methylation-aware sequencing, and
each of the plurality of sequence read pairs comprise at least one single nucleotide polymorphism (SNP);

filter the plurality of sequence read pairs to generate a population of sequence read pairs by:

filtering forward strand sequence reads according to a first ruleset describing forward strand sequence reads of the test sample that indicate contamination, and
filtering reverse strand sequence reads according to a second ruleset describing reverse strand sequence reads of the test sample that indicate contamination;

determine a prior contamination probability for each SNP of the population of

sequence read pairs based on a minor allele frequency for each SNP; apply a contamination model including at least one likelihood test to a sequence read pair of the population using the contamination probabilities for the SNPs in that sequence read pair, each likelihood test configured to produce a test contamination probability representing the likelihood that the
sequence read pair indicates a contamination in the test sample; and identify the contamination in the test sample when the test contamination
probability is above a likelihood threshold.

E35. The non-transitory computer-readable storage medium of E34, wherein the computer program instructions, when executed by the processor, further cause the processor to:

determine, for each of the plurality of forward strand sequence reads, a nucleotide base at an SNP site; and
determine, for each of the plurality of corresponding reverse strand sequence reads, a corresponding nucleotide base at the SNP site.

E36. The non-transitory computer readable storage medium of E35, wherein filtering the plurality of sequence read pairs further causes the processor to execute instructions to:

identify a sequence read pair in the population, wherein:

the nucleotide base is a cytosine base at the SNP site in the forward strand sequence read, and
the corresponding nucleotide base is a guanine base at the corresponding SNP site in the corresponding reverse strand sequence read;

remove the sequence read pair from the population.

E37. The non-transitory computer-readable storage medium of E35, wherein filtering the plurality of sequence read pairs further causes the processor to execute instructions to:

identify a sequence read pair in the population, wherein:

the nucleotide base is a guanine base at the SNP site in the forward strand sequence read, and the corresponding nucleotide base is a cytosine base at the corresponding SNP site in the corresponding reverse strand sequence read;

remove the sequence read pair from the population.

E38. The non-transitory computer-readable storage medium of E35, wherein filtering the plurality of sequence read pairs further causes the processor to execute instructions to:

identify a sequence read pair in the population, wherein:

the nucleotide base is an adenine base at the SNP site in the forward strand sequence read, and the corresponding nucleotide base is a thymine base at the corresponding SNP site in the corresponding reverse strand sequence read;

maintain the sequence read pair in the population.

E39. The non-transitory computer-readable storage medium of E35, wherein filtering the plurality of sequence read pairs further causes the processor to execute instructions to:

identify a sequence read pair in the population, wherein:

the nucleotide base is a guanine base at the SNP site in the forward strand sequence read, and the corresponding nucleotide base is a cytosine base at the corresponding SNP site in the corresponding reverse strand sequence read;

maintain the sequence read pair in the population.

E40. The non-transitory computer-readable storage medium of E35, wherein filtering the plurality of sequence read pairs further causes the processor to execute instructions to:

identify a forward strand sequence read from a sequence read pair in the population wherein:

the nucleotide base is an adenine base at the SNP site in the forward strand sequence read, and the corresponding nucleotide base is a guanine base at the corresponding SNP site in the corresponding reverse strand sequence read;

maintain the forward strand sequence read of the sequence read pair in the population.

E41. The non-transitory computer-readable storage medium of E35, wherein filtering the plurality of sequence read pairs further causes the processor to execute instructions to:

identify a forward strand sequence read from a sequence read pair in the population wherein:

the nucleotide base is a thymine base at the SNP site in the forward strand sequence read, and the corresponding nucleotide base is a guanine base at the corresponding SNP site in the corresponding reverse strand sequence read;

maintain the forward strand sequence read of the sequence read pair in the population.

E42. The non-transitory computer-readable storage medium of E35, wherein filtering the plurality of sequence read pairs further causes the processor to execute instructions to:

identify a forward strand sequence read from a sequence read pair in the population wherein:

the nucleotide base is a guanine base at the SNP site in the forward strand sequence read, and the corresponding nucleotide base is an adenine base at the corresponding SNP site in the corresponding reverse strand sequence read;

maintain the forward strand sequence read of the sequence read pair in the population.

E43. The non-transitory computer-readable storage medium of E35, wherein filtering the plurality of sequence read pairs further causes the processor to execute instructions to:

identify a forward strand sequence read from a sequence read pair in the population wherein:

the nucleotide base is a guanine base at the SNP site of the forward strand sequence read, and
the corresponding nucleotide base is a thymine base at the corresponding SNP site in the corresponding reverse strand sequence read;

maintain the forward strand of the sequence read pair in the population.

E44. The non-transitory computer-readable storage medium of E35, wherein filtering the plurality of sequence read pairs further causes the processor to execute instructions to:

identify a forward strand sequence read from a sequence read pair in the population wherein:

the nucleotide base is a cytosine base at the SNP site of the forward strand sequence read, and
the corresponding nucleotide base is a thymine base at the corresponding SNP site in the corresponding reverse strand sequence read;

removing the forward strand of the sequence read pair in the population.

E45. The non-transitory computer-readable storage medium of E35, wherein filtering the plurality of sequence read pairs further causes the processor to execute instructions to:

identify a forward strand sequence read from a sequence read pair in the population wherein:

the nucleotide base is a cytosine base at the SNP site of the forward strand sequence read, and
the corresponding nucleotide base is an adenine base at the corresponding SNP site in the corresponding reverse strand sequence read;

remove the forward strand of the sequence read pair in the population.

E46. The non-transitory computer-readable storage medium of E35, wherein filtering the plurality of sequence read pairs further causes the processor to execute instructions to:

identify a forward strand sequence read from a sequence read pair in the population wherein:

the nucleotide base is an adenine base at the SNP site of the forward strand sequence read, and
the corresponding nucleotide base is a cytosine base at the corresponding SNP site in the corresponding reverse strand sequence read;

remove the forward strand of the sequence read pair in the population.

E47. The non-transitory computer-readable storage medium of E35, wherein filtering the plurality of sequence read pairs further causes the processor to execute instructions to:

identify a forward strand sequence read from a sequence read pair in the population wherein:

the nucleotide base is a thymine base at the SNP site of the forward strand sequence read, and
the corresponding nucleotide base is a cytosine base at the corresponding SNP site in the corresponding reverse strand sequence read;

remove the forward strand of the sequence read pair in the population.

E48. The non-transitory computer-readable storage medium of E35, wherein filtering the plurality of sequence read pairs further causes the processor to execute instructions to:

identify a reverse strand sequence read from a sequence read pair in the population wherein:

the nucleotide base is an adenine base at the SNP site of the forward strand sequence read, and the corresponding nucleotide base is a cytosine base at the corresponding SNP site in the corresponding reverse strand;

maintain the reverse strand sequence read of the sequence read pair in the population.

E49. The non-transitory computer-readable storage medium of E35, wherein filtering the plurality of test sequences further causes the processor to execute instructions to:

identify a reverse strand sequence read from a sequence read pair in the population, wherein:

the nucleotide base is a thymine base at the SNP site of the forward strand sequence read, and the corresponding nucleotide base is a cytosine base at the corresponding SNP site in the corresponding reverse strand;

maintain the reverse strand sequence read of the sequence read pair in the population.

E50. The non-transitory computer-readable storage medium of E35, wherein filtering the plurality of sequence read pairs further causes the processor to execute instructions to:

identify a reverse strand sequence read from a sequence read pair in the population, wherein:

the nucleotide base is a cytosine base at the SNP site of the forward strand sequence read, and the corresponding nucleotide base is an adenine base at the corresponding SNP site in the corresponding reverse strand;

maintain the reverse strand sequence read of the sequence read pair in the population.

E51. The non-transitory computer-readable storage medium of E35, wherein filtering the plurality of sequence read pairs further causes the processor to execute instructions to:

identify a reverse strand sequence read from a sequence read pair in the population, wherein:

the nucleotide base is a cytosine base at the SNP site of the forward strand sequence read, and the corresponding nucleotide base is a thymine base at the corresponding SNP site in the corresponding reverse strand;

maintain the reverse strand sequence read of the sequence read pair in the population.

E52. The method of E35, wherein filtering the plurality of sequence read pairs further causes the processor to execute instructions to:

identify a reverse strand sequence read from a sequence read pair in the population, wherein:

the nucleotide base is a guanine base at the SNP site of the forward strand sequence read, and the corresponding nucleotide base is an adenine base at the corresponding SNP site in the corresponding reverse strand;

remove the reverse strand sequence read of the sequence read pair in the population.

E53. The non-transitory computer-readable storage medium of E35, wherein filtering the plurality of sequence read pairs further causes the processor to execute instructions to:

identify a reverse strand sequence read from a sequence read pair in the population, wherein:

the nucleotide base is a guanine base at the SNP site of the forward strand sequence read, and
the corresponding nucleotide base is a thymine base at the corresponding SNP site in the corresponding reverse strand;

remove the reverse strand sequence read of the sequence read pair in the population.

E54. The non-transitory computer-readable storage medium of E35, wherein filtering the plurality of sequence read pairs further causes the processor to execute instructions to:

identify a reverse strand sequence read from a sequence read pair in the population, wherein:

the nucleotide base is a adenine base at the SNP site of the forward strand sequence read, and
the corresponding nucleotide base is a guanine base at the corresponding SNP site in the corresponding reverse strand;

remove the reverse strand sequence read of the sequence read pair in the population.

E55. The non-transitory computer-readable storage medium of E35, wherein filtering the plurality of sequence read pairs further causes the processor to execute instructions to:

identify a reverse strand sequence read from a sequence read pair in the population, wherein:

the nucleotide base is a thymine base at the SNP site of the forward strand sequence read, and
the corresponding nucleotide base is a guanine base at the corresponding SNP site in the corresponding reverse strand;

remove the reverse strand sequence read of the sequence read pair in the population.

E56. The non-transitory computer-readable storage medium of E34, wherein filtering the plurality of sequence read pairs to generate the population of sequence read pairs further causes the processor to execute instructions to: remove sequence read pairs comprising one or more nucleotide bases at one or more SNP sites included in an SNP removal table, the SNP removal table indicating SNPs that inaccurately indicate the contamination.

E57. The non-transitory computer-readable storage medium of E34, wherein filtering the plurality of sequence read pairs to generate the population of sequence read pairs further causes the processor to execute instructions to: remove sequence read pairs comprising one or more corresponding nucleotide bases at one or more corresponding SNP sites included in an SNP removal table, the SNP removal table indicating SNPs that inaccurately indicate the contamination.

E58. The non-transitory computer-readable storage medium of E34, wherein applying a contamination model further causes the processor to execute instructions to:
apply a negative binomial probability distribution to the population.

E59. The non-transitory computer-readable storage medium of E34, wherein the test sample is a plasma sample.

E60. The non-transitory computer-readable storage medium of E34, wherein the test sample comprises a plurality of cell-free DNA molecules.

E61. The non-transitory computer-readable storage medium of E34, wherein the plurality of sequence read pairs obtained from methylation-aware sequencing comprise a plurality of cell-free DNA (cfDNA) molecules treated such that unmethylated cytosine bases in the cfDNA molecules are converted to uracil bases, and wherein the sequence read pairs are the treated cfDNA molecules.

E62. The non-transitory computer-readable storage medium of E34, wherein the plurality of sequence read pairs obtained from methylation-aware sequencing comprise a plurality of cell-free DNA (cfDNA) molecules treated with sodium bisulfite, and wherein the sequence read pars are the treated cfDNA molecules.

E63. The non-transitory computer-readable storage medium of E34, wherein the plurality of sequence read pairs obtained from methylation-aware sequencing comprise a plurality of cell-free DNA (cfDNA) molecules treated with a cytidine deaminase and sequencing the treated cfDNA molecules.

E64. The non-transitory computer-readable storage medium of E34, wherein the plurality of sequence read pairs obtained from methylation-aware sequencing are obtained via genome-wide bisulfite sequencing.

E65. The non-transitory computer-readable storage medium of E34, wherein the plurality of sequence read pairs obtained from methylation-aware sequencing are obtained via paired-end massively parallel sequencing.

E66. The non-transitory computer-readable storage medium of E34, wherein the computer program instructions, when executed by the processor, further cause the processor to:
enrich the test sample for a plurality of targeted cfDNA molecules before performing the methylation-aware sequencing.

E67. A system for identifying contamination in a test sample, the system comprising:

one or more memories storing computer program instructions for identifying contamination in the test sample;
one or more processors configured to execute the computer program instructions for identifying contamination, the computer program instructions, when executed, causing the processor to:

receive a plurality of sequence read pairs comprising a plurality of forward strand sequence reads and a plurality of reverse strand sequence reads, wherein:

each of the plurality of sequence read pairs are obtained from methylation-aware sequencing, and
each of the plurality of sequence read pairs comprise at least one single nucleotide polymorphism (SNP);

filter the plurality of sequence read pairs to generate a population of sequence read pairs by:

filtering forward strand sequence reads according to a first ruleset describing forward strand sequence reads of the test sample that indicate contamination, and
filtering reverse strand sequence reads according to a second ruleset describing reverse strand sequence reads of the test sample that indicate contamination;

determine a prior contamination probability for each SNP of the population of sequence read pairs based on a minor allele frequency for each SNP;
apply a contamination model including at least one likelihood test to a sequence read pair of the population using the contamination probabilities for the SNPs in that sequence read pair, each likelihood test configured to produce a test contamination probability representing the likelihood that the sequence read pair indicates a contamination in the test sample; and
identify the contamination in the test sample when the test contamination probability is above a likelihood threshold.

E68. The system of E67, wherein the computer program instructions, when executed by the processor, further cause the processor to:

determine, for each of the plurality of forward strand sequence reads, a nucleotide base at an SNP site; and
determine, for each of the plurality of corresponding reverse strand sequence reads, a corresponding nucleotide base at the SNP site.

E69. The system of E68, wherein filtering the plurality of sequence read pairs further causes the processor to execute instructions to:

identify a sequence read pair in the population, wherein:

the nucleotide base is a cytosine base at the SNP site in the forward strand sequence read, and
the corresponding nucleotide base is a guanine base at the corresponding SNP site in the corresponding reverse strand sequence read;

remove the sequence read pair from the population.

E70. The system of E68, wherein filtering the plurality of sequence read pairs further causes the processor to execute instructions to:

identify a sequence read pair in the population, wherein:

the nucleotide base is a guanine base at the SNP site in the forward strand sequence read, and
the corresponding nucleotide base is a cytosine base at the corresponding SNP site in the corresponding reverse strand sequence read;

remove the sequence read pair from the population.

E71. The system of E68, wherein filtering the plurality of sequence read pairs further causes the processor to execute instructions to:

identify a sequence read pair in the population, wherein:

the nucleotide base is an adenine base at the SNP site in the forward strand sequence read, and
the corresponding nucleotide base is a thymine base at the corresponding SNP site in the corresponding reverse strand sequence read;

maintain the sequence read pair in the population.

E72. The system of E68, wherein filtering the plurality of sequence read pairs further causes the processor to execute instructions to:

identify a sequence read pair in the population, wherein:

the nucleotide base is a guanine base at the SNP site in the forward strand sequence read, and
the corresponding nucleotide base is a cytosine base at the corresponding SNP site in the corresponding reverse strand sequence read;

maintain the sequence read pair in the population.

E73. The system of E68, wherein filtering the plurality of sequence read pairs further causes the processor to execute instructions to:

identify a forward strand sequence read from a sequence read pair in the population wherein:

the nucleotide base is an adenine base at the SNP site in the forward strand sequence read, and
the corresponding nucleotide base is a guanine base at the corresponding SNP site in the corresponding reverse strand sequence read;

maintain the forward strand sequence read of the sequence read pair in the population.

E74. The system of E68, wherein filtering the plurality of sequence read pairs further causes the processor to execute instructions to:

identify a forward strand sequence read from a sequence read pair in the population wherein:

the nucleotide base is a thymine base at the SNP site in the forward strand sequence read, and
the corresponding nucleotide base is a guanine base at the corresponding SNP site in the corresponding reverse strand sequence read;

maintain the forward strand sequence read of the sequence read pair in the population.

E75. The system of E68, wherein filtering the plurality of sequence read pairs further causes the processor to execute instructions to:

identify a forward strand sequence read from a sequence read pair in the population wherein:

the nucleotide base is a guanine base at the SNP site in the forward strand sequence read, and
the corresponding nucleotide base is an adenine base at the corresponding SNP site in the corresponding
reverse strand sequence read;

maintain the forward strand sequence read of the sequence read pair in the population.

E76. The system of E68, wherein filtering the plurality of sequence read pairs further causes the processor to execute instructions to:

identify a forward strand sequence read from a sequence read pair in the population wherein:

the nucleotide base is a guanine base at the SNP site of the forward strand sequence read, and
the corresponding nucleotide base is a thymine base at the corresponding SNP site in the corresponding
reverse strand sequence read;

maintain the forward strand of the sequence read pair in the population.

E77. The system of E68, wherein filtering the plurality of sequence read pairs further causes the processor to execute instructions to:

identifying a forward strand sequence read from a sequence read pair in the population wherein:

the nucleotide base is a cytosine base at the SNP site of the forward strand sequence read, and
the corresponding nucleotide base is a thymine base at the corresponding SNP site in the corresponding
reverse strand sequence read;

remove the forward strand of the sequence read pair in the population.

E78. The system of E68, wherein filtering the plurality of sequence read pairs further causes the processor to execute instructions to:

identifying a forward strand sequence read from a sequence read pair in the population wherein:

the nucleotide base is a cytosine base at the SNP site of the forward strand sequence read, and
the corresponding nucleotide base is an adenine base at the corresponding SNP site in the corresponding
reverse strand sequence read;

remove the forward strand of the sequence read pair in the population.

E79. The system of E68, wherein filtering the plurality of sequence read pairs further causes the processor to execute instructions to:

identifying a forward strand sequence read from a sequence read pair in the population wherein:

the nucleotide base is an adenine base at the SNP site of the forward strand sequence read, and
the corresponding nucleotide base is a cytosine base at the corresponding SNP site in the corresponding
reverse strand sequence read;

remove the forward strand of the sequence read pair in the population.

E80. The system of E68, wherein filtering the plurality of sequence read pairs further causes the processor to execute instructions to:

identifying a forward strand sequence read from a sequence read pair in the population wherein:

the nucleotide base is a thymine base at the SNP site of the forward strand sequence read, and the corresponding nucleotide base is a cytosine base at the corresponding SNP site in the corresponding reverse strand sequence read;

remove the forward strand of the sequence read pair in the population.

E81. The system of E68, wherein filtering the plurality of sequence read pairs further causes the processor to execute instructions to:

identify a reverse strand sequence read from a sequence read pair in the population wherein:

the nucleotide base is an adenine base at the SNP site of the forward strand sequence read, and the corresponding nucleotide base is a cytosine base at the corresponding SNP site in the corresponding reverse strand;

maintain the reverse strand sequence read of the sequence read pair in the population.

E82. The system of E68, wherein filtering the plurality of test sequences further causes the processor to execute instructions to:

identify a reverse strand sequence read from a sequence read pair in the population, wherein:

the nucleotide base is a thymine base at the SNP site of the forward strand sequence read, and the corresponding nucleotide base is a cytosine base at the corresponding SNP site in the corresponding reverse strand;

maintain the reverse strand sequence read of the sequence read pair in the population.

E83. The system of E68, wherein filtering the plurality of sequence read pairs further causes the processor to execute instructions to:

identify a reverse strand sequence read from a sequence read pair in the population, wherein:

the nucleotide base is a cytosine base at the SNP site of the forward strand sequence read, and the corresponding nucleotide base is an adenine base at the corresponding SNP site in the corresponding reverse strand;

maintain the reverse strand sequence read of the sequence read pair in the population.

E84. The system of E68, wherein filtering the plurality of sequence read pairs further causes the processor to execute instructions to:

identify a reverse strand sequence read from a sequence read pair in the population, wherein:

the nucleotide base is a cytosine base at the SNP site of the forward strand sequence read, and the corresponding nucleotide base is a thymine base at the corresponding SNP site in the corresponding reverse strand;

maintain the reverse strand sequence read of the sequence read pair in the population.

E85. The method of E35, wherein filtering the plurality of sequence read pairs further causes the processor to execute instructions to:

identifying a reverse strand sequence read from a sequence read pair in the population, wherein:

the nucleotide base is a guanine base at the SNP site of the forward strand sequence read, and the corresponding nucleotide base is an adenine base at the corresponding SNP site in the corresponding reverse strand;

remove the reverse strand sequence read of the sequence read pair in the population.

E86. The non-transitory computer-readable storage medium of E35, wherein

filtering the plurality of sequence read pairs further causes the processor to execute instructions to:
identifying a reverse strand sequence read from a sequence read pair in the population, wherein:

the nucleotide base is a guanine base at the SNP site of the forward strand sequence read, and
the corresponding nucleotide base is a thymine base at the corresponding SNP site in the corresponding
reverse strand;

remove the reverse strand sequence read of the sequence read pair in the population.

E87. The system of E68, wherein filtering the plurality of sequence read pairs further causes the processor to execute instructions to:

identifying a reverse strand sequence read from a sequence read pair in the population, wherein:

the nucleotide base is a adenine base at the SNP site of the forward strand sequence read, and
the corresponding nucleotide base is a guanine base at the corresponding SNP site in the corresponding
reverse strand;

remove the reverse strand sequence read of the sequence read pair in the population.

E88. The system of E68, wherein filtering the plurality of sequence read pairs further causes the processor to execute instructions to:

identifying a reverse strand sequence read from a sequence read pair in the population, wherein:

the nucleotide base is a thymine base at the SNP site of the forward strand sequence read, and
the corresponding nucleotide base is a guanine base at the corresponding SNP site in the corresponding
reverse strand;

remove the reverse strand sequence read of the sequence read pair in the population.

E89. The system of E67, wherein filtering the plurality of sequence read pairs to generate the population of sequence read pairs further causes the processor to execute instructions to:
remove sequence read pairs comprising one or more nucleotide bases at one or more SNP sites included in an SNP removal table, the SNP removal table indicating SNPs that inaccurately indicate the contamination.

E90. The system of E67, wherein filtering the plurality of sequence read pairs to generate the population of sequence read pairs further causes the processor to execute instructions to:
remove sequence read pairs comprising one or more corresponding nucleotide bases at one or more corresponding SNP sites included in an SNP removal table, the SNP removal table indicating SNPs that inaccurately indicate the contamination.

E91. The system of E67, wherein applying a contamination model further causes the processor to execute instructions to: apply a negative binomial probability distribution to the population.

E92. The system of E67, wherein the test sample is a plasma sample.

E93. The system of E67, wherein the test sample comprises a plurality of cell-free DNA molecules.

E94. The system of E67, wherein the plurality of sequence read pairs obtained from methylation-aware sequencing comprise a plurality of cell-free DNA (cfDNA) molecules treated such that unmethylated cytosine bases in the cfDNA molecules are converted to uracil bases, and wherein the sequence read pairs are the treated cfDNA molecules.

E95. The system of E67, wherein the plurality of sequence read pairs obtained from methylation-aware sequencing comprise a plurality of cell-free DNA (cfDNA) molecules treated with sodium bisulfite, and wherein the sequence read pars are the treated cfDNA molecules.

E96. The system of E67, wherein the plurality of sequence read pairs obtained from methylation-aware sequencing comprise a plurality of cell-free DNA (cfDNA) molecules treated with a cytidine deaminase and sequencing the treated cfDNA molecules.

E97. The system of E67, wherein the plurality of sequence read pairs obtained from methylation-aware sequencing are obtained via genome-wide bisulfite sequencing.

E98. The non-transitory computer-readable storage medium of E67, wherein the plurality of sequence read pairs obtained from methylation-aware sequencing are obtained via paired-end massively parallel sequencing.

E99. The non-transitory computer-readable storage medium of E67, wherein the computer program instructions, when executed by the processor, further cause the processor to:
enrich the test sample for a plurality of targeted cfDNA molecules before performing the methylation-aware sequencing.

E100. A method for identifying contamination in a test sample, the method comprising:

determining an analysis period for a plurality of sequence batches in which the plurality of sequence batches were sequenced;
accessing a plurality of test sequences from the sequence batches in the analysis period, each test sequence comprising at least one single nucleotide polymorphism (SNP) obtained from a test sample, and each test sequence
corresponding to a sequence batch of the plurality of sequence batches; filtering the plurality of test sequences by removing one or more of the test
sequences, the remaining test sequences forming a population; determining a prior contamination probability for each SNP of the population, the prior contamination probability based on a minor allele frequency for each SNP;
applying a contamination model including at least one likelihood test to a test sequence of the population using the associated contamination probability, the tests identifying a contaminated test sequence based on the results of the likelihood tests;
applying a contamination source model to the sequence batch including the contaminated test sequence, the contamination source model identifying a contamination source sequence batch from the plurality of sequence batches based on the contamination probabilities of SNPs of the population and their corresponding test sequences and sequence batches; and
reporting the sequence batch from the analysis period that is the contamination source batch.

E101. A non-transitory computer-readable storage medium storing computer program instructions for identifying contamination in a test sample, the computer program instructions, when executed by a processor, causing the processor to:

determine an analysis period for a plurality of sequence batches in which the plurality of sequence batches were sequenced;
access a plurality of test sequences from the sequence batches in the analysis period, each test sequence comprising at least one single nucleotide polymorphism (SNP) obtained from a test sample, and each test sequence corresponding to a sequence batch of the plurality of sequence batches;
filter the plurality of test sequences by removing one or more of the test sequences, the remaining test sequences forming a population;
determine a prior contamination probability for each SNP of the population, the prior contamination probability based on a minor allele frequency for each SNP;
apply a contamination model including at least one likelihood test to a test sequence of the population using the associated contamination probability, the tests identifying a contaminated test sequence based on the results of the likelihood tests;
apply a contamination source model to the sequence batch including the contaminated test sequence, the contamination source model identifying a contamination source sequence batch from the plurality of sequence batches based on the contamination probabilities of SNPs of the population and their corresponding test sequences and sequence batches; and report the sequence batch from the analysis period that is the contamination source batch.

E102. A system for identifying contamination in a test sample, the system comprising:

one or more memories storing computer program instructions for identifying contamination in the test sample;
one or more processors configured to execute the computer program instructions for identifying contamination, the computer program instructions, when executed, causing the processor to:

determine an analysis period for a plurality of sequence batches in which the plurality of sequence batches were sequenced;
access a plurality of test sequences from the sequence batches in the analysis period, each test sequence comprising at least one single nucleotide polymorphism (SNP) obtained from a test sample, and each test sequence corresponding to a sequence batch of the plurality of sequence batches;
filter the plurality of test sequences by removing one or more of the test sequences, the remaining test sequences forming a population;
determine a prior contamination probability for each SNP of the population, the prior contamination probability based on a minor allele frequency for each SNP;
apply a contamination model including at least one likelihood test to a test sequence of the population using the associated contamination probability, the tests identifying a contaminated test sequence based on the results of the likelihood tests;
apply a contamination source model to the sequence batch including the contaminated test sequence, the contamination source model identifying a contamination source sequence batch from the plurality of sequence batches based on the contamination probabilities of SNPs of the population and their corresponding test sequences and sequence batches; and
report the sequence batch from the analysis period that is the contamination source batch.

**Claims**

1. A computer-implemented method for identifying a contamination source in a group of test samples, the method comprising:

determining an analysis window for a plurality of sequence batches obtained from the group of test samples;
accessing a plurality of test sequences from each sequence batch of the sequence batches in the analysis window, each test sequence comprising at least one single nucleotide polymorphism (SNP);
filtering the plurality of test sequences by removing one or more of the test sequences based on one or more criteria relating to inaccurate contamination indication, the remaining test sequences forming a population;
determining a prior contamination probability for each SNP of the population, the prior contamination probability based at least in part on a minor allele frequency for each SNP;
determining that one or more of the test samples in the group of test samples is contaminated by determining that one or more of the test sequences in the population is contaminated based on the prior contamination probability;
determining a likelihood that each of the one or more contaminated test samples is the contamination source, comprising applying a contamination source model to the test sequences of the population; and
identifying the contamination source based on the determined likelihood that each of the one or more contaminated test samples is the contamination source.

2. The method of claim 1, wherein the test sequences are sequence read pairs comprising forward strand sequence reads and reverse strand sequence reads, and wherein filtering the plurality of test sequences comprises:

filtering the forward strand sequence reads according to a first ruleset describing forward strand sequence reads of the test sequences that indicate contamination based on their methylation state, and
filtering the reverse strand sequence reads according to a second ruleset describing reverse strand sequence reads of the test sequences that indicate contamination based on their methylation state.

3. The method of claim 2, further comprising:

determining, for each of the forward strand sequence reads, a nucleotide base at an SNP site;

determining for each of the reverse strand sequence reads, a corresponding nucleotide base at the SNP site; and removing the sequence read pairs comprising:

one or more nucleotide bases at one or more SNP sites included in an SNP removal table, or
one or more corresponding nucleotide bases at one or more corresponding SNP sites included in the SNP removal table,

wherein the SNP removal table indicates SNPs that inaccurately indicate the contamination.

4. The method of any one of claims 1 to 3, wherein the analysis window is defined based on:

a number of test samples from a whole test sample set,
a period of test samples obtained from a longer time period of sample taking,
test samples associated with a testing location of a plurality of testing locations, or
test samples associated with a testing apparatus of a plurality of testing apparatuses.

5. The method of any one of claims 1 to 4, wherein filtering the plurality of test sequences by removing one or more of the test sequences comprises removing test sequences comprising:

non-informative SNPs,
SNPs with no coverage,
SNPs with high error frequencies,
SNPs with high variance,
SNPs with a depth less than a threshold,
heterozygous SNPs,
SNPs with low coverage, or
SNPs that have a high heterogeneity rate.

6. The method of any one of claims 1 to 5, wherein determining the prior contamination probability for each SNP of the population is based on a likelihood model applied to the minor allele frequency for each SNP.

7. The method of any one of claims 1 to 6, wherein determining that one or more of the test samples in the group of test samples is contaminated comprises applying a contamination model to the test sequences in the population.

8. The method of claim 7, wherein applying the contamination model comprises applying at least one likelihood test to each test sequence of the population using the associated contamination probability and wherein determining that one or more of the test samples in the group of test samples is contaminated is based on the results of the at least one likelihood test.

9. The method of any one of claims 1 to 8, wherein a contamination source sequence batch from the plurality of sequence batches is identified by the contamination source model based on the contamination probabilities of SNPs of the population and their corresponding test sequences and sequence batches.

10. The method of any one of claims 1 to 9, wherein identifying the contamination source further comprises:
ranking the one or more contaminated test samples based on the determined likelihood that the contaminated test sample is the contamination source.

11. The method of claim 10, wherein the identified contamination source comprises:

the one or more contaminated test samples satisfying a threshold ranking; or
the one or more contaminated test samples having a determined likelihood that the contaminated test sample is the contamination source satisfying a threshold.

12. The method of any one of claims 1 to 11, further comprising:
removing the contaminated test samples from further analysis.

13. The method of any one of claims 1 to 12, further comprising:
reporting the sequence batch from the analysis window corresponding to the contaminated test sample.

14. A non-transitory computer-readable storage medium storing computer program instructions for identifying a contamination source in a group of test samples, the computer program instructions, when executed by a processor, causing the processor to:

determine an analysis window for a plurality of sequence batches obtained from the group of test samples;
access a plurality of test sequences from each sequence batch of the sequence batches in the analysis window, each test sequence comprising at least one single nucleotide polymorphism (SNP);
filter the plurality of test sequences by removing one or more of the test sequences based on one or more criteria relating to inaccurate contamination indication, the remaining test sequences forming a population;
determine a prior contamination probability for each SNP of the population, the prior contamination probability based at least in part on a minor allele frequency for each SNP;
determine that one or more of the test samples in the group of test samples is contaminated by determining that one or more of the test sequences in the population is contaminated based on the prior contamination probability;
determine a likelihood that each of the one or more contaminated test samples is the contamination source, comprising applying a contamination source model to the test sequences of the population; and
identify the contamination source based on the determined likelihood that each of the one or more contaminated test samples is the contamination source.

15. A system for identifying a contamination source in a group of test samples, the system comprising:

one or more memories storing computer program instructions for identifying contamination in the test sample;
one or more processors configured to execute the computer program instructions for identifying contamination, the computer program instructions, when executed, causing the processor to:

determine an analysis window for a plurality of sequence batches obtained from the group of test samples;
access a plurality of test sequences from each sequence batch of the sequence batches in the analysis window, each test sequence comprising at least one single nucleotide polymorphism (SNP);
filter the plurality of test sequences by removing one or more of the test sequences based on one or more criteria relating to inaccurate contamination indication, the remaining test sequences forming a population;
determine a prior contamination probability for each SNP of the population, the prior contamination probability based at least in part on a minor allele frequency for each SNP;
determine that one or more of the test samples in the group of test samples is contaminated by determining that one or more of the test sequences in the population is contaminated based on the prior contamination probability;
determine a likelihood that each of the one or more contaminated test samples is the contamination source, comprising applying a contamination source model to the test sequences of the population; and
identify the contamination source based on the determined likelihood that each of the one or more contaminated test samples is the contamination source.

100

```
┌─────────────────────────────────────┐
│                                      │
│      Extract nucleic acid sample     │
│                 110                  │
│                                      │
└─────────────────────────────────────┘
                    │
                    ▼
┌─────────────────────────────────────┐
│                                      │
│   Prepare library using adapter ligation │
│                 120                  │
│                                      │
└─────────────────────────────────────┘
                    │
                    ▼
┌─────────────────────────────────────┐
│                                      │
│    Perform enrichment on the nucleic acid │
│                 sample               │
│                 130                  │
└─────────────────────────────────────┘
                    │
                    ▼
┌─────────────────────────────────────┐
│   Generate sequence reads of the nucleic acid │
│                 sample               │
│                 140                  │
└─────────────────────────────────────┘
```

# FIG. 1

Processing System 200

Sequence Processor 205

Machine Learning Engine 220

Models 225

Score Engine 235

Variant Caller 240

Sequence Database 210

Model Database 215

Parameter Database 230

# FIG. 2

300

Collapse sequence reads
310

Stitch collapsed reads
320

Assemble reads
330

Generate candidate variants
340

Score candidate variants
350

Output candidate variants
360

# FIG. 3

Machine Learning Engine
220

Contamination
detection work flow
400

GUI 450

LOH call files
432

LOH batch
component
430

Output files 440

Single variant call file
412

Single
sample
component
410

Plots 442

MAF variant call file
414

Multiple variant call files
422

Baseline
batch
component
420

# FIG. 4

Probability Distribution Plot
510

FIG. 5A

Probability Distribution Plot
520

FIG. 5B

Probability Distribution Plot
530

FIG. 5C

Probability Distribution Plot
540

FIG. 5D

Probability Distribution Plot
550

Minor Allele Depth (MAD)

**FIG. 5E**

Probability Distribution Plot
560

Minor Allele Depth (MAD)

**FIG. 5F**

Probability
Distribution Plot
570

FIG. 5G

Probability
Distribution Plot
580

FIG. 5H

Contamination Detection
Workflow
600

Start

Clean up sequencing data                     602

Calculate prior probability of
contamination for each SNP                    604

Perform likelihood tests                      606

608
Contamination
detected?                          No ──→  End

Yes

Identify likely source of                     610
contamination

End

# FIG. 6A

Contamination Detection
Workflow
630

```
                    ┌──────────────────┐
                    │      Start        │
                    └──────────────────┘
                             │
                             ▼
        ┌──────────────────────────────────┐      632
        │   Determine an analysis window    │
        └──────────────────────────────────┘
                             │
                             ▼
        ┌──────────────────────────────────┐      634
        │  Access previously measured       │
        │  samples from analysis window     │
        └──────────────────────────────────┘
                             │
                             ▼
        ┌──────────────────────────────────┐      636
        │      Clean up sequencing data     │
        └──────────────────────────────────┘
                             │
                             ▼
        ┌──────────────────────────────────┐      638
        │      Calculate probability of     │
        │   contamination for SNPs in batch │
        └──────────────────────────────────┘
                             │
                             ▼
  640          ◇─────────────────────────────◇        No     ┌──────────┐
         ╱      Contamination Detected?                ─────▶ │   End     │
                ◇─────────────────────────────◇               └──────────┘
                             │
                            Yes
                             │
                             ▼
        ┌──────────────────────────────────┐      642
        │  Determine likelihood that        │
        │  samples in batch are             │
        │  contamination source            │
        └──────────────────────────────────┘
                             │
                             ▼
        ┌──────────────────────────────────┐      644
        │     Identify likely source of     │
        │        contamination              │
        └──────────────────────────────────┘
                             │
                             ▼
                    ┌──────────────────┐
                    │       End         │
                    └──────────────────┘
```

# FIG. 6B

Informative SNP Frequency Plot
700

# FIG. 7A

Informative SNP Spider Plot
710

FIG. 7B

FIG. 8

Expected Power Plot
900

**FIG. 9**

Limit of Detection Plot
1000

Method 600
1010

Linear Regression Model
1015

**FIG. 10**

Validation Method
1100

Start

Train noise baseline for each SNP using
baseline/normal dataset — 1110

Perform validation — 1115

Test on real data — 1120

End

# FIG. 11

**FIG. 12**

Probability Distribution Plot 1300

First Hypothesis 1320

Second Hypothesis 1330

Null Hypothesis 1310

Alternative Allele Depth (AD)

Probability

**FIG. 13**

Probability Distribution Plot
1410

Null Hypothesis
1412

Second Hypothesis
1416

First Hypothesis
1414

cP = 0.9
Δ = 0.2
α = 0.2

Probability

0.08
0.04
0.00

0    10    20    30    40    50    60

Alternative Allele Depth (AD)

# FIG. 14A

Null Hypothesis
1412

Probability Distribution Plot
1420

Second Hypothesis
1416

First Hypothesis
1414

cP = 0.1
Δ = 0.2
α = 0.2

Probability

0.08
0.04
0.00

0    10    20    30    40    50    60

Alternative Allele Depth (AD)

# FIG. 14B

FIG. 14C

Contamination Detection Workflow
1500

```
              ┌─────────┐
              │  Start  │
              └─────────┘
                   │
                   ▼
   ┌─────────────────────────────┐     1510
   │    Clean-up sequence data    │
   └─────────────────────────────┘
                   │
                   ▼
   ┌─────────────────────────────┐     1515
   │   Calculate prior probability of
   │    contamination for each SNP │
   └─────────────────────────────┘
                   │
                   ▼
   ┌─────────────────────────────┐     1520
   │   Determine SNPs including loss of
   │      heterozygosity (LOH)    │
   └─────────────────────────────┘
                   │
                   ▼
   ┌─────────────────────────────┐     1525
   │       Remove LOH SNPs        │
   └─────────────────────────────┘
                   │
                   ▼
   ┌─────────────────────────────┐     1530
   │   Generate Noise Model for remaining
   │            SNPs              │
   └─────────────────────────────┘
                   │
                   ▼
   ┌─────────────────────────────┐     1535
   │  Fit maximum likelihood estimation to
   │            data              │
   └─────────────────────────────┘
                   │
                   ▼
  1540              ◇                        No   ┌───────┐
             Contamination  ──────────────────────│  End  │
               detected?                          └───────┘
                   │
                  Yes
                   │
                   ▼
   ┌─────────────────────────────┐     1545
   │   Identify likely source of  │
   │        contamination         │
   └─────────────────────────────┘
                   │
                   ▼
              ┌─────────┐
              │   End   │
              └─────────┘
```

**FIG. 15**

Validation Plot
1600

Expected = Determined
1610

FIG. 16

Comparison Plot
1710

Equivalent Detection
1712

FIG. 17A

FIG. 17B

FIG. 17C

74

FIG. 18A

FIG. 18B

FIG. 19A

FIG. 19B

FIG. 19C

Contamination Noise Baseline Workflow
2000

```
                    ┌─────────┐
                    │  Start  │
                    └────┬────┘
                         │
         ┌───────────────▼─────────────────┐   2010
         │ Collect variant allele frequencies for │◄──────────┐
         │      each SNP in a sample        │            │
         └───────────────┬─────────────────┘            │
                         │                              │
         ┌───────────────▼─────────────────┐   2015      │
         │      Call genotype for each SNP  │            │
         └───────────────┬─────────────────┘            │
                         │                              │
         ┌───────────────▼─────────────────┐   2020      │
         │      Remove heterozygous SNPs    │            │
         └───────────────┬─────────────────┘            │
                         │                              │
         ┌───────────────▼─────────────────┐   2025      │
         │  "Flip" the frequency of homozygous │          │
         │        alternative SNPs          │            │
         └───────────────┬─────────────────┘            │
                         │                              │
         ┌───────────────▼─────────────────┐   2030      │
         │      Identify "noise" for each SNP │          │
         └───────────────┬─────────────────┘            │
                         │                              │
         ┌───────────────▼─────────────────┐   2035      │
         │ Remove one outlier sample with highest │       │
         │              noise               │            │
         └───────────────┬─────────────────┘            │
                         │                              │
         ┌───────────────▼─────────────────┐   2040      │
         │  Calculate noise rate and other metrics │      │
         │  for each SNP to generate baseline │        │
         └───────────────┬─────────────────┘            │
                         │                              │
         ┌───────────────▼─────────────────┐   2045      │
         │   Re-run contamination detection │            │
         │  algorithm on baseline samples using │        │
         │         generated baseline       │            │
         └───────────────┬─────────────────┘            │
                         │                              │
       2050              ▼                              │
          ◇─────────────────────◇    Yes   ┌────────────┴──────┐  2055
          │   Any samples        ├─────────►│ Remove contaminated │
          │  contaminated?       │          │     sample(s)     │
          ◇─────────────────────◇          └───────────────────┘
                    │ No
                    ▼
              ┌─────────┐
              │   End   │
              └─────────┘
```

FIG. 20

Noise Rate Plot
2100

**FIG. 21**

SNP Distribution Plot
2210

FIG. 22A

SNP Distribution Plot
2220

FIG. 22B

Venn Diagram
2300

FIG. 23

FIG. 24

EP 4 657 445 A2

FIG. 25A

FIG. 25B

**FIG. 25C**

Screenshot 2600

| | | | | | | | | | | | | |
|---|---|---|---|---|---|---|---|---|---|---|---|---|
| 14 | 170109_ARTRefSet_B2_13_CR12 | 0.003 | 0 | 0.9036 | FALSE | 1 | -1345 | -1347 | -1349 | 1.00E-04 | FALSE | 2 |
| 15 | 170109_ARTRefSet_B2_14_W04421659381 | 0.003 | 0 | 0.767 | FALSE | 99 | -2063 | -2063 | -2026 | 0 | FALSE | 0 |
| 16 | 170109_ARTRefSet_B2_10_CR-01 | 0.05 | 0 | 1.2277 | FALSE | 1 | -1306 | -1306 | -1313 | 1.00E-04 | FALSE | 1 |
| 17 | 170109_ARTRefSet_B2_11_W04421659683 | 0.7 | 0 | 0.7196 | FALSE | 33 | -2055 | -2055 | -2017 | 0 | FALSE | 0 |
| 18 | 170109_ARTRefSet_B2_12_W04421654626 | 1 | 0 | 1.0607 | FALSE | 3 | -3167 | -3170 | -3171 | 1.00E-04 | FALSE | 3 |
| 19 | 170109_ARTRefSet_B2_13_W04421659351 | 2.00E-16 | 1.00E-04 | 1.0725 | FALSE | 12 | -1337 | -1349 | -1598 | 3.00E-04 | FALSE | 12 |
| 20 | 170109_ARTRefSet_B2_14_CR_08 | 6.00E-76 | 7.00E-04 | 1.0103 | TRUE | 224 | -2145 | -2650 | -2439 | 9.00E-04 | TRUE | 216 |
| 21 | 170109_ARTRefSet_B2_1_CR_06 | 0.003 | 1.00E-04 | 0.9712 | FALSE | -11 | -1221 | -1222 | -1210 | 1.00E-04 | FALSE | 1 |
| 22 | 170109_ARTRefSet_B2_2_CR_16 | 0.05 | 0 | 1.0177 | FALSE | -12 | -1020 | -1027 | -1014 | 1.00E-04 | FALSE | 1 |

| | | | | | |
|---|---|---|---|---|---|
| 170109_ARTRefSet_B1_03_W04421659432E | 2.00E-04 | FALSE | 170109_ARTRefSet_B1_03_W0442 | | 2 |
| 170109_ARTRefSet_B1_01_CR15 | 0 | FALSE | 170109_ARTRefSet_B1_01_CR15 | | 0 |
| 170109_ARTRefSet_B1_07_CR23 | 2.00E-04 | FALSE | 170109_ARTRefSet_B1_07_CR23 | | 1 |
| 170109_ARTRefSet_B1_01_CR25 | 0 | FALSE | 170109_ARTRefSet_B1_01_CR15 | | 0 |
| 170117_ARTRefSet_B4_3_W04421659203 | 2.00E-04 | FALSE | 170109_ARTRefSet_B1_01_CR15 | | 2 |
| 170109_ARTRefSet_B2_1_CR_06 | 3.00E-04 | FALSE | 170109_ARTRefSet_B1_03_W0442 | | 10 |
| 170124_ARTRefSet_B6_04_W04421657158 | 9.00E-04 | FALSE | 170124_ARTRefSet_B6_06_W0442 | | 107 |
| 170109_ARTRefSet_B3_03_W04421659678 | 2.00E-04 | FALSE | 170109_ARTRefSet_B3_03_W0442 | | 1 |
| 170109_ARTRefSet_B3_03_W04421659678 | 3.00E-04 | FALSE | 170109_ARTRefSet_B3_03_W0442 | | 1 |

**FIG. 26A**

Screenshot 2610

**FIG. 26B**

Likelihood Plot
2710

Contamination Level

**FIG. 27A**

Likelihood Plot
2720

**FIG. 27B**

Dual-strand Filtering
Workflow
<u>2800</u>

Start

2810

Access sequencing data for
set of test samples

2820

Clean up samples

2830

Filter samples to remove
unmethylated SNPS

2840

Determine a contamination event

End

# FIG. 28

Sample Distribution Plot
2900

Number of SNPs assessed from 166 WGBS samples

Median: 11316 SNPs

**FIG. 29**

EP 4 657 445 A2

FIG. 30A

FIG. 30B

Single-strand
Filtering Workflow
3100

```
              ┌──────────────┐
              │    Start     │
              └──────────────┘
                     │
                     ▼
        ┌───────────────────────────┐      3110
        │  Access sequencing data for │
        │     set of test samples     │
        └───────────────────────────┘
                     │
                     ▼
        ┌───────────────────────────┐      3120
        │      Clean up samples       │
        └───────────────────────────┘
                     │
                     ▼
        ┌───────────────────────────┐      3130
        │  Access filtering rule table │
        └───────────────────────────┘
                     │
                     ▼
        ┌───────────────────────────┐      3130
        │  Filter samples based on rules in │
        │          rule table          │
        └───────────────────────────┘
                     │
                     ▼
        ┌───────────────────────────┐      3140
        │ Determine a contamination event │
        └───────────────────────────┘
                     │
                     ▼
              ┌──────────────┐
              │     End      │
              └──────────────┘
```

# FIG. 31

Filter Verification Plot
3200

**FIG. 32**

**FIG. 33A**

**FIG. 33B**

FIG. 34A

FIG. 34B

Blacklist Filtering
Workflow
3500

```
                    ┌─────────────┐
                    │    Start     │
                    └──────┬──────┘
                           │
                           ▼                    3510
         ┌──────────────────────────────┐
         │   Access sequencing data for  │
         │       set of test samples     │
         └──────────────┬───────────────┘
                        │
                        ▼                        3520
         ┌──────────────────────────────┐
         │        Clean up samples        │
         └──────────────┬───────────────┘
                        │
                        ▼                        3530
         ┌──────────────────────────────┐
         │  Filter samples based on blacklist │
         └──────────────┬───────────────┘
                        │
                        ▼                        3530
         ┌──────────────────────────────┐
         │   Determine a contamination event │
         └──────────────┬───────────────┘
                        │
                        ▼
                 ┌─────────────┐
                 │     End      │
                 └─────────────┘
```

# FIG. 35

Contamination Event
Comparison Plot
3600

FIG. 36A

Drift Region
3602

Contamination Event
Comparison Plot
3610

FIG. 36B

Blacklist Generation
Workflow
<u>3700</u>

```
┌─────────────┐
│    Start    │
└─────────────┘
       │
       ▼
┌──────────────────────────┐      3710
│ Access sequencing data   │
│ for set of               │
│ uncontaminated test      │
│ samples                  │
└──────────────────────────┘
       │
       ▼
┌──────────────────────────┐      3720
│ Determine characteristics│
│ for test samples         │
└──────────────────────────┘
       │
       ▼
┌──────────────────────────┐      3730
│    Clean-up samples      │
└──────────────────────────┘
       │
       ▼
┌──────────────────────────┐      3740
│ Determine outlier        │
│ indicator based on       │
│ determined characteristics│
└──────────────────────────┘
       │
       ▼
┌──────────────────────────┐      A50
│ Generate blacklist for   │
│ outlier fraction         │
└──────────────────────────┘
       │
       ▼
┌─────────────┐
│     End     │
└─────────────┘
```

# FIG. 37

Cohort Characteristic Plot 3800

Variant Threshold 3810

Drift Reg 3602

SNP

Observed Minor Allele Frequency

**FIG. 38**

102

FIG. 39

FIG. 40

FIG. 41

Contamination Threshold
Workflow
4200

FIG. 42

FIG. 43

ROC Heuristic Plot
4400

**FIG. 44**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63080670 A
- WO 44216569493 A
- WO 44216552592 A
- US 01931518 A
- US 62460268 A
- WO 44216569928 A

- WO 44216575078 A
- WO 44216564538 A
- WO 44216552592 B6
- WO 44216575078 B6
- WO 44216564538 B6
- WO 44216569493 B1

**Non-patent literature cited in the description**

- **CIBULSKIS, K.** ContEst: estimating cross-contamination of human samples in next-generation sequencing data. *Bioinformatics*, 2011
- **JUN, G.** Detecting and Estimating Contamination of Human DNA Samples in Sequencing and Array-Based Genotype Data. *American Journal of Human Genetics*, 2012

- **BERGMANN, E**. Conpair: concordance and contamination estimator for matched tumor-normal pairs. *Bioinformatics*, 2016